# EUROPEAN PATENT APPLICATION

(11) **EP 1 547 609 A1**
(43) Date of publication of application: **29.06.2005**
(21) Application number: 03794212.5
(22) Date of filing: 03.09.2003
(51) Int. Cl.: A61K 38/00, A61K 48/00, A61K 39/395, A61K 31/7088, A61P 15/00, A61P 37/02, C12N 15/09, C12Q 1/68, G01N 33/50, G01N 33/15, G01N 33/68, G01N 33/53

(54) **EMBRYO IMPLANTATION INHIBITOR**

(30) Priority: 04.09.2002 JP 2002259268
(71) Applicant: Proteinexpress Co., Ltd., Chosi-shi, Chiba 288-0041 (JP)
(72) Inventor: IMAKAWA, Kazuhiko The University of Tokyo, Tokyo 113-8657 (JP); NAGAOKA, Kentaro, Sunto-gun, Shizuoka 441-0943 (JP); WATANABE, Fumiko R & D Department, Choshi-shi, Chiba 288-8680 (JP)
(74) Representative: Helbing, Jörg, Dr. Dipl.-Chem.
(86) International application number: PCT/JP2003/011268
(87) International publication number: WO 2004/022082

(57) **Abstract**

For a pregnancy to be established, communication between conceptus and mother, and implantation of the conceptus on the uterine wall must occur. If a factor mediating the communication between conceptus and mother is investigated and the factor can be used to control the communication, it may be possible to develop a method for treating a disorder such as sterility or preventive measures. Contraception in a safe manner may also be provided. IP-10, which is known as a protein of 10 kDa usually induced by interferon-γ is identified as a factor that controls implantation of conceptus on the uterine wall through involving immune responses and activation of conceptus migration during an implantation period, the promotion of implantation of conceptus to the uterine wall, and chemotactic activity. Based on these finding, an agent for treating sterility, promoting pregnancy, inducing implantation of conceptus on the uterine wall, or controlling interaction between conceptus and mother is provided.

SEQUENCE LISTING

<110> ProteinExpress Co., Ltd.

<120> Regulator for Implantation

<130> PE-01PCT

<150> JP 2002-259268
<151> 2002-9-4

<160> 26

<170> PatentIn Ver. 2.0

<210> 1
<211> 1171
<212> DNA
<213> Ovis aries

<220>
<221> CDS
<222> (60)..(368)

<400> 1
cactcctcaa ctcttcaggc agtctgagct actgcagaag taccttcagt tgcagcacc 59

atg aac aaa agt ggt ttt ctt att ttc tgc ctt atc ctt ctg act ctg 107
Met Asn Lys Ser Gly Phe Leu Ile Phe Cys Leu Ile Leu Leu Thr Leu
 1               5                  10                  15

agt caa ggc ata cct ctc tct agg aac aca cgc tgc acc tgc atc gag 155
Ser Gln Gly Ile Pro Leu Ser Arg Asn Thr Arg Cys Thr Cys Ile Glu
            20                  25                  30

atc agt aat gga tct gtt aat cca agg tcc tta gaa aaa ctt gaa ctg 203
Ile Ser Asn Gly Ser Val Asn Pro Arg Ser Leu Glu Lys Leu Glu Leu
        35                  40                  45

att cct gca agt caa tcc tgc cca cgt gtc gag att att gcc aca atg 251
Ile Pro Ala Ser Gln Ser Cys Pro Arg Val Glu Ile Ile Ala Thr Met
    50                  55                  60

aaa agg aat ggg gag aaa aga tgt ctg aat cca gaa tct aag acc atc 299
Lys Arg Asn Gly Glu Lys Arg Cys Leu Asn Pro Glu Ser Lys Thr Ile
 65                  70                  75                  80

aag aat tta ctg aaa gca att aac aag caa agg act aaa aga tct cct 347
Lys Asn Leu Leu Lys Ala Ile Asn Lys Gln Arg Thr Lys Arg Ser Pro
                 85                  90                  95

cga aca cag aaa gag gca taa tcactgcact actgataaga tggaccagag 398
Arg Thr Gln Lys Glu Ala
            100

agaagctacc tctacaattg tttccctgtg tacagtatat gtcaagccct aattgttcgt 458

ggacttcagt tctcctaaaa ggtgaccaag ccagtcacca aatcagctgc tactactcct 518

gcagggggag ggtggctcat caccctgagc tgttcagtag tgactctgcc ctggcactgt 578

gactgtaagc tataccgggg cgctacgttc tcagttaatg tgctaagtcc cagccttgct 638

actgacagct tcttcccctt tccaatcttt ctaggttatt aagggatctt tccagctctg 698

ggcttattag agaccttagg atctcaaata actaagagac attcaaacca ataatgcaat 758

ctgcttttta aagaaagatc tttactccag gggcttcact gccatccctc caaggggccc 818

gtattctttc aggtgttatg tacatagttc caaatataca gaagcagcca gaaatatctg 878

gaaatgtagg tctaaacagt attacttagt caaaaactat acaaagtaga attcttgaag 938

atatatgttt cttatatgat tttcagtgtt catggaataa cttgtataca actatcaact 998

tatgtaatta ttgcaatgga ataaattttt aaatttagat acatgttctg caggctatgt 1058

aagacaaata tgctaaatgc tttccaaaat aaaagtaatg ttctctccca gaaatactaa 1118

gaaagattat gtaattgttt tagaggccaa aaacataata aataattata act 1171

<210> 2
<211> 102
<212> PRT
<213> Ovis aries

<400> 2
Met Asn Lys Ser Gly Phe Leu Ile Phe Cys Leu Ile Leu Leu Thr Leu
1               5                   10                  15

Ser Gln Gly Ile Pro Leu Ser Arg Asn Thr Arg Cys Thr Cys Ile Glu
            20                  25                  30

Ile Ser Asn Gly Ser Val Asn Pro Arg Ser Leu Glu Lys Leu Glu Leu
        35                  40                  45

Ile Pro Ala Ser Gln Ser Cys Pro Arg Val Glu Ile Ile Ala Thr Met
    50                  55                  60

Lys Arg Asn Gly Glu Lys Arg Cys Leu Asn Pro Glu Ser Lys Thr Ile
65                  70                  75                  80

Lys Asn Leu Leu Lys Ala Ile Asn Lys Gln Arg Thr Lys Arg Ser Pro
                85                  90                  95

Arg Thr Gln Lys Glu Ala
            100

<210> 3
<211> 20
<212> DNA
<213> Artificial Sequence

<220>
<223> Description of Artificial Sequence:Oligonucleotide
      to act as a primer for PCR

<400> 3
cactcctcaa ctcttcaggc                                                20

<210> 4
<211> 20
<212> DNA

<213> Artificial Sequence

<220>
<223> Description of Artificial Sequence:Oligonucleotide
      to act as a primer for PCR

<400> 4
ccattccttt tcattgtggc                                                   20


<210> 5
<211> 20
<212> DNA
<213> Artificial Sequence

<220>
<223> Description of Artificial Sequence:Oligonucleotide
      to act as a primer for PCR

<400> 5
gcatcagctt cgatcggtac                                                   20


<210> 6
<211> 20
<212> DNA
<213> Artificial Sequence

<220>
<223> Description of Artificial Sequence:Oligonucleotide
      to act as a primer for PCR

<400> 6
gatgcgggcg tagcaatagg                                                   20


<210> 7
<211> 20
<212> DNA
<213> Artificial Sequence

<220>
<223> Description of Artificial Sequence:Oligonucleotide
      to act as a primer for PCR

<400> 7
catcttcccc atggccttcg                                                   20


<210> 8
<211> 20
<212> DNA
<213> Artificial Sequence

<220>
<223> Description of Artificial Sequence:Oligonucleotide
      to act as a primer for PCR

<400> 8
tcatctcaaa gtgagttcag                                                   20


<210> 9

<211> 20
<212> DNA
<213> Artificial Sequence

<220>
<223> Description of Artificial Sequence:Oligonucleotide
      to act as a primer for PCR

<400> 9
cgatgaaata cacaagctcc                                                20


<210> 10
<211> 20
<212> DNA
<213> Artificial Sequence

<220>
<223> Description of Artificial Sequence:Oligonucleotide
      to act as a primer for PCR

<400> 10
gattacattg atgctctccg                                                20


<210> 11
<211> 20
<212> DNA
<213> Artificial Sequence

<220>
<223> Description of Artificial Sequence:Oligonucleotide
      to act as a primer for PCR

<400> 11
atggggaagg tgaaggtcgg                                                20


<210> 12
<211> 20
<212> DNA
<213> Artificial Sequence

<220>
<223> Description of Artificial Sequence:Oligonucleotide
      to act as a primer for PCR

<400> 12
atgtgggcca tgaggtccac                                                20


<210> 13
<211> 20
<212> DNA
<213> Artificial Sequence

<220>
<223> Description of Artificial Sequence:Oligonucleotide
      to act as a primer for PCR

<400> 13
atggggaagg tgaaggtcgg                                                20

<210> 14
<211> 20
<212> DNA
<213> Artificial Sequence

<220>
<223> Description of Artificial Sequence:Oligonucleotide
      to act as a primer for PCR

<400> 14
atgtgggcca tgaggtccac                                                20


<210> 15
<211> 20
<212> DNA
<213> Artificial Sequence

<220>
<223> Description of Artificial Sequence:Oligonucleotide
      to act as a primer for PCR

<400> 15
atggagccct cagacatccc                                                20


<210> 16
<211> 20
<212> DNA
<213> Artificial Sequence

<220>
<223> Description of Artificial Sequence:Oligonucleotide
      to act as a primer for PCR

<400> 16
gaggatctcc acgtagcaga                                                20


<210> 17
<211> 20
<212> DNA
<213> Artificial Sequence

<220>
<223> Description of Artificial Sequence:Oligonucleotide
      to act as a primer for PCR

<400> 17
tgctgtgaac cagagtcgtc                                                20


<210> 18
<211> 20
<212> DNA
<213> Artificial Sequence

<220>
<223> Description of Artificial Sequence:Oligonucleotide
      to act as a primer for PCR

<400> 18

atccactgca cagctgtggc                                                    20

<210> 19
<211> 20
<212> DNA
<213> Artificial Sequence

<220>
<223> Description of Artificial Sequence:Oligonucleotide
      to act as a primer for PCR

<400> 19
gaagcaggaa agagagcctg                                                    20

<210> 20
<211> 20
<212> DNA
<213> Artificial Sequence

<220>
<223> Description of Artificial Sequence:Oligonucleotide
      to act as a primer for PCR

<400> 20
ctatatccgt ggctcctttc                                                    20

<210> 21
<211> 20
<212> DNA
<213> Artificial Sequence

<220>
<223> Description of Artificial Sequence:Oligonucleotide
      to act as a primer for PCR

<400> 21
ctcaaatcca gccacagcag                                                    20

<210> 22
<211> 20
<212> DNA
<213> Artificial Sequence

<220>
<223> Description of Artificial Sequence:Oligonucleotide
      to act as a primer for PCR

<400> 22
ccagcgaagt gaaacacagc                                                    20

<210> 23
<211> 20
<212> DNA
<213> Artificial Sequence

<220>
<223> Description of Artificial Sequence:Oligonucleotide
      to act as a primer for PCR

<400> 23
agattggaga cacggtgagc                                                   20


<210> 24
<211> 20
<212> DNA
<213> Artificial Sequence

<220>
<223> Description of Artificial Sequence:Oligonucleotide
      to act as a primer for PCR

<400> 24
gtacttgaaa gtgatcttgc                                                   20


<210> 25
<211> 20
<212> DNA
<213> Artificial Sequence

<220>
<223> Description of Artificial Sequence:Oligonucleotide
      to act as a primer for PCR

<400> 25
gtctgaagat tggggacagc                                                   20


<210> 26
<211> 20
<212> DNA
<213> Artificial Sequence

<220>
<223> Description of Artificial Sequence:Oligonucleotide
      to act as a primer for PCR

<400> 26
ggtacacgct ctggttctcc                                                   20

## Description

### FIELD OF THE INVENTION

The present invention relates to a technology utilizing a regulation factor for communication between a conceptus and mother on implantation for pregnancy. In particular, the present invention relates to a technology based on that IP-10 has important functions for conceptus implantation and in its process.

### BACKGROUND OF THE INVENTION

Implantation is harmonized processes between a conceptus and mother including cellular proliferation, differentiation, and formation of initial placenta. In ruminants, conceptus is lost at a high rate in an implantation period (Roberts R.M. et al., (1990) Oxf. Rev. Reprod. Biol., 12:147-180). This may be caused by failure in communication between conceptus and mother. Once the communication has been established, the maternal system can physiologically and immunologically accept the conceptus. Thus adhesion and invasion (or implantation) of the conceptus and subsequent placentation will occur.

Interferon-tau (IFN-τ) is a protein secreted by trophoblast cells of ruminants during peri-implantation period and has an activity inhibiting the degeneration of corpus luteum. IFN-τ has an anti-virus activity and inhibits cellular proliferation similar to other interferons. Furthermore, IFN-τ may have an immunoregulating activity.

In ruminants, IFN-τ is a main protein secreted by growing conceptus and is involved in the process of maternal recognition of pregnancy (Martal J. et al., (1979) J. Reprod. Fertil., 56:63-72; Godkin J.D. et al., (1982) J. Reprod. Fertil., 65:141-150; Imakawa K. et al., (1987) Nature, 330:377-379; Stewart H.J. et al., (1987) J. Endocrinol., 115:R13-R15; Roberts R.M. et al., (1992) Endocr. Rev., 13:432-452). In ovines, conceptus starts to produce IFN-τ on Day 8 or 9 of pregnancy. The production of IFN-τ is the highest on Day 16, then, decreases according to attachment of the conceptus on the uterine endometrium. The conceptus stops the production of IFN-τ by Day 22 or 23 (Guillomot M. et al., (1990) Bio Cell., 68:205-211; Roberts R.M. et al., (1992) Proc. Soc. Exp. Biol Med., 200:7-18; Flint A.P.F. et al., (1994) Mol. Cell. Endocrinol., 100:93-95). IFN-τ obstructs or partially obstructs degeneration of corpus luteum by inhibiting expression of an estrogen receptor, prevents an oxytocin receptor from stimulation by estrogen, and suppresses intermittent release of PGF_{2α} from the endometrium (Flint A.P.F. et al., (1991) J. Reprod. Fertil. (Suppl.), 43:13-25; Spencer T.E. et al., (1995) Endocrinology, 136:4932-4944; Spencer T.E. et al., (1996) Endocrinology, 137:1144-1147). IFN-τ controls differentiation of lymphocytes and production of cytokines (Differentiation of lymphocytes: Newton G.R. et al., (1989) Am. J. Reprod. Immunol., 19:99-107; Pontzer C.H. et al., Cancer Res., (1991) 51:5304-5307; Skopets B. et al., (1992) Vet. Immunol. Immunopathol., 34:81-96; Assal-Meliani A. et al., (1993) J. Reprod. Immunol., 25:149-65, Production of cytokines: Tuo W. et al., (1999) J. Interferom Cytokine Res., 19:79-87; Emond V. et al., (2000) Biol. Reprod., 62:1728-1737). These show that IFN-τ locally plays an important role in an immunoregulating process for implantation in ruminant ungulates.

Interferon-γ-inducible protein 10 kDa (IP-10) belongs to a chemokine family that controls inflammatory and immune responses in many aspects mainly through chemotactic activity toward subsets of leukocytes. Studies for mice and humans identified that IP-10 belongs to C-X-C chemokine and is induced in various cells such as macrophages, fibroblasts, astrocytes, keratinocytes, epithelial cells, and endothelial cells (Luster A. et al., (1985) Nature, 315:672-676; Ohmori Y. et al., (1990) Biochem. Biophys. Res. Commun., 168:1261-1267; Sauty A. et al., (1999) J. Immunol., 162:3549-3558; Albanesi C. et al., (2000) J. Immunol., 165:1395-1402; Huang D. et al., (2000) Immunol. Rev., 177:52-67). IP-10 preferentially acts on NK cells and activated T cells (phenotype Th1) through C-X-C chemokine receptor 3 (CXCR3).

### SUMMARY OF THE INVENTION

Biochemical communication between conceptus, which includes embryos and embryonic membranes, and a maternal system is important for successful implantation and subsequent placentation. In particular, since conceptus is lost at a high rate in implantation period in ruminants, controlling factors for gestation have been investigated. Especially, the need exists for investigation of a maternal factor responding to a signal from conceptus in the gestation process.

The inventors have diligently investigated a factor controlling pregnancy establishment. In the studies for determining molecules associated with ovine pregnancy, the inventors have successfully identified a 10 kDa protein induced by IFN-γ (IP-10) from cDNA subtraction analysis of cDNA prepared from RNA extracted from uterine endometrial tissues of Day 17 pregnant and Day 15 cyclic ewes. Namely, ovine IP-10, which belongs to a C-X-C chemokine family and lacks glutamine-leucine-arginine (ELR) motif, has been identified from a cDNA subtraction study between uterine endometrial tissues from Day 17 pregnant and Day 15 cyclic ewes.

Northern blot analysis has revealed that a large amount of IP-10 mRNA are present in early pregnant endometrium, the amount is highly larger than that in cyclic endometrium. RT-PCR detection of CXCR3 (a receptor for IP-10) mRNA expression has revealed that the expression in pregnant endometrium is slightly larger than that in cyclic endometrium.

The inventors have determined changes in IFN-τ and IFN-γ mRNAs by Northern blotting and RT-PCR. IFN-τ and IFN-γ may induce the expression of IP-10. The results have revealed that IFN-τ and IFN-γ mRNAs are present in conceptus and pregnant endometrium at high quantities. IFN-γ mRNA has been detected in cyclic endometrium. Immunohistochemical analysis has revealed that both IP-10 and IFN-γ proteins are localized in luminal and glandular epithelium and subepithelial stroma of the uteri. Both IP-10 and IFN-γ proteins are localized in cyclic ewes, but IP-10 staining of the cyclic endometrium is reduced to the minimum. In situ hybridization has revealed that IP-10 mRNA is localized in subepithelial stroma of pregnant uteri. On the other hand, IP-10 mRNA is not detected in cyclic ewes. IP-10 mRNA expression in monocytes is stimulated in accordance with the amounts of IFN-α, IFN-γ, and IFN-τ, and IFN-τ mostly raised the amount of IP-10 mRNA. Analysis of cultured endometrial cells has revealed that IP-10 mRNA can be stimulated by a small amount of IFN-τ. These results suggest IFN-τ induces expression of IP-10 in early pregnant uteri. Thus, it has been identified that interaction between IFN-τ and IP-10 is important for immunological communication between the conceptus and the maternal tissue. Similar phenomena have been identified on caprine IP-10. The inventors have recognized that a technology for controlling implantation may be provided by using IP-10 because IP-10 is widely distributed in various animals and their homologies are very high, for example, between ovine and human, and have completed the present invention on the basis of the above-mentioned findings.

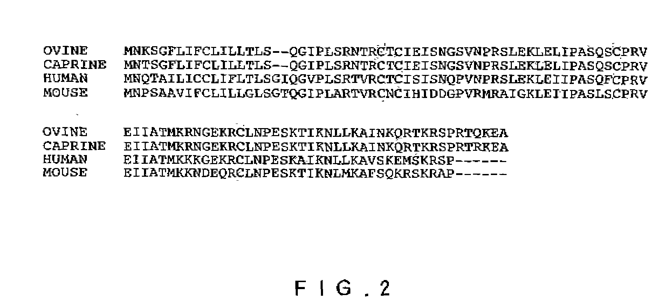

In a more specific aspect, the present invention provides the following:
(1) A pharmaceutical drug and/or animal drug comprising an effective amount of one or more members selected from the group consisting of an IP-10 protein and IP-10 analogues or IP-10 derivatives having at least a deletion, addition, and/or substitution of one or more amino acid residues in IP-10 and having biological activities essentially equal or equivalent to those of intact IP-10,
   the pharmaceutical drug and/or animal drug being an agent selected from the group consisting of (a) an agent for activating conceptus migration, (b) an agent for promoting conceptus implantation on the uterine wall, (c) an agent for treating sterility, (d) an agent for promoting pregnancy, (e) an agent for controlling interaction between conceptus and maternal system, (f) an agent for activating immunocyte migration, and (g) an agent for controlling immune function in the uterus;
(2) The pharmaceutical drug and/or animal drug according to the above (1), which is derived from mammal including human, bovine, buffalo, equine, donkey, ovine, goat, camel, swine, deer, reindeer, yak, canine, cat, and ape;
(3) A method for obtaining a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus,
   which comprises treating a sample with a material selected from the group consisting of IP-10 and IP-10 analogues or IP-10 derivatives having at least a deletion, addition, and/or substitution of one or more amino acid residues in IP-10 and having biological activities essentially equal or equivalent to those of intact IP-10;
(4) A reagent comprising at least a material selected from the group consisting of IP-10 and IP-10 analogues or IP-10 derivatives having at least a deletion, addition, and/or substitution of one or more amino acid residues in IP-10 and having biological activities essentially equal or equivalent to those of intact IP-10 and being useful for the method according to the above (3);
(5) An assay for measuring an IP-10 activity to determine a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus;
(6) A reagent useful for the assay according to the above (5);
(7) A pharmaceutical drug and/or animal drug comprising a nucleic acid having a nucleotide sequence encoding at least a member selected from the group consisting of an IP-10 protein and IP-10 analogues or IP-10 derivatives having at least a deletion, addition, and/or substitution of one or more amino acid residues in IP-10 and having biological activities essentially equal or equivalent to those of intact IP-10,
   the pharmaceutical drug and/or animal drug being an agent selected from the group consisting of (a) an agent for activating conceptus migration, (b) an agent for promoting conceptus implantation on the uterine wall, (c) an agent for treating sterility, (d) an agent for promoting pregnancy, (e) an agent for controlling interaction between conceptus and maternal system, (f) an agent for activating immunocyte migration, and (g) an agent for controlling immune function in the uterus;
(8) A pharmaceutical drug and/or animal drug comprising a nucleic acid selected from the group consisting of
   (i) a nucleotide sequence comprising at least one open reading frame portion present in SEQ ID NO: 1 and,
   (ii) a nucleotide sequence capable of hybridizing with at least one sequence described in the above (i) under a stringent condition, and
   (iii) a nucleotide sequence encoding a peptide containing an amino acid sequence at least 80% homologous to a polypeptide in FIG. 2 or shown by SEQ ID NO: 2, wherein said peptide has a biological activity substantially equal to that of IP-10 (for example, ovine IP-10), including a biological activity, or an antigenic equivalent thereof, selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus,
      the pharmaceutical drug and/or animal drug being an agent selected from the group consisting of (a) an agent for activating conceptus migration, (b) an agent for promoting conceptus implantation on the uterine wall, (c) an agent for treating sterility, (d) an agent for promoting pregnancy, (e) an agent for controlling interaction between conceptus and maternal system, (f) an agent for activating immunocyte migration, and (g) an agent for controlling immune function in the uterus;
(9) A pharmaceutical drug comprising a compound, or its salt, for promoting or inhibiting a biological activity being, for example, selected from (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus,
   said biological activity being owned by
   (A) a material, or its salt, selected from the group consisting of an IP-10 protein and IP-10 analogues or IP-10 derivatives having at least a deletion, addition, and/or substitution of one or more amino acid residues in IP-10 and having biological activities essentially equal or equivalent to those of intact IP-10, or
   (B) a nucleic acid selected from the group consisting of
      (i) a nucleotide sequence comprising at least an open reading frame portion present in SEQ ID NO: 1 and,
      (ii) a nucleotide sequence capable of hybridizing with at least a sequence as defined in the above (i) under a stringent condition, and
      (iii) a nucleotide sequence encoding a peptide containing an amino acid sequence at least 80% homologous to a polypeptide in Fig. 2 or shown by SEQ ID NO: 2, wherein said peptide has a biological activity substantially equal to that of IP-10 (for example, ovine IP-10), including a biological activity, or an antigenic equivalent thereof, selected from (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus;
(10) A method or kit for screening a compound promoting or inhibiting a biological activity owned by a member selected from the group consisting of IP-10, and IP-10 analogues or IP-10 derivatives having at least a deletion, addition, and/or substitution of one or more amino acid residues in IP-10 and having a biological activity essentially equal or equivalent to that of intact IP-10, or salts thereof, and IP-10 nucleic acids encoding the same, wherein said biological activity is, for example, selected from (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus,
   the method or the kit using or comprising (A) a compound, or its salt, selected from the group consisting of IP-10 and the IP-10 analogues or IP-10 derivatives or (B) a material selected from the group consisting of nucleic acids encoding the compound in (A), vectors containing the nucleic acid, and host cells transformed with the nucleic acid or the vector;
(11) The method or kit according to the above (10), which is useful for screening a compound promoting the production of IP-10 to prevent development and/or progress of sterility;
(12) A compound for controlling the production of IP-10, which is obtained or identified by screening with the method or kit according to the above (10) or (11);
(13) A reagent for detecting the presence of a mutated portion capable of altering the activity or expression of IP-10 wherein said mutated portion is present in IP-10, a gene encoding IP-10, or the corresponding RNA, and genetically diagnosing a disease associated with IP-10;
(14) The diagnostic reagent according to the above (13), which comprises at least a material selected from the group consisting of restriction enzymes which specifically recognize a mutated portion in IP-10 gene, mRNA, or hnRNA, and isoschizomers thereof; and oligonucleotide primers useful for amplification of a gene including a mutated portion in IP-10 gene, mRNA, or hnRNA; and
(15) A method for genetic diagnosis of a disease associated with an IP-10 gene which comprises the steps of;
   (a) preparing or obtaining a nucleic acid sample,
   (b) subjecting the nucleic acid sample in the step (a) to gene amplification to give amplified nucleic acid fragments containing one or more mutations potentially present in the IP-10 gene, and
   (c) determining the presence of mutation in the nucleic acid fragment in the step (c).

   In another aspect, the present invention provides the following:
(16) A method for determining or diagnosing a biological activity in a specimen which comprises quantitating an IP-10 polynucleotide present in the specimen and using as an indicator the IP-10 polynucleotide amount to determine or diagnose a biological activity level in the specimen wherein said biological activity is selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus;
(17) A method for determining or diagnosing a biological activity in a specimen which comprises quantitating an IP-10 protein present in the specimen and using as an indicator the IP-10 protein amount to determine or diagnose a biological activity level in the specimen wherein said biological activity is selected from:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus;
(18) A composition for determining or diagnosing a biological activity in a specimen which comprises at least a member selected from oligonucleotides or polynucleotides which hybridize with IP-10 polynucleotide under a stringent condition, and useful for determining or diagnosing a biological activity level in the specimen wherein said biological activity is selected from:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus;
(19) A nucleic acid array for determining or diagnosing a biological activity level in a specimen wherein said biological activity is selected from:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the array being provided with (i) an oligonucleotide or polynucleotide which hybridizes with IP-10 polynucleotide under a stringent condition or (ii) IP-10 polynucleotide;
(20) A primer set for PCR amplifying IP-10 polynucleotide in a specimen and determining or diagnosing a biological activity level in a specimen
   wherein said biological activity is selected from:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus;
(21) A diagnosis kit for determining or diagnosing a biological activity in a specimen which comprises at least an antibody capable of recognizing IP-10 and useful for determining or diagnosing a biological activity level in the specimen
   wherein said biological activity is selected from:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus;
(22) A diagnosis kit for determining or diagnosing a biological activity in a specimen which comprises at least elements consisting of
   (i) an immobilized antibody capable of recognizing IP-10, and
   (ii) a labeled antibody capable of recognizing IP-10 but binding to an epitope different from those targeted by the antibody (i), and useful for determining or diagnosing a biological activity level
   wherein said biological activity is selected from:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus;
(23) A method for measuring a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the method comprising at least the steps of:
   (i) contacting a biological specimen with a support immobilized with an antibody capable of recognizing IP-10;
   (ii) washing the support contacted with the biological specimen;
   (iii) contacting the support contacted with the biological specimen with a labeled antibody, wherein the labeled antibody is capable of recognizing an epitope on IP-10 other than that recognized by the immobilized antibody;
   (iv) measuring the label on the support or being free;
   (v) using the amount of the label measured in the step (iv) as an indicator of the amount of IP-10 and comparing it with a result of a normal biological specimen; and
   (vi) using the amount of IP-10 being significantly different from the result of the normal biological specimen as an indicator of the degree of abnormality or its risk associated with the biological activity;
(24) A method for measuring a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the method comprising at least the steps of:
   (i) preparing RNA from a biological specimen;
   (ii) separating the RNA prepared in the step (i) by electrophoresis;
   (iii) hybridizing the RNA separated in the step (ii) with a labeled nucleotide probe which hybridizes IP-10 polynucleotide under a stringent condition;
   (iv) using the amount of the label hybridized in the step
   (iii) as an indicator of expression of IP-10 polynucleotide and comparing it with a result of a normal biological specimen; and
   (v) using the amount of expression of IP-10 polynucleotide being significantly different from the result of the normal biological specimen as an indicator of the degree of abnormality or its risk associated with the biological activity;
(25) A method for measuring a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the method comprising at least the steps of:
   (i) preparing RNA from a biological specimen;
   (ii) generating a first cDNA strand by using the RNA prepared in the step (i) as a template with a dT primer;
   (iii) amplifying IP-10 polynucleotide by PCR using the cDNA generated in the step (ii) as a template with a primer set for amplifying the IP-10 polynucleotide;
   (iv) separating the PCR product in the step (iii) by electrophoresis;
   (v) hybridizing the PCR product separated in the step (iv) with a labeled nucleotide probe which hybridizes IP-10 polynucleotide under a stringent condition;
   (vi) using the amount of the label hybridized in the step
   (v) as an indicator of expression of IP-10 polynucleotide and comparing it with a result of a normal biological specimen; and
   (vii) using the amount of expression of IP-10 polynucleotide being significantly different from the result of the normal biological specimen as an indicator of the degree of abnormality or its risk associated with the biological activity;
(26) A method for measuring a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the method comprising at least the steps of:
   (i) treating a biological specimen for tissue fixation;
   (ii) sectioning the fixed tissue prepared in the step (i);
   (iii) staining the sectioned tissue immunohistologically with an antibody capable of recognizing IP-10;
   (iv) comparing the degree of the immunohistological dyed biological specimen with that of a normal biological specimen; and
   (v) using the amount of IP-10 protein being significantly different from the result of the normal biological specimen as an indicator of the degree of abnormality or its risk associated with the biological activity;
(27) A method for measuring a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the method comprising at least one step selected from the group consisting of:
   (i) amplifying IP-10 polynucleotide in the biological specimen by PCR using a primer set for amplifying the IP-10 polynucleotide;
   (ii) analyzing a nucleic acid fraction separated from the biological specimen by using (I) an oligonucleotide or polynucleotide which hybridizes with IP-10 polynucleotide under a stringent condition or (II) a nucleic acid array provided with IP-10 polynucleotide; and
   (iii) hybridizing a nucleic acid fraction separated from the biological specimen with an oligonucleotide or polynucleotide which hybridizes with IP-10 polynucleotide under a stringent condition; and
   a step of measuring the amount of IP-10 polynucleotide in the biological specimen, and then comparing the amount of the IP-10 polynucleotide in the specimen with that in a normal biological specimen;
(28) The method according to any one of the above (23) to (27) for measuring abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      which comprises quantitatively measuring IP-10 protein or expression of IP-10 polynucleotide;
(29) A reagent used in the method according to any one of the above (23) to (27) for measuring abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the reagent containing (i) an antibody capable of recognizing IP-10, (ii) an antibody capable of binding an epitope on IP-10 which is different from that recognized by the antibody (i), (iii) an immobilized antibody (i) or antibody(ii), or (iv) a labeled antibody (i) or antibody (ii);
(30) A method for measuring or diagnosing a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      which comprises measuring the amount of IP-10 protein or expression of IP-10 polynucleotide in a sample by using an antibody capable of recognizing IP-10; and
(31) A reagent for measuring or diagnosing a degree of abnormality or its risk associated with a biological activity selected from the group consisting of:
   (a) activating conceptus migration, (b) promoting conceptus implantation on the uterine wall, (c) treating sterility,
   (d) promoting pregnancy, (e) controlling interaction between conceptus and maternal system, (f) activating immunocyte migration, and (g) controlling immune function in the uterus,
      the reagent containing an antibody capable of recognizing IP-10 for measuring the amount of IP-10 protein or expression of IP-10 polynucleotide in a sample.

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### BRIEF DESCRIPTION OF THE DRAWINGS

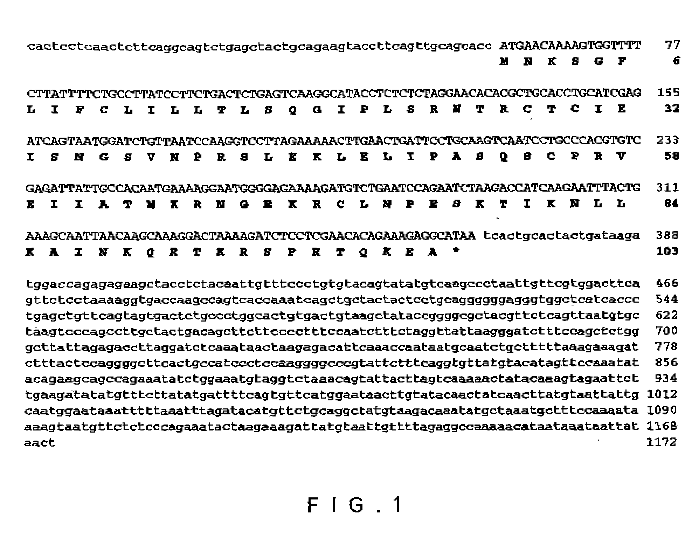

FIG. 1 shows a nucleotide sequence of ovine IP-10 and an amino acid sequence deduced from thereof.
FIG. 2 illustrates the comparison of IP-10 amino acid sequences of ovine, goat, human, and mouse. Four cysteine residues are conserved in these animals, but a glutamine-leucine-arginine (ELR) motif preceding the two cysteine residues from the N-terminal is not present. The homology of ovine IP-10 to human IP-10 is a higher than that of mouse IP-10. (see Table 1).

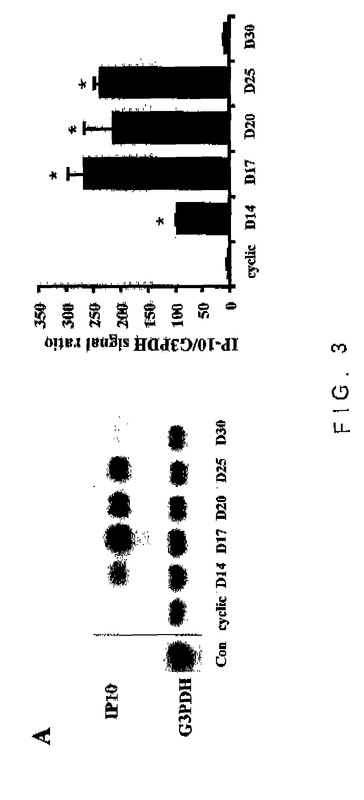

FIG. 3 shows levels of IP-10 mRNA expression in the ovine uterus during implantation examined by Northern blot analysis, an electrophoresis picture(the left) and a graph of densitometric analysis (the right) of the Northern blot analysis.

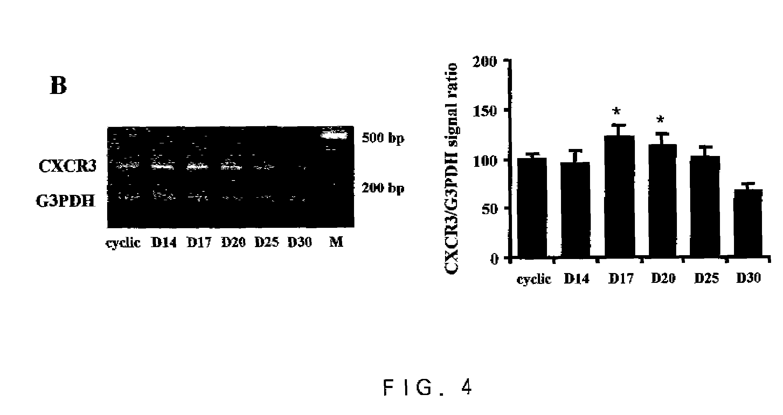

FIG. 4 shows an electrophoresis picture (the left) of the results of semiquantitative PCR of CXCR3 mRNA and G3PDH mRNA in the uterus of pregnant ewes, and a graph (the right) of densitometric analysis of the semiquantitative PCR products for CXCR3 mRNA and G3PDH mRNA.

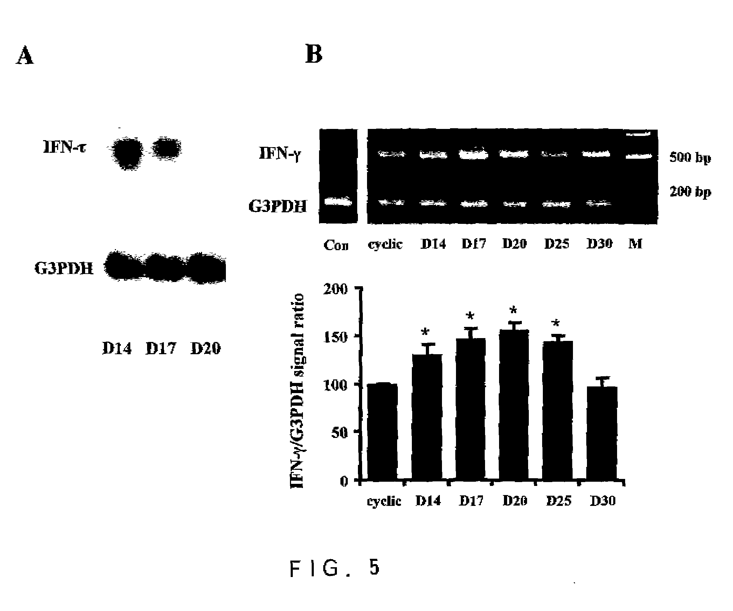

FIG. 5 shows levels of IFN-τ and IFN-γ mRNA in the ovine conceptus and uterus during implantation, A (the left) shows the results of Northern blot analysis of IFN-τ mRNA in the conceptuses of pregnant ewes, and B (the right) shows an electrophoresis picture (the top) of the results of semiquantitative PCR of IFN-γ mRNA and G3DPH mRNA in the ovine uterus and conceptus and a graph (the bottom) of densitometric analysis.

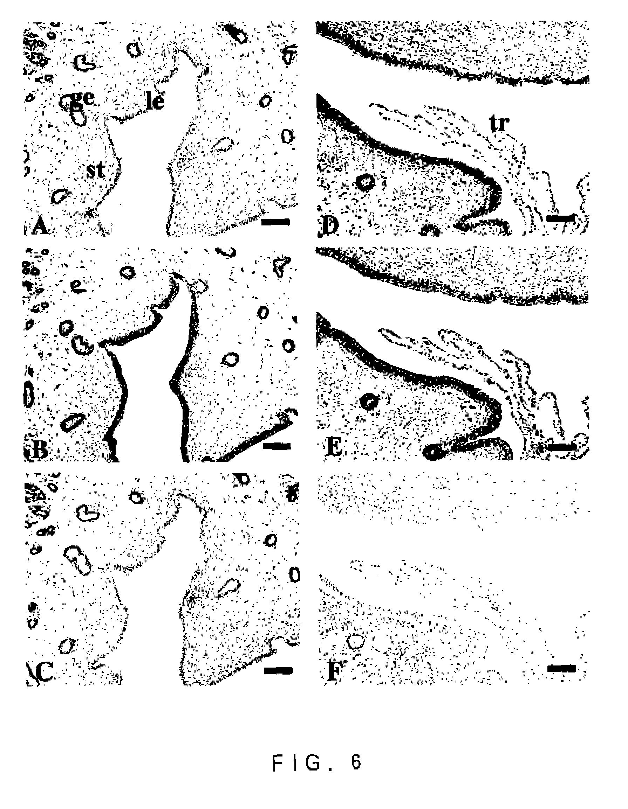

FIG. 6 is pictures of biological tissues in immunohistochemical analysis of ovine uterus and conceptus tissue sections for IP-10 and IFN-γ, in which le means luminal epithelium, ge means glandular epithelium, st means subepithelial stroma, and tr means trophoblast cell; and the scale bar equals to 100 µm.

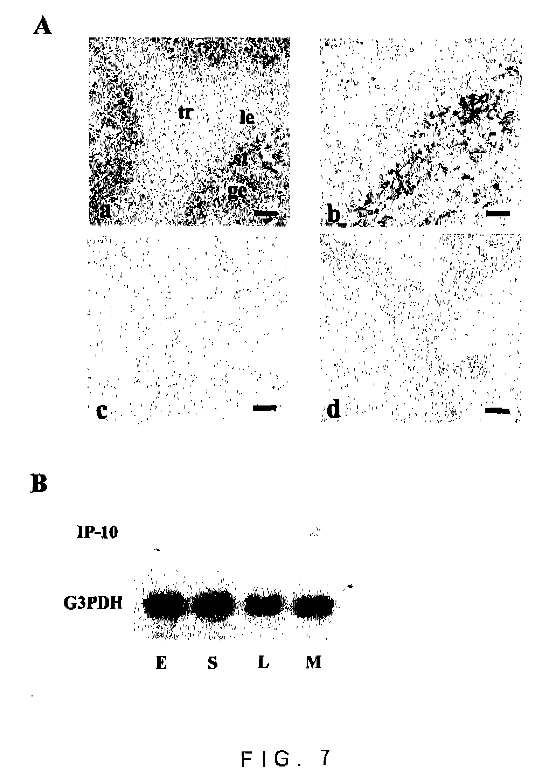

FIG. 7 is pictures of biological tissues in situ hybridization analysis of IP-10 in the ovine uterus, in which le means luminal epithelium, ge means glandular epithelium, st means subepithelial stroma, and tr means trophoblast cell; and the scale bars equal to 40 µm in a, c, and d, and equal to 10 µm in b.

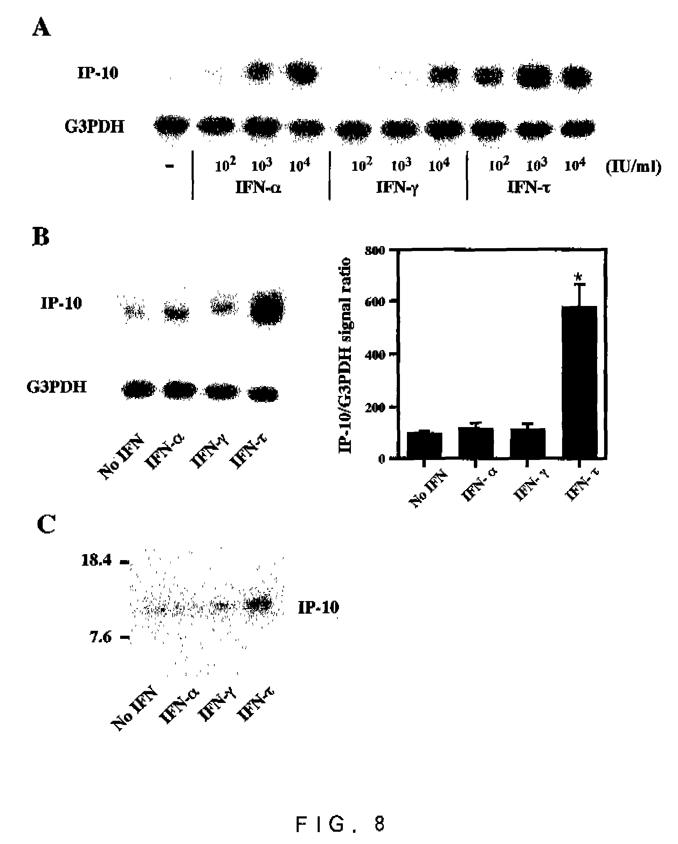

FIG. 8 is pictures, in which A shows electrophoresis pictures showing effect of IFNs on IP-10 mRNA levels, B shows effects of IFNs on IP-10 mRNA levels in the endometrial explants from cyclic ewes, electrophoresis pictures (the left in B) of Northern blot analysis of IP-10 mRNA in the endometrial explants stimulated by predetermined amounts of IFNs, and a graph (the right in B) of densitometric analysis of Northern blot analysis of IP-10 mRNA and G3PDH mRNA, and C shows an electrophoresis picture of Western blot analysis of IP-10 in the culture medium from endometrial explants stimulated by IFNs.

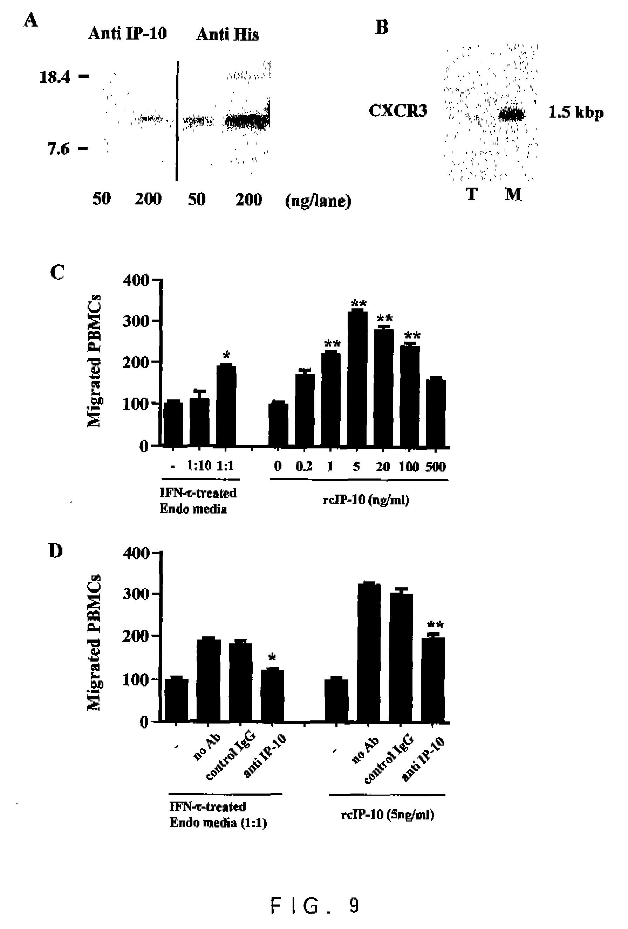

FIG. 9 shows effects of IFN-τ-stimulated endometrial culture medium and recombinant IP-10 on migratory activity of PBMCs, in which A shows the results of Western blot analysis of recombinant caprine IP-10 (IP-10), B shows the results of Northern blot analysis of CXCR3 mRNA in PBMCs, C shows migration of PBMCs of IFN-τ-treated or untreated endometrial cultures and IP-10, and D shows migration of PBMCs of IFN-τ-treated or rcIP-10-treated endometrial cultures in the presence of an anti-IP-10 antibody.

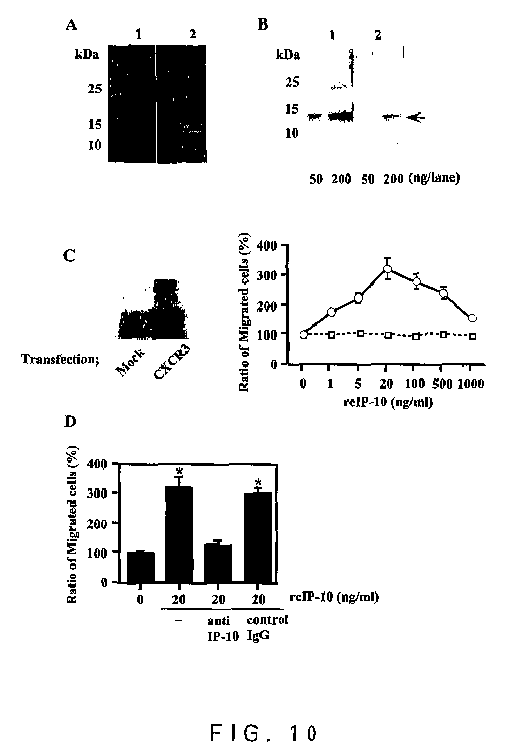

FIG. 10 shows the results of preparation of a recombinant caprine IP-10 protein and its antibodies, in which A shows the results of SDS-PAGE of cell lysates, before (lane 1) and after (lane 2) purification using a nickel-chelating column, of Escherichia coli BL21-SI expressing recombinant caprine IP-10 (rcIP-10); B shows the results of Western blot analysis of purified rcIP-10 protein with anti-His-tag antibody (lane 1) and anti-caprine IP-10 antibody (lane 2); the left in C shows the results of Northern blot analysis of CXCR3 mRNA, using RNA extracted from KU-1 cells that were transiently transfected with pcDNA3.1-caprine CXCR3 (CXCR3) or with parental pcDNA3.1 (Mock); the right of C shows the results of chemotaxis assay of biological activity of rcIP-10 to CXCR3 transfected KU-1 cells expressing (○) and not expressing (□) CXCR3; and D shows the results of chemotaxis assay of KU-1 cells in the presence of rcIP-10, to which pretreated with the anti-caprine IP-10 antibody (anti IP-10), no further treatment applied (-), or pretreated with control rabbit IgG (control IgG).

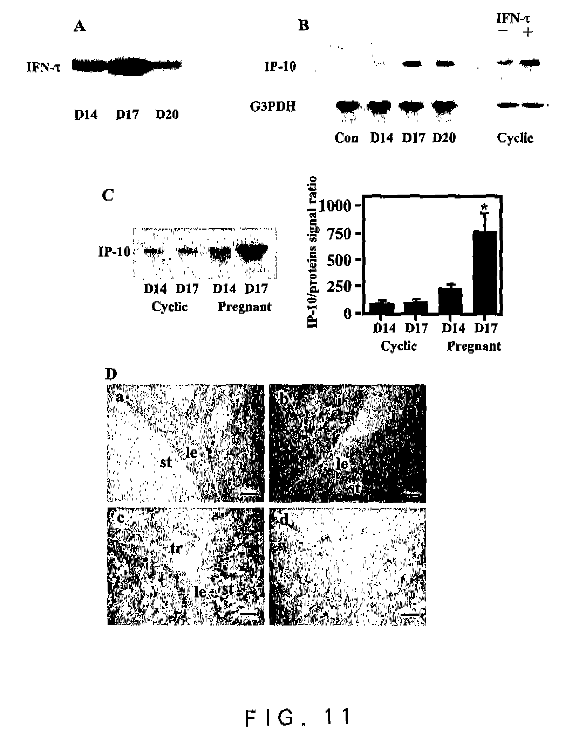

FIG. 11 shows expression of IP-10 in the early pregnant caprine uterus, in which A shows the results of Western blot analysis for presence of IFN-τ in the culture medium derived from caprine conceptuses on Days 14 (D14), Day 17 (D17), and Day 20 (D20) of pregnancy; B shows the results of Northern blot analysis of endometrial IP-10 mRNA using RNA from pregnant goat (panel left) and from cyclic goat (panel right) stimulated with rcIFN-τ; the left of C shows the results of Western blot analysis of IP-10 in the uterine flushing media from cyclic and pregnant goats; the right of C shows densitometric analysis of Western blot of IP-10; and D shows in situ hybridization analysis of IP-10 mRNA in the caprine uterus.

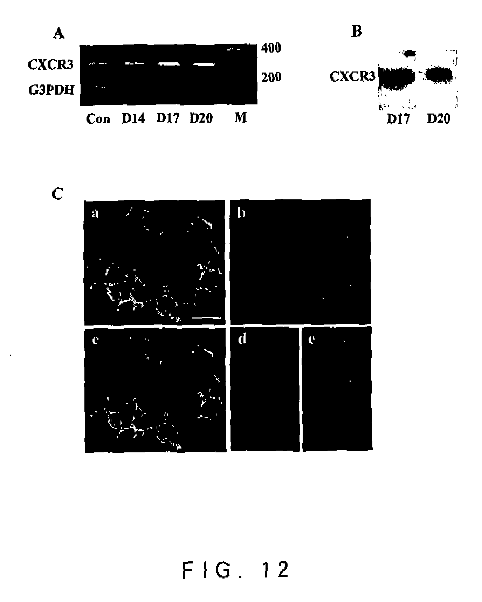

FIG. 12 shows expression and cellular localization of CXCR3 in the caprine conceptuses, in which A shows levels of CXCR3 mRNA expression examined by RT-PCR; B shows CXCR3 mRNA expression confirmed by Northern blot analysis; and C shows CXCR3 expression examined by immunofluorescence analysis using anti-CXCR3 antibody.

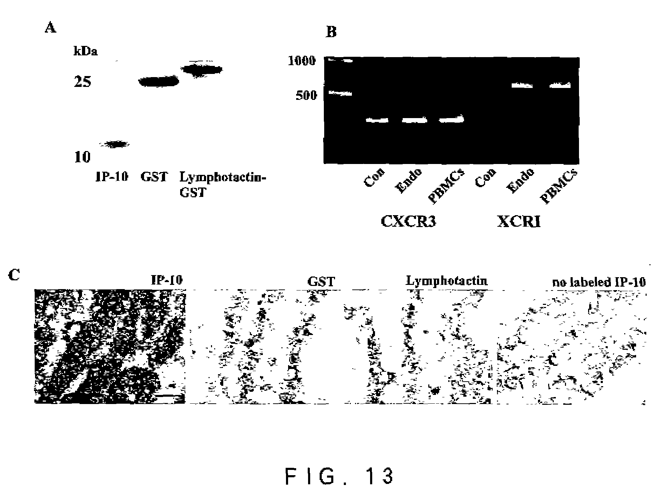

FIG. 13 shows binding of rcIP-10 to the caprine trophoblast cells, in which A shows the rate of biotinylation of recombinant proteins; B shows expression of IP-10 receptor CXCR3 mRNA and lymphotactin receptor XCR1 mRNA confirmed by RT-PCR; and C shows visualized on Day 17 of pregnant caprine conceptuses by using biotinylated protein and horseradish peroxidase labeled streptavidin.

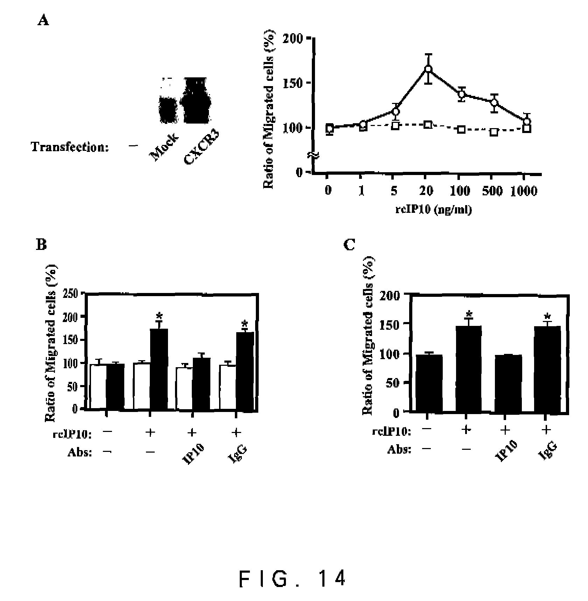

FIG. 14 shows stimulation of rcIP-10 on the migration of CXCR3-expressing conceptus cells, in which the left of A shows the results of Northern blot analysis of RNA extracted from HTS-1; the right of A shows the results of chemotaxis assay of HTS-1; B shows effect of rcIP-10 on migration activity of HTS-1 transfected with CXCR3 expression plasmid; and C shows the results of chemotaxis assay for primary trophoblast cells, on Day 17 of pregnancy, expressing CXCR3.

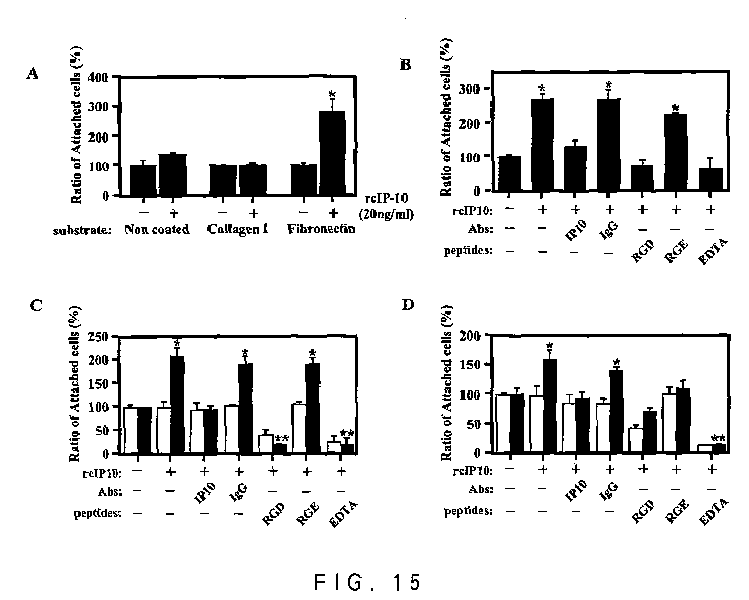

FIG. 15 shows effect of rcIP-10 on the adhesion of trophoblast cells to fibronectin or endometrial epithelial cells, in which A shows adhesion of caprine trophoblast cells (on Day 17 of pregnancy) stimulated with rcIP-10; B shows effect of rcIP-10 on adhesion of trophoblast cells to fibronectin; C shows effect of rcIP-10 on adhesion of HTS-1 transfected with caprine CXCR3 expression vector to fibronectin; and D shows effect of rcIP-10 on endometrial epithelial cells for HTS-1 transfected with caprine CXCR3 expression vector.

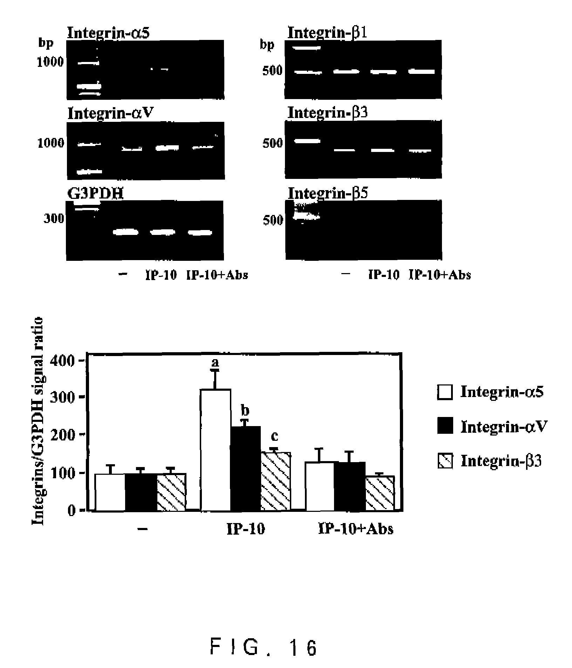

FIG.16 shows expression of integrin subunits in the caprine trophoblast cells activated with rcIP-10, in which upper shows RT-PCR analysis of integrin α5, αV, β1, β3, and β5 subunits mRNAs for HTS-1 cells transfected with caprine CXCR3 expression vector [IP-10+Abs: rcIP-10 (20 ng/ml) and pretreatment with anti-IP-10 antibody (30 µg/ml), IP-10: rcIP-10 (20 ng/ml)]; and lower shows densimetric analysis of PCR products.

### BEST MODES OF CARRYING OUT THE INVENTION

In the specification, "IP-10" means a 10 kDa protein induced by interferon-γ(interferon-γ inducible protein 10 kDa). This protein factor may indicate IP-10 belongs to a chemokine family that controls inflammatory and immune responses in many aspects mainly through chemotactic activity toward subsets of leukocytes. The IP-10 may be the protein identified during the studies for mice and humans as belonging to C-X-C chemokine and being induced in a various cells such as macrophages, fibroblasts, astrocytes, keratinocytes, epithelial cells, and endothelial cells. The IP-10 may preferentially act on NK cells and activated T cells (phenotype Th1) through C-X-C chemokine receptor 3 (CXCR3).

Bovine IP-10 has been particularly investigated in order to identify the specific activities during implantation. Therefore, any ungulate IP-10 is included in the present invention from the viewpoint of these specific characteristics. Furthermore, human IP-10, its derivatives, analogues, equivalents, and the like are included from the viewpoint of using the activities during implantation.

Ovine IP-10 is a peptide composed of 102 amino acid residues and has four cysteine residues. The four cysteine residues are conserved within a chemokine family, and the first two cysteines are separated by a single amino acid residue to form a C-X-C motif. Similar to IP-10s of other animals, ovine IP-10 lacks ELR motif preceding the first two cysteine residues and has a high similarity to human IP-10 with 82.7% homology in the nucleotide sequence and 75.5% homology in the amino acid sequence. IP-10s including ovine IP-10 according to the present invention have a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus.

Typically, IP-10s including ovine IP-10 and caprine IP-10 according to the present invention may be native peptides present in living body (endogenous peptides). A typical IP-10 according to the present invention is generated by a sequence encoded by SEQ ID NO: 1 to be generated, for example, polypeptides having an amino acid sequence shown by SEQ ID NO: 2 or its essentially equivalent amino acid sequences. A typical IP-10 according to the present invention includes at least 1 to 102 continuous amino acid residue of the amino acid sequence shown by SEQ ID NO: 2, and has a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus. Furthermore, IP-10 according to the present invention includes a polypeptide exhibiting characteristics as in above and having a homology to the amino acid sequence shown by SEQ ID NO: 2 at least 60%, preferably at least 75.5%, still more preferably at least 82.7%, even more preferably at least 90%, and most preferably at least 95%. Novel one is the specifically preferable.

Examples of "polypeptide" according to the present invention include IP-10, and relating polypeptides thereof, especially ungulate IP-10s such as ovine IP-10 and caprine IP-10. As for pregnancy, the IP-10 and its relating polypeptides may be derived from human from the viewpoint of using an activity during implantation. Examples of the polypeptide include ovine IP-10, typically, peptides having a homology with an amino acid sequence shown by SEQ ID NO: 2 at least 60%, preferably at least 70%, still more preferably at least 80%, even more preferably at least 85%, even more preferably at least 90%, even more preferably 95%, and most preferably at least 97%, and having a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus, or substantially equivalent biological activity such as antigenicity.

IP-10s according to the present invention include IP-10 molecules derived from human and ungulates such as ovine and goat, and novel amino acid sequences each having a distinctive domain or motif or a segment thereof within IP-10, from the viewpoint of using an activity during implantation for pregnancy. More preferably, peptides according to the present invention include amino acid sequences having an activity during implantation for pregnancy described above, and having a homology with each IP-10 family at least 60%. Typically, the peptide according to the present invention is selected from the group consisting of peptides containing an amino acid sequence substantially equivalent to the amino acid sequence shown by SEQ ID NO: 2. Furthermore, the peptide according to the present invention includes a part or whole amino acid sequence shown by SEQ ID NO: 2. These sequences are included in the present invention.

The term "homologous" or "homology" as used herein means the amount (or number) of amino acid residues or bases, which are identified as the same with another amino acid residues or bases from polypeptide sequence (or amino acid sequence) or polynucleotide sequence (or nucleotide sequence), respectively, in the relation of compatibility between the two chains, i.e. a degree of sequence correlation in two polypeptide sequences or in two polynucleotide sequences. Homology can be easily calculated. Many methods for measuring a homology between two polynucleotide sequences or polypeptide sequences are known. A term of homology (or identity) is widely known to those skilled in the art. (For example, Lesk, A. M. (Ed.), (1988) Computational Molecular Biology, Oxford University Press, New York; Smith, D. W. (Ed.), (1993) Biocomputing: Informatics and Genome Projects, Academic Press, New York; Grifin, A. M. & Grifin, H. G. (Ed.), (1994) Computer Analysis of Sequence Data: Part I, Human Press, New Jersey; von Heinje, G., (1987) Sequence Analysis in Molecular Biology, Academic Press, New York; Gribskov, M. & Devereux, J. (Ed.), (1991) Sequence Analysis Primer, M-Stockton Press, New York). The general methods for determining a homology between two sequences are, but not limited to, disclosed in Martin, J. Bishop (Ed.), (1994) Guide to Huge Computers, Academic Press, San Diego; Carillo, H. & Lipman, D., (1988) SIAM J. Applied Math., 48: 1073, etc. Preferable methods for determining a homology are designed to identify the most compatible parts in two sequences of a question. These methods may be assembled in a computer program. Examples of the preferable computer program for determining a homology between two sequences include, but not limited to, GCG program package (Devereux, J. et al., (1984) Nucleic Acids Research, 12(1): 387), BLASTP, BLASTN, and FASTA (Atschul, S. F. et al., (1990) J. Molec. Biol., 215: 403). Methods widely known in the art can be also used.

A nucleic acid encoding each polypeptide or protein according to the present invention, from the viewpoint of using the activity during implantation for pregnancy described above, may include within the sequence a nucleotide sequence encoding a member selected from IP-10, IP-10-relating polypeptides, or their segments having continuous amino acid residues; or a nucleotide sequence encoding a member selected from IP-10 derived from ungulates such as ovine and goat, its relating polypeptides, IP-10 derived from human, its relating polypeptides, or their continuous amino acid sequence segments. A typical nucleic acid contains a nucleotide sequence encoding the peptide shown by SEQ ID NO: 2 or its segments having continuous amino acid residues, for example, a nucleotide sequence composed of at least positions 63-365 of the nucleotide sequence shown by SEQ ID NO: 1, a nucleotide sequence composed of from ATG of 60-62 positions to TAA of positions 366-368 of the nucleotide sequence shown by SEQ ID NO: 1 (the termination codon TAA may be TGA or TAG), and a nucleotide sequence added an initiation codon (i.e. codon encoding Met) and a termination codon. Furthermore, any nucleic acid can be included as long as the nucleic acid containing a nucleotide sequence equivalent to the above nucleotide sequences, for example, a nucleic acid containing a nucleotide sequence encoding a peptide containing an amino acid sequence having a homology with the proteins encoded by the above-mentioned nucleotide sequences at least 80%, 85%, 90%, 95%, or 98%, and containing at least 1 to 16 continuous amino acid residues of the amino acid sequence shown by SEQ ID NO: 2, and also having a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus, or a substantially equivalent biological activity such as antigenicity. Examples of the nucleic acid encoding IP-10 are single-stranded DNA, double-stranded DNA, RNA, DNA:RNA hybrid, synthetic DNA, and the like. The nucleic acid may be an ovine, caprine, or human genomic DNA; an ovine, caprine, or human genomic DNA library; cDNA derived from ovine or human tissues (or cells); or a synthetic DNA. The nucleotide sequence of the nucleic acid encoding IP-10 may be modified (for example, addition, deletion, and substitution). These modified nucleotide sequences are also included in the present invention. The nucleic acid according to the present invention may encode a peptide or its segment of the present invention as described below, and DNA is preferable. The term "nucleotide sequence equivalent" means, for example, a nucleotide sequence capable of hybridizing with a continuous at least 5 nucleotide sequences, preferably at least 10, more preferably at least 15, still more preferably at least 20 nucleotide sequence in the nucleotide sequence shown SEQ ID NO: 1 under a stringent condition, and substantially equivalent to IP-10. The stringent condition is a condition enabling selectively detectable specific binding of a predetermined polynucleotide with an oligonucleotide probe or a polynucleotide probe. The stringent condition is defined by a salt content, an organic solvent (for example, formamide), temperature, and other known conditions. Namely, the stringency increases by decreasing a salt content, increasing an organic solvent content, or raising a hybridization temperature. For example, a stringent salt content is generally lower than about 750 mM NaCl and lower than about 75 mM sodium citrate, more preferably, lower than about 500 mM NaCl and lower than about 50 mM sodium citrate, and most preferably, lower than about 250 mM NaCl and lower than about 25mM sodium citrate. A stringent organic solvent content is higher than about 35% formamide, most preferably, higher than about 50%. A stringent temperature is higher than about 30°C, more preferably, higher than about 37°C, and most preferably, higher than about 42°C. Other conditions are a hybridization time, a washing agent (for example, surfactant such as SDS) content, and the presence of a carrier DNA, and the like. Combination of these conditions defines various stringent conditions. For example, a condition defined by a sodium content of about 19 to 20 mM and a temperature of about 60°C to 65°C is preferable, more preferably, a sodium content of about 19 mM and a temperature of about 65°C.

The confirmation of predetermined gene products may be performed by using appropriate animal cells such as 293T cell and COS-1 cell, which are transfected with the genes. The introduction of the foreign genes to animal cells such as mammal cells can be performed by methods known in the field of the art or by substantially equivalent methods thereof, such as a calcium-phosphate method (for example, F. L. Graham et al.,(1973) Virology, 52: 456), a DEAE-dextran method (for example, D. Warden et al.,(1968) J. Gen. Virol., 3: 371), an electroporation method (for example, E. Neumann et al., (1982) EMBO J, 1: 841), a micro-injection method, a liposome method, a viral infection method, and a phage particle method. Gene products produced by animal cells transfected with a predetermined gene, for example, IP-10 gene can be analyzed.

Any plasmid can be used as a plasmid incorporating DNA prepared according to the present invention (for example, IP-10 gene) as long as the plasmid can express the DNA in a host cell (for example, prokaryotes such as Escherichia coli and Bacillus subtilis, yeast, eukaryotic cells such as CHO cell and COS cell, and insect cells such as sf21) widely used in a genetic engineering field. Such sequences may contain, for example, a modified codon appropriate for the expression in a selected host cell, a restriction enzyme moiety, a controlling or promoting sequence for the readily expression of a target gene, a linker and adaptor for binding a target gene, and also useful sequences (including genes encoding hybrid proteins and fused proteins) for the control of antibiotics or the like, metabolism, and selection. Preferably, appropriate promoters are used. For example, plasmids using Escherichia coli as a host cell may use a tryptophan promoter (trp), a lactose promoter (lac), a tryptophan-lactose promoter (tac), a lipoprotein promoter (1pp), λ-phage P_{L} promoter, etc., plasmids using animal cells as a host cell may use SV40 late promoter, MMTV LTR promoter, RSV LTR promoter, CMV promoter, SRα promoter, etc., and plasmids using yeast as a host cell may use GAL1, GAL10 promoters, etc.

Examples of the plasmid using Escherichia coli as a host cell include pBR322, pUC18, pUC19, pUC118, pUC119, pSP64, pSP65, pTZ-18R/-18U, pTZ-19R/-19U, pGEM-3, pGEM-4, pGEM-3Z, pGEM-4Z, pGEM-5Zf(-), and pBluescript KS™ (Stratagene). Examples of a plasmid vector suitable for expression in Escherichia coli include pAS, pKK223 (Pharmacia), pMC1403, pMC931, pKC30, and pRSET-B (Invitrogen). Examples of the plasmids using animal cells as host cells include SV40 vector, polyoma virus vectors, vaccinia virus vectors, retrovirus vectors, for example, pcD, pcD-SRα, CDM8, pCEV4, pME18S, pBC12BI, and pSG5 (Stratagene). Examples of the plasmid using yeast as a host cell include YIp type vectors, YEp type vectors, YRp type vectors, and YCp type vectors, for example, pGPC-2. As for the host cells, examples of Escherichia coli as the host cell include Escherichia coli K12 strain-derived strain such as NM533, XL1-Blue, C600, DH1, DH5, DH11S, DH12S, DH5α, DH10B, HB101, MC1061, JM109, and STBL2, and B834 strain-derived strain such as BL21(DE3)pLysS. Examples of the animal cells as the host cell include African green monkey fibroblast-derived COS-7 cells, COS-1 cells, CV-1 cells, mouse fibroblast-derived COP cells, MOP cells, WOP cells, Chinese hamster cell-derived CHO cells, CHO DHFR⁻ cells, human HELA cells, murine cell-derived C127 cells, and murine cell-derived NIH 3T3 cells. Regarding the insect cells, Bombyx mori nuclear polyhedrosis virus or a derivative thereof may be used as a vector, and silkworm larvae or cultured silkworm cells such as BM-N cells may be used. It is also possible to use plant cells as host cells, the plant cells are widely known in the field of the art as well as appropriate vectors. In the genetic engineering technique according to the present invention, restriction enzymes, reverse transcriptase, DNA modifying or decomposing enzymes for modifying or converting DNA fragments to structures suitable for cloning, DNA polymerases, terminal nucleotidyl transferases, and DNA ligases can be used. Examples of vectors suitable for DNA gene cloning to construct DNA libraries include plasmids, λ phages, cosmids, P1 pharges, F factor, and YAC, preferably, the λ phage-derived vectors such as Charon 4A, Charon 21A, λgt10, λgt11, λDASHII, λFIXII, λEMBL3, and λZAPII™ (Stratagene).

A transformant according to the present invention is obtained by transformation with an expression vector containing a nucleic acid encoding a protein of the present invention and a cell line of the transformant having high and stable expression capability can be obtained by repeating cloning optionally using an appropriate selective marker. For example, when the dhfr gene is utilized as the selective marker in transformant cells derived from animal cells as host cells, a cell line, which amplifies a DNA encoding the protein of the present invention and causes a higher level of expression, can be obtained by culturing as gradually increasing the MTX concentration, and then by selecting a resistant strain. When the transformant of the present invention is cultured under conditions enabling the expression of a nucleic acid encoding the protein of the present invention, the transformant can produce and accumulate the target product. The transformant can be cultured in a medium that is commonly used in the art. For example, a liquid medium can be suitably used for a transformant derived from a prokaryotic cell such as Escherichia coli and Bacillus subtilis or from yeast as a host cell. The medium can contain a carbon source, a nitrogen source, inorganic substances, and so forth which are necessary for the growth of the transformant. Examples of the carbon source include glucose, dextrin, soluble starch, and sucrose. Examples of the nitrogen source include inorganic or organic substances such as ammonium salts, nitrate salts, corn steep liquor, peptone, casein, meat extract, malt extract, soybean cake, potato extract. The inorganic substances include calcium chloride, sodium dihydrogenphosphate, magnesium chloride, and calcium carbonate. Yeast, vitamins, casamino acids, growth promoters, and the like may be also added. An agent such as 3β-indolylacrylic acid is optionally added so that the promoter can efficiently function. Preferably, the medium has a pH of about 5 to 8.

In the case of Escherichia coli, the culture is generally performed at about 15°C to 45°C for about 3 to 75 hours. Aeration and stirring are performed if necessary. In the culture of a transformant derived from animal cells as host cells, a medium such as MEM medium containing about 5% to 20% fetal bovine serum, PRMI 1640 medium, or DMEM medium is used. The pH is preferably about 6 to 8. The culture is generally performed at about 30°C to 40°C for about 15 to 72 hours. If necessary, aeration and stirring are performed. In the extraction from the cells cultured as in above, the bacteria or cells are collected by a conventional method after the culture. For example, the collected bacteria or cells are suspended in an appropriate buffer solution and then disrupted by, for example, sonication, lysozyme, and/or freezing and thawing. Then, a crude extract is prepared by centrifugation or filtration. A protein denaturing agent such as urea and guanidine hydrochloride, and a surfactant such as Triton X-100 (trademark) and Tween-80 (trademark) may be added to the buffer solution. When the target product is secreted into the culture fluid, the supernatant is collected by separation from the bacteria or cells by a per se known method after the completion of the culture. The target product in the resulting culture supernatant or extract can be purified by an appropriate combination of per se known methods of separation and purification. The product can be purified by, for example, salting out with ammonium sulfate, gel filtration using Sephadex, ion exchange chromatography using a carrier having diethylaminoethyl or carboxymethyl groups, hydrophobic chromatography using a carrier having hydrophobic groups such as butyl, octyl, or phenyl groups, dye gel chromatography, electrophoresis, dialysis, ultrafiltration, affinity chromatography, and high-performance liquid chromatography. Preferably, the product can be purified and isolated by the treatment with polyacrylamide gel electrophoresis, or affinity chromatography utilizing an immobilized ligand or the like. For example, gelatin-agarose affinity chromatography and heparin-agarose chromatography may be used.

Furthermore, modified proteins corresponding to IP-10 (for example, human IP-10, ovine IP-10, and caprine IP-10) as resulting from mutation such as substitution, deletion, insertion, translocation and addition of one or a plurality of amino acids of the IP-10 can be produced based on the nucleotide sequence if the gene according to the present invention by using a method generally used in genetic engineering. As the methods of such mutation, conversion, or modification are described in Japanese Biochemical Society (ed.), (1986) "Zoku Seikagaku Jikken Koza (Experiments in Biochemistry, Second Series) 1, Idensi Kenkyuho (Methods of Gene Studies) II", p. 105 (Susumu Hirose), Tokyo Kagaku Dozin; Japanese Biochemical Society (ed.), (1992) "Shin Seikagaku Jikken Koza (Experiments in Biochemistry, Updated) 2, Kakusan (Nucleic Acids) III (Recombinant DNA Technology", p. 233 (Susumu Hirose), Tokyo Kagaku Dozin; R. Wu, L. Grossman, ed., (1987) "Methods in Enzymology", Vol. 154, p. 350 & p. 367, Academic Press, New York; R. Wu, L. Grossman, ed., (1983) "Methods in Enzymology", Vol. 100, p. 457 & p. 468, Academic Press, New York; J. A. Wells, et al., (1985) Gene, 34: 315; T. Grundstroem, et al., (1985) Nucleic Acids Res., 13: 3305; J. Taylor, et al., (1985) Nucleic Acids Res., 13: 8765; R. Wu, ed., (1987) "Methods in Enzymology", Vol. 155, p. 568, Academic Press, New York; A. R. Oliphant, et al., (1986) Gene, 44: 177. For example, there may be mentioned methods such as site-directed mutagenesis (site-specific mutagenesis) using a synthetic oligonucleotide (Zoller, et al., (1987) Nucl. Acids Res., 10: 6487; Carter, et al., (1986) Nucl. Acids Res., 13: 4331), Cassette mutagenesis (Wells, et al., (1985) Gene, 34: 315), Restriction selection mutagenesis (Wells, et al., (1986) Philos. Trans. R. Soc. London Ser A, 317: 415), alanine scanning (Cunningham & Wells, (1989) Science, 244: 1081-1085), PCR mutagenesis, Kunkel method, dNTP[αS] method (Eckstein), and region-directed mutagenesis using sulfurous acid or nitrous acid.

Further, the resulting proteins (or peptides) according to the present invention can be modified by amino acid residue contained therein by a chemical technique, or can be altered into its derivatives by modification or partial decomposition by enzymes such as peptidases, e.g., pepsin, chymotrypsin, papain, bromelain, endopeptidase, and exopeptidase. In general, the protein according to the present invention has a carboxyl group (-COOH) or a carboxylate group (-COO⁻) at the C-terminal, but may have an amido group (-CONH₂) or an ester (-COOR) at the C-terminal. The R in the ester may be a C₁₋₆ alkyl group such as methyl, ethyl, n-propyl, isopropyl, and n-butyl, a C₃₋₈ cycloalkyl group such as cyclopentyl and cycloalkyl, a C₁₋₁₂ aryl group such as phenyl and α-naphthyl, or a C₇₋₁₄ aralkyl group including a phenyl-C₁₋₂ alkyl group such as benzyl and phenethyl, and an α-naphthyl-C₁₋₂ alkyl group such as α-naphthylmethyl, as well as a pivaloyloxymethyl group which is widely used as an oral administration ester. When the protein according to the present invention has a carboxy group (or carboxylate) other than at C-terminal, the carboxy group may be amidated or esterified. Such proteins are also included in the present invention. Such esters are the same as the ester at C-terminal described above.

Furthermore, examples of the above-mentioned proteins according to the present invention include proteins of which methionine residue at the N-terminal is protected by, for example, a C₁₋₆ acyl group such as a C₁₋₅ alkyl-carbonyl group, e.g. formyl and acetyl; proteins having pyroglutamated N-terminal which is converted from a glutamyl group generated by breakage of N-terminal in living body, proteins of which a substitute (e.g. -OH, -COOH, amino group, imidazole group, indol group, guanidine group) on a side chain is protected by a protecting group (i.e. C₁₋₆ acyl group such as formyl, acetyl), or proteins on which a sugar chain is bound, i.e. conjugated protein such as glycoprotein. In producing by gene recombinant technology, it is possible to cause expression of IP-10 in the form of a fused protein. The resulting IP-10 may be changed or processed in vivo or in vitro into a protein having substantially the same biological activity as native IP-10. The fusion protein can be produced by a gene-engineering method widely used, and can be purified by affinity chromatography utilizing the fused portion thereof. Examples of the fused protein include proteins fused to histidine tag, or β-galactosidase (β-gal), maltose-binding protein (MBP), glutathione-S-transferase (GST), thioredoxin (TRX), or Cre recombinase amino acid sequence. Similarly, a heterogeneous epitope tag is added to the polypeptide for enabling purification by immunoaffinity chromatography using an antibody specifically binding to the epitope. In a more suited embodiment of the epitope, AU5, c-Myc, CruzTag 09, CruzTag 22, CruzTag 41, Glu-Glu, HA, Ha.11, KT3, FLAG (registered trademark, Sigma-Aldrich), Omni-probe, S-probe, T7, Lex A, V5, VP16, GAL4, and VSV-G are mentioned as examples. See, Field et al., (1988) Molecular and Cellular Biology, 8: 2159-2165; Evan et al., (1985) Molecular and Cellular Biology, 5: 3610-3616; Paborsky et al., (1990) Protein Engineering, 3(6): 547-553; Hopp et al., (1988) BioTechnology, 6: 1204-1210; Martin et al., (1992) Science, 255: 192-194; Skinner et al., (1991) J. Biol. Chem., 266: 15163-15166; Lutz-Freyermuth et al., Proc. Natl. Acad. Sci. USA, 87: 6393-6397 (1990); etc.).

Further, the fused protein may be labeled with a marker to be detectable. In a more preferred embodiment, the detectable marker may be Biotin Avi tag based on the biotin/streptavidin, a fluorescent substance, and so forth. Examples of the fluorescent substance include green fluorescent protein (GFP) derived from fluorescent jellyfish such as Aequorea victorea; variants thereof (GFP variants) such as Enhanced-humanized GFP (EGFP), red-shift GFP (rsGFP), yellow fluorescent protein (YFP), green fluorescent protein (GFP), cyan fluorescent protein (CFP), and blue fluorescent protein (BFP); and GFP derived from Renilla reniformis (Atsushi Miyasawa (ed.), (2000) Jikken Igaku (Experimental Medicine), an extra number, Posutogenome Jidai no Jikken Koza (Experiments in Postgenomic Era) 3-GFP and Bioimaging, Yodosha). The detection can be also performed using an antibody (including a monoclonal antibody and a fragment thereof) capable of specifically recognizing the fused tag. The two-hybrid method using yeast can be also utilized.

Examples of the object proteins (for example, human IP-10 and ovine IP-10) may include a protein having at least one amino acid residue being different from that of a native protein in the terms of identity and position, and a protein having a specific activity during implantation, for example, an activity during implantation for pregnancy, more specifically, a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus. Examples of the object protein of the present invention include deletion analogues lacking one or more (e.g., 1 to 80, preferably 1 to 60, more preferably 1 to 40, further preferably 1 to 20, particularly 1 to 10) amino acid residues peculiar to the human IP-10 or ovine IP-10, substitution analogues having one or more (e.g., 1 to 80, preferably 1 to 60, more preferably 1 to 40, further preferably 1 to 20, particularly 1 to 10) peculiar amino acid residues substituted by other residues, and addition analogues having one or more (e.g., 1 to 80, preferably 1 to 60, more preferably 1 to 40, further preferably 1 to 20, particularly 1 to 10) amino acid residues added thereto. The present invention encompasses all of the mutants such as those described above as long as they maintain a domain structure or receptor binding activity which is characteristics of native IP-10. Furthermore, the present invention may encompass those having a primary structure conformation substantially equivalent to that of native IP-10 or a part thereof, and also those having a biological activity substantially equivalent to that of native IP-10. The object proteins of the present invention have a homology of at least 60%, preferably higher than 70%, more preferably higher than 80% or 90% with, for example, an amino acid sequences selected from the group consisting of (1) amino acid residues from 2- to 102-positions, (2) from 1- to 102- positions, and (3) from 20- to 80-positions of amino acid shown by SEQ NO: 2. A part of the object protein of the present invention means a partial peptide of these proteins (i.e. partial peptide of the protein) showing substantially equivalent activities with ovine IP-10 according to the present invention. For example, the partial peptide according to the present invention has at least 5 amino acids residue, preferably at least 20 amino acid residues, more preferably at least 70 amino acid residues, further preferably at least 80 amino acid residues, further preferably at least 90 amino acid residues, particularly at least 95 amino acid residues of ovine IP-10 structure amino acid according to the present invention. Preferably, these amino acid residues are continuous, or are the same as the corresponding portion of the amino acid shown by SEQ ID NO: 2 in the terms of homology.

In the present specification, the terms "substantially equivalent" and "substantially the same" mean that the activities of a protein, for example its activity during implantation period, physiological activity, and biological activity, are substantially the same as those of another protein. The terms include, within the meaning thereof, the case of a protein having a substantially the same nature as compared with another. The activity of substantially the same nature is, for example, a biological activity selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus. The activities of substantially the same nature indicate that the activities are each qualitatively the same nature, for example physiologically, pharmacologically, or biologically, as compared with the counterpart. For example, it is preferred that the activities selected from the group consisting of (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus, be equivalent (for example, about 0.001 to about 1,000 times, preferably about 0.01 to about 100 times, more preferably about 0.1 to about 20 times, further preferably about 0.5 to about 2 times) to the counterpart. However, the levels of these activities, the molecular weight of the protein, and other quantitative factors may differ. Substitution, deletion, or addition of amino acid may not often modify the physiological or chemical characteristics, but may advantageously modify them. In such cases, a polypeptide applied the substitution, deletion, or addition may be substantially the same as those being not applied such substitution, deletion, or addition from the terms of predetermined objects. Examples of the substantially the same substituent in the amino acid sequence can be selected from other amino acids in the group [for example, nonpolar (hydrophobic) amino acids such as alanine, phenylalanine, leucine, isoleucine, valine, proline, triptophan, and methionine; polar (neutral) amino acids such as glycine, serine, threonine, cysteine, tyrosine, asparagine, and glutamine; positive charged amino acids (basic amino acid) such as arginine, lysine, and histidine; and negative charged amino acids (acidic amino acid) such as aspartic acid and glutamic acid], to which the substituent belongs.

For synthesizing the protein and partial peptides thereof according to the present invention, methods known in the field of peptide synthesis, for example chemical synthetic method such as a liquid phase method and a solid phase method can be used. In such methods, peptides are synthesized by binding adequately protected amino acids one by one on a resin used in protein or peptide synthesis to form a desired amino acid sequence by any of various per se known condensation methods. Regarding the condensation reaction, carbodiimides such as dicyclohexylcarbodiimide are preferable reagents. When the resulting product has a protective group, removal of the protective group is appropriately performed to yield the target product.

Regarding the protein and its partial peptides of the present invention, when they are obtained in the free form, they may be converted to salts by a per se known process or a modification thereof. When they are obtained in a salt form, they may be converted to the free form or to other salts by a per se known method or a modification thereof.

The salts of the protein and partial peptides thereof according to the present invention preferably include, but are not limited to, physiologically or pharmaceutically acceptable ones. Such salts may include salts with inorganic acids such as hydrochloric acid, hydrobromic acid, sulfuric acid, nitric acid, and phosphoric acid; and salts with organic acid such as acetic acid, formic acid, maleic acid, fumaric acid, succinic acid, citric acid, tartaric acid, malic acid, benzoic acid, methanesulfonic acid, p-toluenesulfonic acid, and benzene sulfonic acid. The salts may also include ammonium salts and salts with organic bases such as ethylamine, dimethylamine, trimethylamine, and hydoxyethylamine.

These IP-10, its mutants, modifications, and derivatives can be separated and purified by the above-mentioned methods. In the present invention, the terms "fragment", "derivative", and "analogue" mean the polypeptide that essentially retains the same biological functions or activities of the polypeptide shown by SEQ ID: 2, the polypeptide that is encoded by an hnRNA or mRNA that are transcribed from the sequence shown by SEQ ID NO: 1 and received or not received specific splicing, or the polypeptide encoded by genomic DNA, when the terms "fragment", "derivative", and "analogue" are used in connection with these peptides.

Since the present invention provides unknown information relating to functions of IP-10 protein derived mammal, the utilization of such information is included in the present invention. Such utilization includes the development of screening methods or reagents for identifying the functions of the IP-10, for example, activating conceptus migration, promoting conceptus implantation on the uterine wall, treating sterility, promoting pregnancy, activating conceptus migration to uterus during early pregnancy, and controlling interaction between conceptus and maternal system, by isolating and detecting genomic DNA and cDNA derived from mammal, especially human, encoding the IP-10, and related proteins thereof.

For example, by using an IP-10 DNA sequence such as ovine IP-10 DNA sequence as a probe, a function analysis of IP-10 and its related proteins, for example activating conceptus migration, promoting conceptus implantation on the uterine wall, treating sterility, promoting pregnancy, activating conceptus migration to uterus during early pregnancy, and controlling interaction between conceptus and maternal system, can be performed. The probe is optionally labeled with a label known in the field of the art. The label includes radioisotope (RI) and non-RI, and the non-RI is preferable. Examples of the non-RI label include fluorescence label, biotin label, and chemical luminescence label. The fluorescence label is most preferable. Fluorescence that can bind with base portion of an oligonucleotide is suitably selected. Examples of the fluorescence label include cyanine dyes (e.g. Cy3 and Cy5 belonging to Cy Dye™ series), rhodamine 6G reagent, N-acetyl-N²-acetylaminofluorene (AAF), snf AAIF (iodide derivative of AAF). For example, the isolation of gene can be performed by PCR and RT-PCR (i.e. PCR using reverse transcriptase). IP-10 cDNA and its related DNAs can be used for isolation and detection of IP-10 related gene by PCR, competitive PCR, RT-PCR, Real-Time PCR (Journal of Molecular Endocrinology, (2000) 25, 169-193), and other methods using a DNA primer (including a primer set). The primer is designed according to a specific sequence region selected from an amino acid sequence deduced from cloned IP-10 cDNA sequence and chemically synthesized. The primer size (the number of base) is 15 to 40 bases, preferably 15 to 30 bases, for specific annealing with a template DNA. Long accurate (LA) PCR requires at least 30 bases. A pair of (two) primers consisting of sense strand (5' side) and antisense strand (3' side) is avoided to have a sequence complementary to each other so as not to anneal with each other, as well as avoided to have a self-complementary sequence so as not to form a hairpin structure within the primers. Furthermore, the primers should contain GC at a content of about 50% in order to secure a stable binding with the template DNA, and should be avoided to have GC-rich or AT-rich regions. Since the annealing temperature depends on the melting temperature (Tm), the primers having approximate Tm values ranging from 55°C to 65°C should be selected in order to obtain highly specific PCR products. Furthermore, it is required to adjust the primer final concentration in PCR to about 0.1 to 1µM. Commercially available software for designing primers, for example, Oligo™ (National Bioscience Inc., U.S.A.), GENETYX (Software Development Co., Ltd., Japan) can be used. Universal primers, i.e. known primers such as dT primer can be used. For example, IP-10 mRNA expression in human tissues can be studied by Northern blot analysis for poly (A)⁺ RNA derived from various tissues. According to the present invention, IP-10 mRNA expression in human tissues, IP-10 gene itself, and so forth can be detected or measured by using the cDNA as a probe by, for example, Northern blotting, Southern blotting, in situ hybridization, dot blotting, and RNase protection assay. Consequently, cellular protein metabolism in cells, activation of hormone precursor, and physiological functions involved in various processes in normal cells including the above-mentioned activities relating to pregnancy such as a role in interaction between conceptus and mother, sterility, and abortion can be performed. The cDNA can be also used for gene diagnosis of diseases relating to IP-10. The abnormality in nucleic acids encoding the IP-10 and its related proteins, for example, damage, mutation, and a decrease or increase in expression can be diagnosed by such gene diagnosis.

In situ hybridization includes direct and indirect non-RI in situ hybridization. In the direct method, a detectable molecule (reporter) is directly bound to the nucleic acid probe, and in the indirect method, for example, an antibody against the reporter molecule is used to amplify the signal. A functional group (for example, a primary aliphatic amino group, an SH-group) is introduced to oligonucleotide in the nucleic acid probe, and may bind to a hapten, a fluorescent dye, or an enzyme. Examples of the label of the nucleic acid include digoxigenin (DIG), biotin, and fluoresceine, and can be appropriately selected from labels described blow in a description of antibodies. Furthermore, multilabelling can be used, and a labeled antibody can be also used. Regarding the labeling of the nucleic acid probe, known methods in the field of the art can be suitably used, e.g. random priming method, nick translation method, DNA amplification by PCR, labeling/tailing method, and in vivo transcription method. The observation of the treated samples is performed by a known method in the field of the art by using a dark-field microscope, a phase different microscope, a reflection contrast microscope, a fluorescence microscope, a digital imaging microscope, or an electron microscope. Flow cytometoryl can be also used.

A gene diagnosis (detection method) of physiological phenomena during early pregnancy involved in IP-10 is provided according to the present invention. The gene diagnosis may include the steps of (a) a step of preparing a nucleic acid sample, (b) a step of amplifying the nucleic acid sample prepared in the step (a) by PCR, nucleic acid amplification using RNA polymerase, strand displacement amplification, or the like to prepare nucleic acid segments, which include mutations existing in the IP-10 gene, and (c) a step of detecting the presence of mutation in the nucleic acid segments in the step (b). The methods for amplifying nucleic acid include gene amplification process generally used, e.g. PCR (including RT-PCR), nucleic acid sequence based amplification method (NASBA), transcription-mediated amplification method (TMA), strand displacement amplification method (SDA). The target of the amplification can be selected according to the objective. For example, a region containing a mutation, which causes a functional decrease, of IP-10 gene nucleotide sequence of the present invention is one of the objects for the amplification. Examples of such a region is, but not limited to, a region containing bases at a predetermined position in the nucleotide sequence shown by SEQ ID NO: 1. In the step (c) in above, any method for detecting mutation known by those in the field of the art can be used. For example, the detection of mutations can be performed by investigating the DNA fragment length by an allele-specific PCR method (ASPCR), but not limited to this. The method for investigating the DNA fragment length can be performed by using fluorescence DNA sequencer, or the like. But it is not limited to this. Examples of the method for detecting mutation used in this step include a process for detecting restriction fragment length polymorphism (RFLP). The detection of mutation can be performed by a known method, e.g. hybridization using an appropriate DNA containing a mutation as a probe and a single-strand conformation polymorphism (SSCP). The gene diagnosis of IP-10 of the present invention can be performed by the gene diagnosis according to the present invention to diagnose expression or polymorphism of IP-10, which may be a resistance or sensitivity factor relating to interaction between conceptus and mother, e.g. sterility and implantation disorder. Furthermore, gene therapy method for decreasing a risk of the matter or the related dysfunction based on the diagnostic results can be provided.

In the present specification, IP-10, its related proteins, fragments, and nucleic acids (including mRNA and oligonucleotide) including DNA can be used for genomics and proteomics technologies by alone or organically, and further in combination with gene manipulation technology or antibody manipulation technology (for example, an antisense method, an antibody including a monoclonal antibody, and a transgenic animal) which are widely known in the field of the art. IP-10 variants can be used for a function analysis utilizing the dominant-negative effect, and can be also applied RNA interference (RNAi) technology using a double-stranded RNA (dsRNA). Thus, gene polymorphisms, mainly single nucleotide polymorphisms (SNP), analysis, an nucleic acid array (including a DNA microarray; Mark Shena (Ed.), "Microarray Biochip Technology", Eaton Publishing, March 2000), gene expression analysis using a protein array, gene function analysis, relating gene analysis, protein interaction analysis, relating disorder analysis, and therapeutic drug analysis are possible.

When RNAi technology is used, short interfering RNA (siRNA) may be designed and introduced. When the siRNA is designed, a 19-base strand, which starts with AA about 50 to 100 bases downstream, preferably at least 75 bases downstream of the start codon of target gene, is typically selected. Preferably, 5'- and 3'-UTR and in the vicinity of start codon are avoided, in some case, UTR-region can be selected. The GC content of the selected region is about 30% to 70%, preferably about 45% to 55%. A strand of two bases (AA) and 19 bases (i.e. 21 bases), for example AA(N19) (N: arbitrary nucleotides), is screened for DNA database such as NCBI (e.g. BLAST search) to identify the specificity. A synthetic RNA having the selected strand may be added dTdT (or UU) at the 3' side. Synthetic RNAs of sense strand and the corresponding antisense strand are prepared to form dsRNA. The dsRNA is introduced into cells or the like. Examples of typical annealing buffer solution used in the dsRNA preparation include, but not limited to, 30 mM HEPES-KOH (pH 7.4) containing 100 mM KOAc and 2 mM MgOAc; and 10 mM Tris (pH 7.5 to 8.0) containing 50 mM NaCl and 1 mM EDTA. Typical conditions for the annealing are heating at 95°C for 2 or 3 minutes and then gradually cooling up to 37°C to 25°C in 45 minutes to 60 minutes, but it is not limited to this. The prepared dsRNA can be collected by extraction with phenol/chloroform and precipitation with ethanol. The introduction of siRNA into cells of mammal or the like can be performed by a method known in the field of the art or substantially the same method thereof, such as a calcium phosphate method (e.g. F. L. Graham et al., (1973) Virology, 52: 456), DEAE-dextran method (e.g. D. Warden et al., (1968) J. Gen. Virol., 3: 371), liposome method such as cationic lipid complex formation, electroporation (e.g. E. Neumann et al., (1982) EMBO J, 1: 841), microinjection, and biolytic particle delivery method. The nucleic acid introduction is technically improved to be efficiently performed by transfection. The introduction can be performed by commercially available kit according to the protocol disclosed by manufacturers, e.g. Invitrogen Corporation and QIAGEN Inc., or distributors. Regarding siRNA technology, Elbashir et al., (2001) Nature, 411: 494-498; Elbashir et al., (2001) Genes Dev., 15: 188-200; and Kazunari Tabira (ed.), (2001) Jikken Igaku (Experimental Medicine), an extra number, Protocol Series

### (Functional Inhibition Experiments in Gene) Yodosha can be referred to.

A DNA microarray according to a nucleic acid array technology may be used. Regarding the preparation of microarrays, two methods are known, i.e. a method of directly synthesizing oligonucleotides on a surface of a solid carrier (on-chip method) and a method of immobilizing oligonucleotides or polynucleotides that are synthesized in advance on a surface of a solid carrier. Both methods can be applied to manufacturing microarrays used in the present invention. In the on-chip method, a selective synthesis on a predetermined shallow matrix region is possible by a combination with the use of a protective group which is selectively removed by light irradiation, a photolithography technology used in semiconductor preparation, and a solid-phase synthesis technology (masking technology: e.g. Fodor, S.P.A., (1991) Science 251: 767). When oligonucleotides or polynucleotides prepared in advance are immobilized on a surface of a solid carrier, a functional group is introduced to the oligonucleotides or polynucleotides and the resulting oligonucleotides or polynucleotides are attached on a treated surface of a solid-carrier to covalently bind (e.g. Lamture, J.B. et al., (1994) Nucl. Acids Res. 22: 2121-2125; Guo, Z. et al., (1994) Nucl. Acids Res. 22: 5456-5465). In general, oligonucleotides or polynucleotides are covalently bound to a treated surface of a solid carrier via a spacer or a cross-linker. A known method includes the steps of arraying micro pieces of polyacrylamide gel on a glass surface, then covalently binding synthetic oligonucleotides on the surface (Yershov, G. et al., (1996) Proc. Natl. Acad. Sci. U.S.A. 94: 4913). Another method includes the steps of arraying microelectrodes on a silicon microarray, forming a penetration layer composed of agarose and streptavidin on the electrodes to provide a reaction region, and immobilizing biotinized oligonucleotides on the reaction region by positively charging the region. In this method, the fast and strict hybridization can be performed by controlling the charge of the region (Sosnowski, R.G. et al., (1997) Proc. Natl. Acad. Sci. U.S.A. 94: 1119-1123). In analysis using nucleic acid array, cDNA library is utilized. DNAs, which are prepared by PCR technology, is mounted on a substrate at a high-density by a spotting device. Specimens are analyzed by hybridization with such DNAs. In arraying, DNA are attached and arrayed at the predetermined position on a substrate of glass, silicon, or plastic using a needle or pin, or using inkjet printing technology. Signals that show the results of hybridization on the nucleic acid array are observed to obtain data. The signals may be labels such as fluorescence (e.g. Cy3, Cy5, BODIPY, FITC, Alexa Fluor dyes (trade name), and Texas red (trade name)). The detection can be conducted using a laser scanner. The resulting data can be processed with a computer system equipped with a program of appropriate algorithm. In DNA analysis using a typical microarray, cDNA is synthesized using mRNA isolated from cells as a template, and then amplified by PCR. In this process, labeled dNTP is incorporated to produce labeled cDNA. The labeled cDNA is contacted with a microarray. Complementary DNA that hybridized to a capture probe (oligonucleotide or polynucleotide) on the microarray is detected. The hybridization is performed by dispensing a labeled cDNA solution to a 96-well or 384-well plastic plate. The amount of the solution may range about 1 to 100 nL. Preferably, the hybridization is performed at the ranges from room temperature to 70°C for 6 to 20 hours. After the hybridization, the plate is washed with a solution mixture of a surfactant and a buffer to remove unreacted labeled cDNA. Sodium dodecyl sulfate (SDS) is a preferable surfactant. Examples of the buffer include a citric acid buffer, a phosphoric acid buffer, a boric acid buffer, a tris buffer, and a Good's buffer. The citric acid buffer is preferable.

In protein array technology, a tagged recombinant expression product protein may be used. Two-dimentional electrophoresis (2-DE), mass spectrometry (MS) for samples including enzyme-digested fragments, staining technique, isotope-labeling and analysis, and image processing technique may be used. The MS includes electrospray ionization (ESI) and matrix-assisted laser desorption/ionization (MALDI) and MALDI-TOF MS, ESI-triple quadruple MS, and ESI-iontrap MS may be used. Therefore, software, database, and the like that are obtained or used as above concerning with function analysis of interaction between conceptus and mother which correlates with IP-10 and antibody thereof can be included in the present invention.

According to the present invention, antisense oligonucleotide (nucleic acid) that can inhibit IP-10 gene expression can be designed and synthesized based on a nucleotide sequence information about DNA encoding IP-10 which is cloned and determined. The antisense oligonucleotide can be used in surveying or regulating the investigated IP-10 function. Such an oligonucleotide (nucleic acid) can hybridize with mRNA of IP-10 gene. The mRNA can inhibit the function of mRNA or can regulate or control IP-10 gene expression via interaction with IP-10 related mRNA. In some cases, the mRNA can regulate or control IP-10 gene expression by controlling an expression-control region. Oligonucleotides complementary to a sequence selected from IP-10 related gene and oligonucleotides capable of specifically hybridizing to IP-10 related gene are useful for the regulation and control of IP-10 gene in vivo and in vitro, and useful therapy or diagnosis of diseases related thereof.

As in described above, the target IP-10 is obtained by introducing IP-10 gene and recombinant DNA molecule and expressing IP-10 according to the results of the research by the inventors. A method using the results for researching and surveying function relating to interaction between conceptus and mother is provided. In connection with the found functions, recombinant or transfectant substantially expressing IP-10 gene, manufacturing method thereof, and their use are provided according to the present invention.

An object of the present invention is to provide a good method or a reagent kit thereof for the detection or fractional measurement of IP-10, its gene, or producing cells thereof in a specimen for the research or survey of a phenomenon and function relating to the interaction between conceptus and mother using IP-10 gene, probes induced thereof, or an inhibitor of IP-10 if necessary. It is understood that each reagent of the reagent kit that can detect or fractionally measure IP-10, its gene, or producing cells thereof are included in embodiment according to the present invention. The object of the present invention is to provide a method, reagent, or diagnostic agent for monitoring factors or physiological phenomena relating to metabolism of cellular protein, activating hormone precursor, activating conceptus migration, promoting conceptus implantation on the uterine wall, treating sterility, promoting pregnancy, introducing conceptus to the uterus during implantation period, controlling interaction between conceptus and maternal system, and processes thereof by the detection or fractional measurement of IP-10, its gene, and producing cells thereof by using the above-mentioned method.

Therefore, it is understood that the various use of the reagents in medical and physiological fields, and the uses of the reagents for research, analysis, and measurement of responses, symptoms, and diseases caused by controlling interaction between conceptus and IP-10, activating conceptus migration, promoting conceptus implantation on the uterine wall, treating sterility, promoting pregnancy, introducing conceptus to the uterus during implantation period, and controlling interaction between conceptus and maternal system, and the use for diagnosis, prevention, and therapy, these utilization are all included in embodiments according to the present invention.

IP-10 and its related substances may be factors having important functions between conceptus and mother during early pregnancy. For a pregnancy (implantation) to be established, mother must secrete IP-10 in proper time and in proper quantity as a response to a signal from conceptus. The maternal system establishes physiological and immunological responding paths, and pregnancy (implantation) progresses. When the response to the signal is insufficient, pregnancy is not established. Therefore, the protein is useful for communication between conceptus and mother. Namely, Drugs containing a member selected from IP-10, variants, modifications, or derivatives may cure sterility caused by the insufficient IP-10 activity. Polypeptides according to the present invention are useful as a pharmaceutical drug for the treatment and/or prevention of these diseases.

For example, when the biological activity in cells is insufficient or abnormal due to IP-10 decrease or deficiency in the living body, the phenomena are improved by (A) administering a protein of the present invention, (B) administering nucleic acid such as DNA of the present invention to express the proteins of the present invention in vivo, or (C) transplanting cells transduced nucleic acid such as DNA of the present invention so as to express the protein in vivo. Namely, the pregnancy ratio may be improved by inducing the IP-10 production or administering IP-10.

According to the biologically functional analysis of IP-10 of the present invention, a compound (agonist or promoting agent) promoting the function of IP-10 or its salts can be used as useful medicine (including veterinary medicine) for curing and/or preventing a variety of diseases relating to biological activities described above, e.g. deficiency in pregnancy caused by a dysfunction of IP-10. Contrary to this, a compound (antagonist or suppressing agent) suppressing the function of IP-10 or its salts can be used as useful medicine to cure and/or prevent an excessive IP-10 function and control of pregnancy.

By focusing the function of IP-10 according to the present invention, IP-10 and its related polypeptides are useful for screening agonists, antagonists, or salts thereof to physiological phenomena relating to interaction between conceptus and mother. The present invention provides a screening method of such agonists, antagonists, or salts thereof, which promote or inhibit the physiological or biological activities.

In the screening method, for example, (i) contacting conceptus with protein, its partial peptide, or a salt thereof (transformant expressing the protein may be included; the same shall apply thereinafter) according to the present invention, (ii) contacting conceptus and a test sample with the protein, its partial peptide, or a salt thereof, and results in (i) and (ii) are compared. Specifically, a biological activity (e.g. activity relating to interaction between conceptus and uterine membrane) is measured in the screening method, and compared. A substrate instead of conceptus can be used. The substrate can be used as it is, preferably, the substrate labeled with a fluorescence such as fluoresceine, an enzyme, or an radioisotope is used.

Examples of the test sample include proteins, peptides, non-peptidic compounds, synthetic compounds, fermentation products, plant extracts, tissue extracts of animal, cell extracts. Examples of the test sample include anti-IP-10 antibodies, anti-CXCR3 antibodies, anti-IFN-τ antibodies, inhibitors of binding between CXCR3 and IP-10, compounds for inhibiting conceptus implantation, and compounds for promoting conceptus implantation, and especially synthetic compounds thereof. These compounds may be novel or known. The screening can be performed according to an ordinary method for measuring a binding activity. Methods known in the field of the art can be used. Namely, methods are performed according to the protocols using various labels, buffer solutions, and other appropriate reagents. The peptides that are used in the measurements may be treated with an activating agent, or may be activated in advance from precursors or potential types. The measurement is usually performed in a buffer solution that does not affect the reaction. For example, tris-HCl buffers and phosphate buffers, at pH about 4 to 10 (preferably pH about 6 to 8) can be used. Actual screening systems may be constructed with relation to IP-10, polypeptides or peptides which have substantially the same activities as those of IP-10 of the present invention, with general technique known by those in the arts as well as ordinary conditions and manipulations of individual systems. Details of these general technological means can be referred to review articles and monographs (for example, Methods in Enzymology, Academic Press, U.S.A.).

Compounds or their salts which are obtained by the screening method or using the screening kit according to the present invention are selected from the above-mentioned test compounds, e.g. peptides, proteins, non-peptidic compounds, synthetic compounds, fermentation products, cell-extract solutions, plant-extract solutions, and animal-tissue-extract solutions. These compounds promote or inhibit functions of proteins of the present invention. Examples of pharmacologically acceptable salts of the compounds include salts with inorganic base, salts with organic base, salts with inorganic acid, salts with organic acid, and salts with basic or acidic amino acid. Preferable examples of the salts with inorganic base include salts with alkali metal such as sodium and potassium, salts with alkaline-earth metal such as calcium and magnesium, salts with aluminium, and salts with ammonium. Preferable examples of the salts with organic base include salts with trimethylamine, triethylamine, pyridine, picoline, 2,6-lutidine, ethanolamine, diethanolamine, triethanolamine, cyclohexylamine, dicyclohexylamine, and N,N'-dibenzylethylenediamine. Preferable examples of the salts with inorganic acid include salts with hydrochloric acid, hydrobronic acid, sulfuric acid, and phosphoric acid. Preferable example of the salts with organic acid include salts with formic acid, acetic acid, propionic acid, fumaric acid, oxalic acid, tartaric acid, maleic acid, citric acid, succinic acid, malic acid, methanesulfonic acid, benzenesulfonic acid, and benzoic acid. Preferable examples of the salts with basic amino acid include salts with arginine, lysine, and ornithine. Preferable examples of the salts with acidic amino acid include salts with aspartic acid and glutamic acid.

In the present specification, the term "antibody" may be used in a broad sense. Thus, it includes individual monoclonal antibodies to the desired IP-10 polypeptides and related peptide fragments, and antibody compositions having specificity for various epitopes. It also includes univalent antibodies and polyvalent antibodies as well as polyclonal antibodies and monoclonal antibodies. Furthermore, it includes the native (intact) molecules, and fragments and derivatives thereof, i.e. such fragments as F(ab')₂, Fab', and Fab and further, chimera antibodies or hybrid antibodies having at least two antigen or epitope binding sites, or bispecific recombinant antibodies such as quadromes and triomes, interspecific hybrid antibodies, anti-idiotype antibodies and, further, chemically modified or processed ones which can be considered to be derivatives of these, antibodies obtained by applying a per se known cell fusion or hybridoma technology or antibody engineering technology or utilizing a synthetic or semisynthetic technology, antibodies prepared by applying conventional technology known as antibody-preparing method by using a recombinant DNA technology, and antibodies having neutralizing characteristic or binding characteristic against the target antigen substance or target epitope as described herein. A particularly preferred antibody according to the present invention can specifically recognize the native IP-10 polypeptide.

The monoclonal antibody produced against the antigenic substance can be produced by any of the methods capable of causing the production of antibody molecules in a series of cell lines under cultivation. The modifier "monoclonal" indicates the characteristic of an antibody that belongs to a substantially homogeneous antibody population. It is not to be construed that the antibody should be produced by a certain specific method. Individual monoclonal antibodies each includes a population of the same antibodies except that a slight amount of a possible mutant being spontaneously formed may be present therein. Monoclonal antibodies each have high specificity and are directed to one single antigenic site. As compared with ordinary (polyclonal) antibody preparations typically containing various antibody species directed to different antigenic determinants (epitopes), each monoclonal antibody is directed to one single antigenic determinant on the antigen. In addition to their specificity, monoclonal antibodies are synthesized by hybridoma culture and are superior in that they are not or only a little contaminated with other immunoglobulins. The monoclonal antibodies include hybrid antibodies and recombinant antibodies. So long as they show the desired biological activities, a constant region domain may be substituted for variable region domain thereof (for example, humanized antibody), or heavy chain may be substituted for a light chain thereof, a chain derived from a certain species may be replaced with a chain derived from another species, or they may be fused with a heterogeneous protein, irrespective of their origin or immunoglobulin class or subclass (e.g. U.S. Patent No. 4,816,567; Monoclonal Antibody Production Techniques and Applications, pp.79-97, Marcel Dekker, Inc., New York, 1987) .

As exemplary method for producing the monoclonal antibody, there may be mentioned the hybridoma method (G. Kohler and C. Milstein, (1975) Nature, 256: 495-497); human B cell hybridoma method (Kozbor et al., (1984) Immunology Today, 4: 72-79; Kozbor, (1984) J. Immunol., 133: 3001); Brodeur et al., (1987) Monoclonal Antibody Production Techniques and Applications, pp.51-63, Marcel Dekker, Inc., New York; trioma method; EBV-hybridoma method (Cole et al., (1985) Monoclonal Antibodies and Cancer Therapy, Alan R. Liss, Inc., pp.77-96)(methods for producing human monoclonal antibodies); U.S. Patent No. 4,946,778 (technology for producing single-chain antibodies) and, further, the following references may be cited for antibodies: S. Biocca et al., (1990) EMBO J, 9: 101-108; R.E. Bird et al., (1988) Science, 242: 423-426; M.A. Boss et al., (1984) Nucl. Acids Res., 12: 3791-3806; J. Bukovsky et al., (1987) Hybridoma, 6: 219-228; M. DAINO et al., (1987) Anal. Biochem., 166: 223-229; J.S. Huston et al., (1988) Proc. Natl. Acad. Sci. U.S.A., 85: 5879-5883; P.T. Jones et al., (1986) Nature, 321: 522-525; J.J. Langone et al. (ed.), "Methods in Enzymology", Vol. 121 (Immunochemical Techniques, Part I: Hybridoma Technology and Monoclonal Antibodies), Academic Press, New York (1986); S. Morrison et al., (1984) Proc. Natl. Acad. Sci. U.S.A., 81: 6851-6855; V.T. Oi et al., (1986) BioTechniques, 4C: 214-221; L. Riechmann et al., (1988) Nature, 332: 323-327; A. Tramontano et al., (1986) Proc. Natl. Acad. Sci. U.S.A., 83: 6736-6740; C. Wood et al., (1985) Nature, 314: 446-449; Nature, 314: 452-454; and references cited therein (the description therein are incorporated herein by reference).

The monoclonal antibody according to the present invention particularly includes "chimera" antibodies (immunoglobulins), in which part of the heavy chain and/or light chain has a sequence identical or homologous to the corresponding sequence of an antibody derived from a specific species or belonging to a specific antibody class or subclass while the remaining portions are identical or homologous to the corresponding sequences of an antibody derived from another species or belonging to another antibody class or subclass, so long as they show the desired biological activities (U.S. Patent No. 4,816, 567; Morrison et al., (1984) Proc. Natl. Acad. Sci. U.S.A., 81, pp.6851-6855).

Antibody may be fragments, e.g. Fab, Fab', and F(ab')₂, obtained by treatment of the antibodies with an enzyme such as trypsin, papain, or pepsin, if necessary followed by reduction.

The antibodies can be used in any known assay method, e.g. competitive binding assay, direct and indirect sandwich assay, and immunoprecipitation [Zola, Monoclonal Antibodies: A Manual of Techniques, pp.147-158 (CRC Press, Inc., 1987)].

For conjugating the antibodies to a detectable atomic group, any method known in the field of the art can be used. For example, David et al., (1974) Biochemistry, 13: 1014-1021; Pain et al, (1981) J. Immunol. Meth., 40: 219-231; and "Methods in Enzymology", Vol. 184, pp.138-163 (1990) are mentioned. As the antibodies to be labeled, IgG fraction and the specific binding portion Fab' obtained by reduction-following pepsin digest can be used. In these cases, examples of the label include enzymes (e.g. peroxidase, alkaline phosphatase, or β-D-galactosidase), chemical substances, fluorescent substances, and radioisotopes.

The detection and assay according to the present invention can be performed by immunostaining, e.g. tissue or cell staining and immune-electron microscopy; and immunoassay, e.g. competitive immunoassay and noncompetitive immunoassay. Radioimmunoassay (RIA), fluorescent immunoassay (FIA), luminescent immunoassay (LIA), enzyme immunoassay (EIA), enzyme-linked immunosorbent assay (ELISA), and the like can be used. Assaying may be performed with or without B-F separation. RIA, EIA, FIA, and LIA are preferable, and sandwich assay is preferably used. In the sandwich assay, simultaneous sandwich assay, forward sandwich assay, and reversed sandwich assay may be included.

Examples of the label include enzymes, enzyme substrates, enzyme inhibitors, prosthetic groups, coenzymes, enzyme precursors, apoenzymes, fluorescent substances, chromophores, chemiluminescent compounds, luminescent substances, chromogens, magnetic substances, metal particles such as colloidal gold, and radioisotopes. Examples of the enzyme include oxyreductase such as dehydrogenases, reductases, oxidases; transferases catalyzing the transfer of amino, carboxyl, methyl, acyl, or phosphoryl group; hydrolases hydrolyzing the ester, glycoside, ether, or peptide bond; lyases; isomerases; and ligases. A plurality of enzymes may be utilized in combination for detection purposes. For example, enzymatic cycling can by utilized.

A biotin label and enzyme-labeled avidin (streptavidin) may be substituted for the enzyme label. A plurality of different labels can be used. In such case, a plurality of measurements can be performed continuously or discontinuously, and simultaneously or separately.

The labeling can be performed utilizing the reaction between a thiol group and a maleimide group, the reaction between a pyridyl disulfide group and a thiol group, or the like that is known or obvious to those skilled in the art, or modifications thereof.

Regarding the assaying IP-10 or the like according to the present invention, tissue samples are advantageously assayed by protein assaying systems such as immunostaining and immune-electron microscopy and expressed gene assaying systems such as in situ hybridization; tissue extracts are assayed by protein assaying systems such as EIA, RIA, FIA, LIA, and Western blotting and expressed gene assaying systems such as Northern blotting, dot blot, RNase protection assay, RT-PCR, Real-Time PCR, and competitive PCR, and blood, body fluids are assayed by protein assaying systems such as EIA, RIA, FIA, LIA, and Western blotting. For example, in EIA systems using a competitive method, the anti-IP-10 antibody is used as an immobilized antibody and a labeled antigen and an unlabeled antigen (IP-10 or a fragment peptide thereof may be mentioned as the antigen) are used. In the case of a non-competitive method such as sandwich method, an immobilized anti-IP-10 antibody and labeled anti-IP-10 antibody may be used. A labeled anti-IP-10 may be used, or a labeled antibody to the anti-IP-10 antibody may be used. Regarding the sensitivity-enhancing method, there may be mentioned the utilization of, in the combination with a non-enzyme-labeled primary antibody, a macromolecular polymer and an enzyme and the primary antibody [application of Envision reagent; Enhanced polymer one-step staining (EPOS)]; and, in the combination with a non-enzyme-labeled secondary antibody, the combination of an enzyme and an anti-enzyme antibody as in the peroxidase-antiperoxidase technique (PAP), the combination of a biotin-labeled secondary antibody and a biotin-labeled enzyme-avidin complex as in the avidin-biotin complex method (ABC), the combination of a biotin-labeled secondary antibody and a biotin-labeled enzyme-streptavidin complex as in the streptavidin-biotinylated peroxidase complex method (SABC), the labeled streptavidin-biotin method (LSAB), the combination of a biotin-labeled tyramide and an enzyme-labeled streptavidin on the SABC as in the catalyzed signal amplification method (CSA), and the use of a macromolecular polymer labeled with a secondary antibody and an enzyme.

When these various analyses and quantitative assays including immunoassays are applied to the measuring methods according to the present invention, particular conditions and manipulations are not necessarily established. According to typical conditions and manipulations in each method as well as typical technical arrangements by those skilled in the art, assay systems may be established in relation with the target substances or substances having substantially equivalent activities thereof.

For the details of these general technological means, review articles and monographs may be referred to [e.g. Hiroshi Irie (ed.), "Radioimmunoassay", Kodansha, published in 1974; Hiroshi Irie (ed.), "Radioimmunoassay, supplement", Kodansha, published in 1979; Eiji Ishikawa et al. (ed.), "Koso Men'eki Sokuteiho (Enzyme immunoassay)" Igaku Shoin, published in 1978; Eiji Ishikawa et al. (ed.), "Koso Men'eki Sokuteiho (Enzyme immunoassay)" (second edition), Igaku Shoin, published in 1982; "Koso Men'eki Sokuteiho (Enzyme immunoassay)" (third edition), Igaku Shoin, published in 1987; H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Anibodies and Antigens, Nucleic Acids, Polysaccharides, and Drugs), Academic Press, New York (1991), and references cited therein (the description therein are incorporated herein by reference)].

When active ingredients of the present invention are used as medicine [for example, (a) IP-10 polypeptide, its partial peptides or its salts, and related peptides thereof, (b) nucleic acids, e.g. DNA encoding the IP-10 or IP-10 polypeptides, (c) antibodies according to the present invention, partial fragments thereof (including monoclonal antibodies), or derivatives thereof, (d) compounds or their salts promoting and/or activating biological activities such as interaction between conceptus and mother (e.g. implantation) by IP-10, and denaturation, excessive production, and decomposition of the related tissues and proteins; compounds or their salts controlling the production of IP-10; and compounds or their salts suppressing and/or preventing biological activities such as interaction between conceptus and mother (e.g. implantation) by IP-10, and denaturation, excessive production, and decomposition of the related tissues and proteins, (e) antisense oligonucleotides to the nucleic acids of DNA according to the present invention, (f) active substances found by utilizing the present invention], the ingredients can be administered alone or combination with pharmacologically acceptable adjuvants as medical components or medical preparations for, for example, promoting interaction between conceptus and mother. Preferably, they are administered in the forms of dosage preparations suitable for the use in oral administration, local administration, or parenteral administration. Administration forms (including inhalation, rectal administration) may be determined according on the objective.

The active ingredients of the present invention may be used in combination with substances known in the field of the art, such as hormone effectors, vitamins, cytokines, interferons, other physiologically active substances and/or immunomodulator, which are not limited so long as they have a useful function.

Examples of the parenteral administration form include local, transcutaneous, intravenous, intramuscular, hypodermal, intradermal, and intra-abdominal administration. Direct administration to affected area is possible, and it is suitable in some cases. Preferably, they are administered to mammals including human orally or parenterally (e.g. intracellular, internal, intravenous, intramuscular, hypodermal, intradermal, intra-abdominal, intra-thoracic administration, spinal cavity, instillation, intestinal infusion, through the rectum, ear drop, eye drop, nose drop, and apply to teeth, skin and mucous membrane). Examples of the preparation form include liquid, suspension, semi-solid, powder, shaped, and exudative forms, e.g. tablets, coated tablets, sugar-coated tablets, balls, troches, hard-capsules, soft-capsules, microcapsules, embedding, powders, dispersible powders, granules, fine-powders, injections, liquid, elixirs, emulsions, affusions, syrups, liquid, emulsions, suspension, liniment, lotion, aerosol, spray, inhalation, atomize, ointment, hard ointment, plaster, paste, cataplasm, cream, oleum, suppository (e.g. rectum suppository), tincture, liquid for skin, eye drops, nose drops, ear drops, swab, infusion, powder for injection, lyophilized preparations, and gel preparations.

Drug components are prepared according to general methods. Optionally, physiologically acceptable carriers, pharmaceutically acceptable carriers, adjuvants, excipients, forming agents, diluents, flavoring agents, essence, sweeteners, vehicles, antiseptics, stabilizers, binders, pH regulators, buffers, surfactants, bases, solvents, fillers, extenders, solubilizing supplements, solubilizers, isotonizing agents, emulisifiers, suspending agents, dispersing agents, thickeners, gelling agents, stiffening agents, absorbents, adhesives, flexing agents, plasticizers, collapsing agents, emitting agents, preservatives, antioxidants, shading agents, lubricants, mitigatives, antistatic agents, analgesic agents are used alone or combination. These additives are mixed with proteins or the like of the present invention so that unit doses which are required in typical drugs are prepared.

Examples of the parenteral drugs include injections such as abacterial solutions or suspensions of active ingredients and water or other pharmaceutically acceptable solvents. Preferable examples of carrier of the injections generally include water, saline, dextrose solutions, other sugar solutions, ethanol, and glycols, e.g. propyleneglycol and polyethyleneglycol. An injection solution, suspension, or emulsion is prepared by methods using a carrier such as distilled water, Ringer's solution, or saline, a dispersing agent or moisturizing agent, and a suspending agent, which are known in the field of the art.

Since polyethyleneglycol (PEG) has very low toxicity in mammals, the conjugation of PEG is particularly useful. The conjugation of PEG may effectively reduce the immunogenicity and antigenicity of heterologous compounds. The compound may be delivered in a microcapsulation device. Polymers such as PEG can be readily attached to α-amino groups of the amino-terminal amino acids, ε-amino groups of lysine side chains, carboxyl groups of aspartyl and glutamyl side chains, α-carboxyl groups of carboxy-terminal amino acids, or to activated derivatives of glycosyl chains attached serine or threonine residues.

Numerous activated forms of PEG for direct reaction with proteins are known. Examples of useful PEG reagents for reaction with amino acid groups of proteins include active esters of carboxylic acid or carbonate derivatives, particularly those in which the leaving groups are N-hydroxysuccinimide, p-nitrophenol, imidazole, or 1-hydroxy-2-nitrobenzene-4-sulfonate. Likewise, PEG reagents containing amino hydrazine or hydrazide groups are useful to the reaction with aldehydes generated by periodate oxidation of proteins.

When nucleic acid such as DNA of the present invention is used for the treatment and/or preventive medicine, the nucleic acid can be used alone or combination with the above-mentioned appropriate vector used in gene recombination technology, for example virus-derived vectors, e.g. retrovirus-derived vectors. The nucleic acid such as DNA of the present invention can be administered by well-known methods as it is or as a medicine component or medicine preparation with appropriate adjuvants or physiologically acceptable carriers so as to promote the intake into cells. Methods known as genetic therapy are applicable.

Active ingredients according to the present invention can be administered in various doses. Doses and frequencies are determined depending on sex, age, weight, general conditions of a subject, meal, administration time, administration method, excretion rate, combination with other medicine, degrees of symptoms of the subject at the treatment, and other factors.

Regarding the manufacturing of the medicine, the Japanese pharmacopoeia commission (ed.), "Manual of Japanese Pharmacopoeia 14^{th} edition", published on June 27, 2001, Hirokawa Shoten; and Ichibangase Naoshi, et al., (ed.) "Iyakuhin No Kaihatsu (Development of Pharmaceuticals) Vol. 12: Seizai Sozai [II] (Pharmaceutical Necessities [II])", published on October 28, 1990, Hirokawa Shoten are referred, and additives and preparation methods optionally selected from the descriptions therein can be applied.

The active ingredients according to the present invention typically have biological activities to promote or activate interaction between conceptus and mother. Preferably, such ingredients show advantageous functions. Examples of the active ingredients according to the present invention include (a) IP-10, its variant polypeptides, or partial peptides or salts thereof, (b) nucleic acids, e.g. DNA encoding the IP-10 or IP-10 variant polypeptides, (c) antibodies according to the present invention, partial fragments thereof (including monoclonal antibodies), or derivatives thereof, (d) compounds or salts thereof having at least one advantageous function such as activating conceptus migration, promoting conceptus implantation on the uterine wall, treating sterility, promoting pregnancy, activating migration of conceptus to uterus during an implantation period, and controlling interaction between conceptus and mother.

The active ingredients according to the present invention are expected as agents using biological activities, e.g. activating conceptus migration, promoting conceptus implantation on the uterine wall, treating sterility, promoting pregnancy, introducing conceptus to the uterus during an implantation period, and controlling interaction between conceptus and mother, and are expected to be useful for a preventive or treating method.

In the present invention, predetermined nucleic acids are isolated and sequenced by "gene recombination technology", recombinants are prepared, and predetermined peptides are obtained. A gene recombination technology known in the field of the art may be used in the present specification. For example, the methods described in: J. Sambrook, E. F. Fritsch & T. Maniatis, "Molecular Cloning: A Laboratory Manual", (2nd edition, 1989 & 3rd edition, 2001), Cold Spring Harbor Laboratory Press, Cold Spring Harbor, New York; D. M. Glover et al. ed., "DNA Cloning", 2nd ed., Vol. 1 to 4, (The Practical Approach Series), IRL Press, Oxford University Press (1995); "Methods in Enzymology" series, Academic Press, New York, for example, R. Wu ed., ibid., Vol. 68 (Recombinant DNA), (1980); R. Wu et al. ed., ibid., Vol. 100 (Recombinant DNA, Part B) & 101 (Recombinant DNA, Part C), (1983); R. Wu et al. ed., ibid., Vol. 153 (Recombinant DNA, Part D), 154 (Recombinant DNA, Part E) & 155 (Recombinant DNA, Part F), (1987); J. H. Miller ed., ibid., Vol. 204, (1991); R. Wu ed., ibid., Vol. 216 (Recombinant DNA, Part G), (1992); R. Wu ed., ibid., Vol. 217 (Recombinant DNA, Part H) & 218 (Recombinant DNA, Part I), (1993); J. L. Campbell ed., ibid., Vol. 262 (DNA Replication), (1995); P. M. Conn ed., ibid., Vol. 302 (Green Fluorescent Protein), (1999); S. Weissman ed., ibid., Vol. 303 (cDNA Preparation and Characterization), (1999); J. C. Glorioso et al. ed., ibid., Vol. 306 (Expression of Recombinant Genes in Eukaryotic Systems), (1999); M. lan Phillips ed., ibid., Vol. 313 (Antisense Technology, Part A: General Methods, Methods of Delivery and RNA Studies) & 314 (Antisense Technology, Part B: Applications), (1999); J. Thorner et al. ed., ibid., Vol. 326 (Applications of Chimeric Genes and Hybrid Proteins, Part A: Gene Expression and Protein Purification), 327 (Applications of Chimeric Genes and Hybrid Proteins, Part B: Cell Biology and Physiology) & 328 (Applications of Chimeric Genes and Hybrid Proteins, Part C: Protein-Protein Interactions and Genomics), (2000), and methods described in references cited therein and substantially the same methods or modifications thereof are mentioned (the description therein are incorporated herein by reference). The preparation of the reagents used in the diagnosis methods according to the present invention are performed by methods described in, for example, A. Gennaro ed., "Remington's Pharmaceutical Sciences", 18th Edition, Mack Publishing Co., Easton, PA, 1990, and by molecular biological technology described in, for example, Ausubel, F. M. et al., "Current Protocols in Molecular Biology", John Wiley & Sons, New York, N.Y, 1995, and methods or technology described in references cited therein and substantially the same methods or modifications thereof are mentioned (the description therein are incorporated herein by reference).

In the specification and figures, terms according to IUPAC-IUB Commission on Biochemical Nomenclature or the usual practice in the field of the art are used.

### Examples

Details of the present invention are described by the following examples but such examples are provided only for illustrative purposes, and for referential embodiments of the present invention. These examples have been described herein for the purpose of illustrating specific embodiments of the present invention but should in no way be construed as limiting and restricting the scope of the invention disclosed herein. It should be understood in the present invention that various embodiments can be made or executed within the spirit, scope and concept disclosed herein.

All the examples were carried out or can be carried out, unless otherwise disclosed herein specifically, by standard techniques which are well known and conventional to those skilled in the field of the art.

The concrete manipulations and treatment conditions in the following Examples are performed, unless indicated particularly, according to methods described in J. Sambrook, E. F. Fritsch & T. Maniatis, "Molecular Cloning: A Laboratory Manual", (2nd edition, 1989 & 3rd edition, 2001), Cold Spring Harbor Laboratory Press, Cold Spring Harbor, New York and D. M. Glover et al. ed., "DNA Cloning", 2nd ed., Vol. 1 to 4, (The Practical Approach Series), IRL Press, Oxford University Press (1995) for DNA cloning; and H. A. Erlich ed., PCR Technology, Stockton Press, 1989 ; D. M. Glover et al. ed., "DNA Cloning", 2nd ed., Vol. 1, (The Practical Approach Series), IRL Press, Oxford University Press (1995) and M. A. Innis et al. ed., "PCR Protocols", Academic Press, New York (1990) for PCR method. Commercially available reagents and kits are used according to the attached protocols using the attached substances.

### Example 1

### [Animal and tissue preparation]

Whitefaced crossbred ewes were maintained at the US Meat Animal Research Center (Clay Center, NE, U.S.A.). The protocol for sheep experimentation has been reviewed and approved by the animal care committees at the USDA.

Animal care and estrous synchronization procedures were performed as in Imakawa K. et al., Endocr. J., 45: 441-450 (1998). Uteri from cyclic ewes of on Day 15 (n = 9) and pregnant ewes on Days 14 (n = 3), 17 (n = 9), 20 (n = 3), 25 (n = 3), and 30 (n =3) were removed immediately after slaughter. Whole uteri from Day 15 cyclic and Day 17 pregnant ewes (n = 3 each) were immobilized as in Imakawa K. et al., Endocr. J., 45: 441-450 (1998), and then frozen immediately for subsequent in situ hybridization studies. Endometrial and conceptus (embryos and embryonic membranes) tissues collected from the remaining pregnant ewes (n = 3 for each day examined) were frozen and stored at -70°C. These frozen tissues were used for RNA extraction. At the University of Tokyo farm, whole blood was collected from three cyclic ewes. Peripheral blood mononuclear cells (PBMCs) were obtained from the whole blood. These samples were used for IFN dose response and chemotaxis assays. Whole uteri were obtained from three additional cyclic ewes, and endometrial explants were cultured to examine the stimulatory effect of IFNs on IP-10 production. The use of sheep has been approved by the animal care committee at the University of Tokyo. Animal care and estrous synchronization were performed accordingly as in Aida H. et al., J Peprod. Dev., 45: 249-257 (1999) .

### [In vitro culture]

Primary monocytes and lymphocytes were isolated from Day 15 cyclic ewes using a method (Domenech A. et al., (2000) J Gen. Virol., 81: 109-118) with minor modification. PBMCs were separated from EDTA-treated blood (80 mL) by density gradient centrifugation (800 × g, 20°C, 30 min, OptiPrep™, Nycomed, Roskilde, Denmark) and were suspended in RPMI 1640 medium supplemented with 10% fetal calf serum (FCS), 40 units/mL of penicillin, 40µ/mL of streptomycin, and anti-pleuropneumonia-like organisms (PPLO; Invitrogen Corp., Carlsbad, CA, U.S.A.). PBMCs (3 × 10⁷ cells/mL) were plated in 6-well coaster plates (3 mL/well), which were incubated at 37°C in a 5% CO₂-95% air atmosphere for 2 hours. The adherent cells (monocytes) were separated from nonadherent cells (lymphocytes) and then cultured in the fresh RPMI 1640 medium with same supplements as above. To determine effective doses of IFNs on IP-10 expression, monocytes were treated with 10², 10³, or 10⁴ IU/mL recombinant human IFN-α (rhIFN-α, Sigma, St. Louis, Missouri, U.S.A.), recombinant human IFN-γ (rhIFN-γ, Upstate biotechnology, Lake Placid, New York, U.S.A.), or recombinant bovine IFN-τ (rbIFN-τ, Katakura Industries Co.,). After 20 hours at 37°C in a 5% CO₂-95% atmosphere, culture media were harvested and stored at -70°C, and cells were immediately processed for total RNA extraction. Ovine uterine epithelial and stroma cells (Johnson G.A. et al., (1999) Biol. Reprod., 61: 1324-1330) were cultured in Dulbecco's modified Eagle's medium (DMEM) supplemented with 40 units/mL of penicillin, 40 µ/mL of streptomycin, and 10% FCS. The cells are a gift from Dr. Bazer, Texas A & M.

Endometrial tissues (approximately 600 mg wet weight/culture dish) from Day 15 cyclic ewes were cultured in 20 mL of DMEM supplemented with 40 units/mL of penicillin and 40 µ/mL of streptomycin, which were treated with 10² IU/mL of rhIFN-α, rhIFN-γ, or rbIFN-τ. The doses of those IFNs had been determined by the dose-response experiments with monocytes. After 20 hours at 37°C in a 5% CO₂ atmosphere, culture media and endometrial tissues were frozen separately and stored at -70°C until subsequent Western and Northern blot analyses.

### [Cloning of Ovine IP-10]

Complementary DNA library construction and substruction experiments were completed using Day 17 pregnant and Day 15 cyclic endometrial RNAs according to the method described in Akopyants N.S. et al., Proc. Natl. Acad. Sci. U.S.A., 95: 13108-13113 (1998). A cDNA fragment encoding ovine IP-10 was identified from numerous cDNA substruction studies. The full-length IP-10 was subsequently obtained using 5'-RACE, 3'-RACE, and full-length polymerase chain reaction (PCR) methodologies (Kuraishi T. et al., (2000) Biochem. J., 347: 579-583). The PCR products of full-length IP-10 cDNA were ligated to a pCRII™ vector (Invitrogen) and then subjected to an automated sequence analysis using a Perkin-Elmer sequencer (model ABI Prism 377 XL: Roche Molecular Systems, Branchburg, NJ, U.S.A.). Nucleotide sequence of ovine IP-10 cDNA were analyzed with Genetyx software program (Software Development Co., Ltd. Tokyo, Japan).

### [Preparation of probe and semiquantitative analysis by RT-PCR]

To prepare cRNA probes specific for ovine IFN-τ, IP-10, and G3PDH, respective cDNAs were generated from ovine endometrial or conceptus RNA using RT-PCR. Total RNA samples were reversely transcribed with SuperScriptII™ (Invitrogen) and oligo-dT primer (20 µL reaction volume), and the resulting cDNAs were then subjected to PCR amplification with primers shown in Table I.

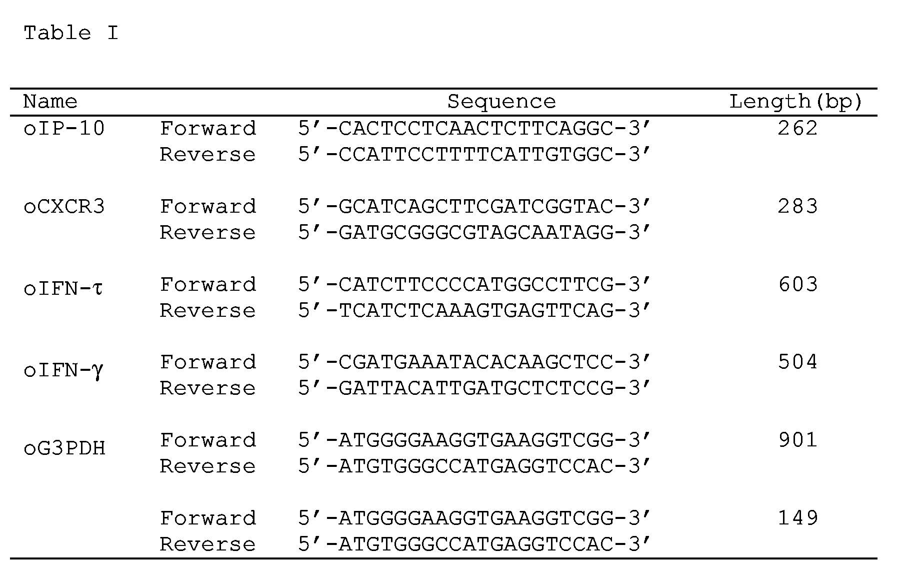

Table I shows primer sets used for PCR. Wherein, Name: target gene's name for PCR amplification, Sequence: nucleotide sequence of primer, and Length: chain length of the target gene.

Each RT-PCR-derived fragment was subcloned into the pCRII™ vector and then subjected to an automated sequence analysis. Sequence comparisons were performed using the BLAST network program (National Center for Biotechnology Information, NIH, Bethesda, MD, U.S.A.) and confirmed correct ovine cDNAs. DIG-labeled cRNA probes for Northern blot analysis were generated from those cDNA constructs using T7 RNA polymerase or SP6 RNA polymerase (Kuraishi T. et al., (2000) Biochem. J., 347: 579-583).

Amounts of uterine IFN-γ mRNA and CXCR3 mRNA were determined from PCR amplification using oligonucleotide primers (Table I). Each reaction consisting of primer pairs for IFN-γ/G3PDH or CXCR3/G3PDH was run with RT template (1 µL) and AmpliTaq Gold™ (1.25 Unit; Roche Molecular Systems) in a final volume of 25 µL. Ratios of primer pairs that gave each PCR product within the linear range had been determined, i.e. 6:1 for IFN-γ:G3PDH and 5:2 for CXCR3:G3PDH. PCR conditions were 95°C for 11 min, 40 cycles at 95°C for 1 min, 57°C for 1 min, and 72°C for 1 min followed by a final extension at 72°C for 5 min. PCR products were quantified using an image analysis system (ToYoBo, Osaka, Japan) equipped with Quantity one v3.0 software.

### [Northern blot analysis]

Total RNA was isolated from some endometrial and conceptus tissues or cells using Isogen (Nippon Gene, Tokyo). Poly(A)⁺RNA was obtained from total RNA that had been isolated from PBMCs (TaKaRa, Tokyo, Japan). These RNAs were separated by electrophoresis on a 1.0% agarose-formaldehyde gel and transferred to a nylon membrane (Biodyne-B; Pall, East Hills, NY, U.S.A.). The nylon membrane was prehybridized in the hybridization buffer containing 5× SSC, 50% formamide, 50 mM PBS, 7% SDS, 0.1% N-lauryl sarcosine, 50 µg/mL salmon sperm DNA (ssDNA), and 2% blocking reagent (Roche Diagnostics, Mannheim, Germany) at 65°C for 1.5 hours, and then hybridized with cRNA probe in fresh hybridization buffer at 63°C for 12 hours. After hybridization, the nylon membrane was washed once with 2× SSC and 0.1% SDS at 65°C for 30 min, washed twice with 0.1× SSC and 0.1% SDS at 65°C for 30 min, and then incubated with RNase-A (20 µg/mL) at 37°C for 1 hour. The nylon membrane was incubated in the blocking buffer (1% blocking reagent) at room temperature for 1 hour followed by the addition of anti-DIG antibody (1:10,000 dilution; Roche Diagnostics, Mannheim, Germany). The nylon membrane was treated three times with the washing solution containing 100 mM maleic acid (pH 7.5), 150 mM NaCl, and 0.3% Tween 20 for 10 min each and finally rinsed in 100 mM Tri-HCl (pH 9.5) and 100 mM NaCl. The chemluminescent reaction was performed in 100 mM Tri-HCl (pH 9.5) and 100 mM NaCl containing CSPD reagent (1:100 dilution; Roche Diagnostics, Mannheim, Germany), and the nylon membrane was exposed to X-ray film.

### [In situ hybridization]

In situ hybridization was performed according to a known method (Kanai Y. et al., J Cell Biol., (1996) 133: 667-681) with minor modification. Frozen tissues were sectioned (10 µm), mounted onto silan-coated slides, and fixed in 4% paraformaldehyde in PBS. Slide sections were pretreated sequentially with 0.2 N HCl and 20 µg/ml proteinase K in Tris-HCl (ph 7.6), 4% paraformaldehyde, and 0.2% glycine. Slide sections were then prehybridized in the solution containing 50% formamide, 5 × SSC, 1 × Denhardt's, 100 µg/mL heparin, 10 mM dithiothreitol, 10% dextran sulfate, and 0.1 mg/mL denatured tRNA and ssDNA, followed by hybridization with DIG-labeled antisense or sense cRNA probes at 45°C for 16 hours. After hybridization and washing once with 4 × SSC at 42°C for 20 min, slide sections were incubated with RNase-A (10 µg/mL) at 37°C for 30 min and then washed twice 2 × SSC at 65°C for 30 min and twice with 0.1 × SSC at 65°C for 30 min. The slides were blocked with a blocking reagent (Boehringer Mannheim) and incubated with an anti-DIG alkaline phosphatase-conjugated antibody (Boehringer Mannheim). The signals were detected with 5-bromo-4-chloro-3-indolyl phosphate and nitro blue tetaozlium (Promega, Madison, WI, U.S.A.) .

### [Expression and purification of recombinant caprine IP-10]

Through the use of RT-PCR, caprine IP-10 (cIP-10) cDNA was amplified from caprine endometrial RNA by using primer set as in ovine IP-10. The cDNA was cloned into a pSTBlue (trade name; TaKaRa) plasmid. The nucleotide sequence encoding the mature region of cIP-10 was amplified and cloned into an expression vector, pET-14b (trademark, Novagen, Madsion, WI, U.S.A.), which consisted of a histidine tag at the N-terminal side of cIP-10. The resulting expression vector was referred as pET-14b-cIP-10. The expression vector was used transform Escherichia coli BL21-SI (Invitrogen) cells. The grown cells were harvested, suspended in 50 mM NaH₂PO₄, 500 mM NaCl, and 10 mM imidazole (pH 7.4), and disrupted on ice by sonication. After cell debris and insoluble proteins were removed by centrifugation, recombinant cIP-10 in the supernatant was examined using SDS-PAGE. Recombinant cIP-10 was purified using a nickel-chelating column (Hi-trap Chelating HP, Amersham Pharmacia Biotech) on the chromatography system (AKTA, Amersham Pharmacia Biotech). The protein was eluted from the column with a linear gradient of imidazole (20-500 mM), which then dialyzed against PBS to remove imidazole.

### [Western blot analysis]

The culture media after in vitro culture of endometrial tissues and the dialyzed protein (recombinant IP-10) were analyzed for the presence of IP-10 by using Western blot analysis. Culture medium (40µl) or recombinant IP-10 (50 ng/40 µL or 200 ng/40 µL) was boiled for 5 min in the SDS sample buffer, electrophoresed on 15% SDS-PAGE gels under reducing conditions, and transferred onto nitrocellulose membranes (Immunobilon; Millipore, Bedford, MA, U.S.A.). The membranes were blocked with Block Ace (Dainippon Pharmceutical, Osaka) at room temperature for 1 hour and then incubated with a mouse monoclonal antibody to human IP-10 (Genzyme/Techne, Minneapolis, MN, U.S.A.) or with a rabbit polyclonal antibody (Sigma) to histidine tag at 4°C for 12 hours. After incubation, the membranes were washed three times (10 min each) in a TBS-Tween 20 solution, and incubated with donkey anti-mouse IgG or anti-rabbit IgG conjugated with horseradish peroxidases at room temperature for 1 hour, and washed three times (15 min each) in a TBS-Tween 20 solution. The bands were detected with SuperSignal West Femto Maximum Sensitivity Substrate kit (Pierce, Pockford, IL, U.S.A.).

### [Immunohistochemical analysis]

Whole ovine uteri containing both the endometrial and conceptus tissues were fixed in 4% paraformaldehyde, embedded in paraffin, and slide sectioned (6 µm). After deparaffin, slide sections were immersed in 0.01 M sodium citrate buffer solution (pH 6.0) and treated in autoclave at 121°C for 15 min. The slide sections were incubated in a methanol solution containing 4.5% H₂O₂ at room temperature for 1 hour, then incubated with Block Ace in a high humidity chamber for 1 hour. Then, the slide sections were incubated with a first antibody, i.e. anti-human IP-10 monoclonal mouse antibody (1 µg/ml; Genzyme/Techne) or polyclonal anti-human IFN-γ (500 ng/ml; Peprotech, London, U.K.) at 4°C overnight. After the incubation, the slide sections were treated with a secondary antibody (Vectastain ABC Kit; Vector Labs, Burlingame, CA, U.S.A.) for 1 hour, and then incubated with an avidin-biotin complex in a PBS at room temperature for 30 min. Antibodies bound to the slide sections were visualized with Metal Enhanced DAB Substrate kit (Pierce). For evaluating non-specific binding (negative control), normal mouse or rabbit IgG prepared to the same concentration is used as semi-serial segments of tissues. The tissue segments were counterstained with hematoxylin.

### [In vitro chemotaxis assay]

Migration of PBMCs was assessed in a 96-well modified Boyden chamber (NeuroProbe, Cabin John, MD, U.S.A.) using polyvinylpyrrolidone-free polycarbonate membrane (5 µm pore size; NeuroProbe), which had been coated with 10 µg/ml bovine plasma fibronectin for 2 hours before use. The assay was performed as described in Gasperini S. et al., (1999) J. Immunol., 162: 4928-4937 with minor modifications. DMEM without FCS and DMEM containing recombinant IP-10 (0.2 to 500 ng/mL) or the supernatants from endometrial cultures treated with or not treated with IFN-τ were added to the bottom wells of the chemotaxis chamber, and PBMCs (5×10⁶ cells/mL) in DMEM without FCS were added to the top wells of the chamber. After the chambers were incubated at 37°C in a 5% CO₂ atmosphere for 2 hours, the membranes were removed, washed with PBS, fixed, and stained with Dif-Quick. The number of cells that migrated to the lower layer was microscopically counted in six randomly chosen high-power fields. For the blocking experiments, the supernatants from endometrium stimulated with IFN-τ or 5 ng/mL of recombinant IP-10 were preincubated at 37°C for 1 hour with 30 µg/mL of anti-IP-10 or control mouse IgG before addition to the top chamber. The assays were replicated in triplicate.

### [Statistical Analysis]

Each data point shows three samples that are independent to each other and represented by means ± SEM. These data were analyzed by one-way ANOVA, and then Duncan's multiple range tests. Additionally, Northern blotting data in pregnant ovine were analyzed by regression analysis using the least-squares method. Differences with a P values < 0.05 were considered statistically significant.

### Results

### [Cloning of IP-10 cDNA]

Ovine IP-10 cDNA fragments were obtained from endometrial tissues collected on Day 17 of pregnancy, and a full-length ovine IP-10 cDNA was cloned using 5'-RACE and 3'-RACE. The resulting ovine IP-10 cDNA encompasses 1172 base pairs (bp) with an open reading frame (ORF) corresponding to 102 amino acids. The nucleotide sequence of the cloned ovine IP-10 and an amino acid sequence deduced therefrom are shown in Fig. 1. In 1172 bp cDNA sequence (GenBank™ Accession No. AB070717), shown in Fig. 1, containing the ORF corresponding to 102 amino acid residues, the first 9 amino acids segment of the coded amino acid sequence (ovine IP-10; GenBank™ Accession No. BAB63958) was presumed as a signal peptide.

Caprine IP-10 cDNA was cloned from caprine endometrial RNA by RT-PCR using a primer set designed from ovine IP-10 cDNA. The cIP-10 cDNA sequence was the same as that of caprine other than that the coded 99^{th} amino acid residue was arginine in cIP-10 instead of glutamine in ovine IP-10.

Comparative analysis of the amino acid sequences among various species is shown in Fig. 2. Four cysteine residues are conserved within chemokine family, and the first two cysteines are separated by a single amino acid to make C-X-C. This cysteine motif was found in amino acid residues deduce from ovine IP-10 cDNA and caprine IP-10 cDNA. The C-X-C chemokines are subdivided into two classes depending on the presence of the glutamine-leusine-arginine (ELR) motif preceding the first two cysteines (Rollins B.J., (1997) Chemokines, 90: 909-928). Similar to IP-10s of other species, the ovine IP-10 lacked the ELR motif and showed a high degree of similarity to human IP-10, i.e. 82.7% and 75.5% at the nucleotide and amino acid levels, respectively (Table II).

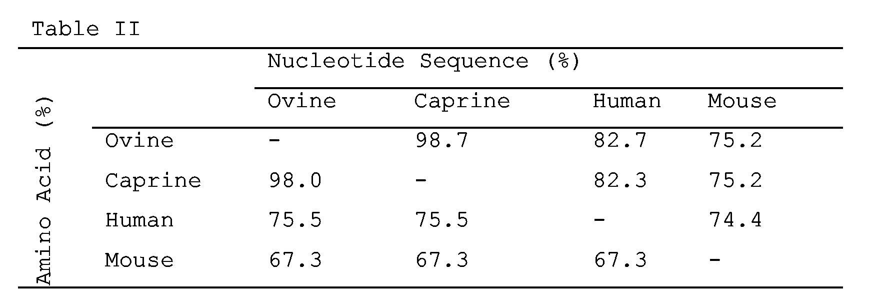

Table II shows the homology (identity) among ovine, caprine, human, and mouse in amino acid and nucleotide sequences.

### [Changes of IP-10, CXCR3, IFN-τ, and IFN-γ mRNA expression during early pregnancy]

Uterine IP-10 mRNA levels on Days 14, 17, 20, 25, and 30 of pregnancy were examined by Northern blot analysis (Fig. 3). Fig. 3 shows levels of IP-10 mRNA expression in the ovine uterus during early pregnancy. In left of Fig. 3, results in IP-10 mRNA in the uterus of pregnant ewes (n = 3 for each of Days 14, 17, 20, 25, and 30), cyclic ewes (Day 15, n = 3), and in the Day 17 conceptus (Con) are shown, which are each one result from three independent experiments. In right of Fig. 3, signal ratios IP-10/G3PDH derived from all the results of Northern blot analysis of IP-10 are shown, in which mean ± SEM are represented by bars. An asterisk in right of Fig. 3 indicates a statistical difference (p < 0.05) when compared with the value from Day 15 cyclic uteri. Result of regression analysis on IP-10 mRNA and day of pregnancy was y = -51.5x² + 288.2x - 131.2 (R² = 0.823, p < 0.01).

Consistent with the result from ovine IP-10 cDNA cloning experiments, a single transcript (approximately 1.1 kb) for IP-10 mRNA was detected in uterine endometrium of pregnant ewes. The expression of endometrial IP-10 mRNA was much higher on Days 14, 17, 20, and 25 of pregnancy than that in cyclic ewes. Expression of endometrial CXCR3 mRNA, a receptor for IP-10, was higher on Days 17 and 20 in pregnant ewes (Fig. 4). Fig. 4 shows levels of CXCR3 mRNA expression. In left of Fig. 4, results of semiquantitative PCR of CXCR3 mRNA and G3PDH mRNA in the uterus of pregnant (n = 3 for each of Days 14, 17, 20, 25, and 30) and cyclic ewes (Day 15, n = 3) are shown, which are each one result from three independent experiments. In right of Fig. 4, results of densitometric analysis of semiquantitative PCR products for CXCR3 mRNA and G3PDH mRNA are shown as ratios of CXCR3 mRNA to G3PDH mRNA (CXCR3 mRNA/G3PDH mRNA). Bars represent mean ± SEM. An asterisk in right of Fig. 4 indicates a statistical difference (p < 0.05) when compared with the value from Day 15 cyclic uteri. Result of regression analysis on CXCR3 mRNA and day of pregnancy was y = -8.9x² + 45.8x + 61.4 (R² = 0.431, p < 0.01).

Fig. 5 shows levels of IFN-τ and IFN-γ mRNA in the ovine conceptus and uterus during early pregnancy. In left (A) of Fig. 5, results of Northern blot analysis of IFN-τ mRNA in the conceptuses of pregnant ewes (n = 3 for each of Days 14, 17, and 20) are shown, and in right (B), results of semiquantitative PCR of IFN-γ mRNA and G3PDH mRNA in the uterus of pregnant ewes (n = 3 for each of Days 14, 17, 20, 25, and 30), cyclic ewes (Day 15, n = 3), and in the Day 17 conceptus (Con) are shown, which are each one result from three independent experiments. In the bottom of right in Fig. 5, results of densitometric analysis of semiquantitative PCR products of IFN-γ mRNA and G3PDH mRNA are shown as ratios of IFN-γ mRNA to G3PDH mRNA (IFN-γ mRNA/G3PDH mRNA). Bars represent mean ± SEM. An asterisk indicates a statistical difference (p < 0.05) when compared with the value from Day 15 cyclic uteri. Result of regression analysis on IFN-γ mRNA and day of pregnancy was y = -10.6x² + 56.6x + 81.3 (R² = 0.762, p < 0.01).

Expression of IFN-τ mRNA was detected in the conceptus, and the level of this expression was higher in the Day 14 conceptus than others (Fig. 5A). Expression of IFN-γ mRNA was detected in the uterine endometrium of both pregnant and cyclic ewes and was higher on Days 14, 17, 20, and 25 in pregnant ewes that others (Fig. 5B).

### [Localization of IP-10 and IFN-γ proteins]

To determine localization of IP-10 and IFN-γ, immunohistochemical analysis was performed on sections of uterine and conceptus tissues prepared from Day 15 cyclic and Day 17 pregnant ewes (Fig. 6). In Fig. 6, the Day 15 cyclic ewes (left; A, B, and C) and Day 17 pregnant ewes (right; D, E, and F) are practically shown as a series of sections. Photomicrographs A and D show IP-10, B and E show IFN-γ, and C and F show control.

Both IP-10 and IFN-γ proteins localized in luminal and glandular epithelium and subepithelial stroma of the cyclic and pregnant uteri. Amount of IFN-γ protein detected in endometrium of cyclic ewes was the same as that of pregnant ewes (Fig. 6). IP-10 protein was detected in endometrium of cyclic ewes, but the degree of the staining was the minimum.

### [Localization of IP-10 mRNA]

To determine a cellular source of IP-10 in the sheep endometrium, in situ hybridization was performed on sections of uterine tissues prepared from Day 15 cyclic and Day 17 pregnant ewes (Fig. 7A). In Fig. 7, photomicrographs a and b are results of analysis of IP-10 mRNA in the uterus in Day 17 pregnant ewes by DIG-labeled antisense IP-10 cRNA, photomicrograph c is results of analysis of IP-10 mRNA in the uterus in Day 17 pregnant ewes by DIG-labeled sense IP-10 cRNA, and photomicrograph d is results of analysis of IP-10 mRNA in the uterus in Day 15 cyclic ewes by DIG-labeled antisense IP-10 cRNA.

IP-10 mRNA was detected in the subepithelial stroma but not in the luminal and glandular epithelium of the pregnant uterus. At higher magnification, the signal appeared to be present in immune cells. Northern blot analysis was performed to identify tissue and/or cell types expressing IP-10. IP-10 mRNA was found in RNA extracted from monocytes but not in RNA from lymphocytes, epithelial cells, or stroma cells (Fig. 7B).

### [Effect of IFN-τ on IP-10 expression]

Various IFNs were investigated for their dose responses and their ability to stimulate IP-10 mRNA expression in monocytes. Monocytes isolated from ewes were cultured in vitro for 20 hours in the presence or absence of 10² to 10⁹ IU/mL of IFN-α, IFN-γ, or IFN-τ. Effects of several IFNs on IP-10 mRNA levels are shown in Fig. 8. Results of the dose-response experiments for the expression of IP-10 mRNA in IFNs-stimulated monocytes are shown in Fig. 8A, which are each one result from three independent experiments.

All IFNs stimulated the expression of IP-10 mRNA in ovine monocytes, however, the effective doses were different for each IFN (Fig. 8A). IFN-τ at a dose of 10² IU/mL stimulated the expression of IP-10 mRNA more effectively than the other IFNs examined.

In cyclic ewes, it was investigated whether IFNs could effect endometrial IP-10 mRNA production. Endometrial explants from Day 15 cyclic ewes were cultured in vitro for 20 hours in the presence or absence of 10² to 10⁴ IU/mL of IFN-α, IFN-γ, or IFN-τ. Total RNA was extracted from IFN-stimulated endometrial tissues and examined for IP-10 mRNA by Northern blot analysis. Minute levels of IP-10 mRNA were detected in controls and in IFN-α-stimulated or IFN-γ-stimulated samples, but high levels of IP-10 mRNA were found only after stimulation with IFN-τ (Fig. 8B). Western blot analysis confirmed that endometrial IP-10 production was stimulated by IFN-τ (Fig. 8C) .

Fig. 8B shows effect of several IFNs on IP-10 mRNA levels in the endometrial explants from cyclic ewes. In left of Fig. 8B, results of Northern blot analysis of IP-10 mRNA in the endometrial explants stimulated by 10² IU/mL of IFN-α, IFN-γ, or IFN-τ are shown, which are each one result from three independent experiments. In right of Fig. 8B, results of densitometric analysis of Northern blots of IP-10 mRNA and G3PDH mRNA are shown. Signal ratios IP-10/G3PDH were derived from all the results of Northern blot analysis. Bars represent mean ± SEM. An asterisk indicates a statistical difference (p < 0.05) when compared with the value from endometrial explants cultured without IFN. In Fig. 8C, results of Western blot analysis of proteins in the culture medium from endometrial explants stimulated by IFNs are shown.

### [Effect of IP-10 on PBMC migration]

To ascertain whether recombinant caprine IP-10 (rcIP-10) and endometrium-derived IP-10 were biologically active, effect of rcIP-10- or IFN-τ-stimulated endometrial culture media on migratory activities of PBMCs were investigated by in vitro chemotaxis assays. Recombinant cIP-10 was purified using a nickel chelating column, and detected by Western blot using antibody against IP-10 and histidine-tag (Fig. 9A). Receptor for IP-10, CXCR3, mRNA exists in PBMC was confirmed (Fig. 9B, lane T: total RNA, 10 µg, lane M: poly(A)⁺ RNA, 2 µg). Chemotaxis assay confirmed that PBMC responded to 0.2 to 5 ng/mL of rcIP-10 but the reaction decreased in higher levels of rcIP-10. As a result, the dose-response showed a characteristic bell-shape curve (Fig. 9C). Medium from endometrial tissue culture that had been stimulated by IFN-τ exerted significant chemotaxis effect on PBMCs, whereas the medium obtained from the endometrium cultured without IFN-τ was ineffective (Fig. 9C). Bars represent mean ± SEM. An asterisk indicates a value (p < 0.05) when compared with the migration of PBMCs with IFN-τ-untreated endometrial culture medium. Double asterisks indicate a value (p < 0.05) when compared with the migration of PBMCs in the absence of rcIP-10. Immunoneutralization experiments revealed that an anti-IP-10 antibody reduced the chemotactic activities of culture medium from IP-10- or IFN-τ-stimulated endometrium by 60 to 70%, indicating that the chemotactic activity exhibited by the culture medium from IFN-τ-stimulated endometrium was due mostly to IP-10 (Fig. 9D). Bars represent mean ± SEM. An asterisk indicates a migration value of PBMCs in IFN-τ-treated endometrial culture medium in the absence of an anti-IP-10 antibody at p < 0.05. Double asterisks indicate a migration value of PBMCs by IP-10 in the absence of anti-IFN-τ antibody at p < 0.05.

### [Discussion]

The present invention disclosed that ovine IP-10 was present in the pregnant uterus and to a much lesser degree in the cyclic endometrium. IP-10 is a member of the C-X-C chemokine family, which regulates multiple aspects of inflammatory and immune responses through the receptor for IP-10, CXCR3, and/or chemotactic activity of subsets of leukocytes (Farber J.M. et al., (1997) J Leukoc Biol., 61: 246-257).

The present invention successfully disclosed by the dose-response experiment using monocytes that IFN-α, IFN-γ, and IFN-τ stimulated IP-10 mRNA expression, but the effective doses differed among IFNs. IFN-τ effectively stimulated IP-10 expression in monocytes at a dose of 10² IU/mL, whereas rhIFN-γ at the same dose was less effective. It is unclear whether this low level of stimulation of IP-10 mRNA by IFN- y reflects the fact that IFN- y is highly species specific compared other IFNs (Pestka S. et al., (1987) Ann. Rev. Biochem., 56: 727-777). It should be paid attention that a high dose of rhIFN-γ (10⁴ IU/mL) stimulated the expression of IP-10 mRNA in monocytes. Thus, the low stimulatory effect of IFN-γ on IP-10 production was not solely due to a loss of activity. The pattern of endometrial IP-10 mRNA expression appeared to have followed that of conceptus IFN-τ mRNA during early pregnancy, and changes in IP-10 expression were similar to changes in other endometrial chemokines such as monocyte chemoattractant protein (MCP)-1 and MCP-2.

IP-10 mRNA was detected by in situ hybridization analysis in the subepithelial stroma, and the signal appeared to be localized in immune cells. In studies about human and mouse hitherto known, IP-10 is secreted by monocytes (Luster A. et al., (1985) Nature, 315: 672-676; Ohmori Y. et al., (1990) Biochem. Biophys. Res. Commun., 168: 1261-1267), and in the present invention, IP-10 was likewise expressed in ovine monocytes but not lymphocytes, epithelial cells, or stroma cells. MCP mRNAs are also localized in the subepithelial stroma, but MCP-positive cells are eosinophils (Asselin E. et al., (2001) Biol. Peprod., 64: 992-1000). These observations suggest that IP-10 in the subepithelial stroma is produced by resident macrophages and/or macrophages that have been recruited to that region. IP-10 protein was localized in luminal and glandular epithelium of the uteri as well as localized in subepithelial stroma region. In the conceptus, very small amount of IP-10 protein was detected, but IP-10 mRNA was not detected. It was suggested that IP-10 produced by macrophages in the subepithelial region migrate into epithelium, which stimulates migration of CXCR3-expressing immune cells toward or near the site of conceptus implantation to the maternal endometrium.

As the conclusion, the present invention revealed that IP-10 and IFN-γ mRNA expressions time-varyingly and cell-specifically occur during early pregnancy in the ovine uterus and that conceptus IFN-τ has an ability to regulate endometrial IP-10 expression. These findings suggest that IP-10 regulated by IFN-τ plays an important role in the recruitment and/or redistribution of immune cells during early pregnancy in ruminants.

The present invention provides a way for investigating relationship between IP-10 and IFN-τ and IP-10 functions in uterus.

### Example 2

### [Preparation of animal tissues]

Uteri from cyclic goats on Days 14 and 17 and pregnant goats on Days 14, 17, and 20 were removed by hysterectomy under isoflurane anesthesis. Whole uteri from Day 14 cyclic and Days 14 and 17 pregnant goats were frozen for in situ hybridization analysis. Uteri from Days 14 and 17 pregnant and Days 14 and 17 cyclic goats were subjected to uterine flushing procedure for the isolation of conceptuses and/or collection of flushed media. PBS (15 mL) was gently flushed into the uterus, and the uterine flushing media collected were concentrated (Macrosep, Pall Corporation, East Hills, NY) and subjected to the Western blot analysis for the detection of IP-10. Conceptus tissues collected from Days 14 (whole conceptus), 17 (approximately one half of conceptus), and 20 (trophoblast region only) of pregnant goats were subjected to in vitro culture. The remaining Days 17 and 20 conceptus tissues were frozen for subsequent RNA extraction and immunostaining. The in vitro culture media were subjected to Western blot analysis for the detection of IFN-τ. Uteri from Day 14 cyclic goats were used for epithelial cell isolation. Endometrial explants from Day 14 cyclic goats were cultured to examine the stimulatory effect of IFN-τ on IP-10 production. Endometrial tissues of goats from which conceptuses had been removed were frozen and subjected to RNA extraction.

### [In vitro culture]

Whole conceptuses (Day 14, n = 3) and minced conceptuses (Days 17 and 20, about 200 mg/culture dish, n = 3 each) were cultured under conditions according to a method of Imakawa, et al. (Endocrine (1995), 3, 511-517).

The procedures for endometrial epithelial cell dissociation and subsequent characterization were performed according to a method of Takahashi, et al. (J. Reprod. Dev. (2001) 47, 181-187). Uterine horns from Day 14 cyclic goats were filled with 0.76% EDTA-PBS and preincubated at 37°C for 1 hour. The endometrial epithelium was scraped off by a surgical blade and then incubated at 37°C for 8 min in a 0.1% collagenase (Sigma, St. Louis, MO). The cell suspension was passed through a nylon mesh (70 µm) and subjected to density gradient centrifugation. Collected epithelial cells were resuspended in Dulbecco's modified Eagle's medium/Ham's F-12 (DMEM/Ham's F12, Sigma) supplemented with 40 units/mL of penicillin, 40 µg/mL of streptomycin, and 10% FCS. The cells were plated in 24-well dishes coated with swine-skin type I collagen (Nitta gelatin, Osaka, Japan) and subjected to the adhesion assay when the cells had grown to confluent.

Cells were prepared from Day 17 goat conceptus. These conceptuses were dissected into small pieces and incubated in 0.2% collagenase (Sigma) at 37°C for 30 min. The cells were passed through a nylon mesh (70 µm) and resuspended in DMEM with antibiotics, which were immediately used for chemotaxis or adhesion assays. In addition, bovine B cells (KU-1, 33) and caprine trophoblast clone cells (HTS-1, 33) were cultured in DMEM supplemented with 10% to 20% FCS and antibodies.

Endometrial tissues (about 600 mg wet weight/culture dish) from Day 14 cyclic goats were cultured in 20 mL of DMEM supplemented with antibiotics, and were treated with 5 nM recombinant bovine IFN-τ (rbIFN-τ, Katakura Industries Co., Inc., Tokyo, Japan). After 24 hours at 37°C and 5% CO₂ atmosphere, endometrial tissues were frozen and stored at -70°C for subsequent RNA extraction and Northern blot analyses.

### [Cloning of caprine IP-10 and CXCR3 cDNA]

Through the use of RT-PCR, as shown Example 1, caprine IP-10 and CXCR3 cDNAs were amplified from caprine endometrial RNA and subcloned into a pSTBlue vector (Takara, Tokyo, Japan), which was then subjected to an automated sequence analysis using a PerkinElmer sequencer (model ABI Prism 377 XL, Roche Molecular System, Branchburg, NJ). Nucleotide sequence comparisons of caprine IP-10 and CXCR3 were performed using the BLAST network program (National Center for Biotechnology Information, NIH, Bethesda, MD) and GenBank accession numbers were obtained (AB099892 and AB099893, respectively).

### [Transfection of CXCR3 expression plasmid]

The nucleotide sequence encoding the open reading frame of caprine CXCR3 was PCR-amplified and cloned into a mammalian expression vector pcDNA3.1 (Invitrogen Corp., Carlsbad, CA). This plasmid was transfected into KU-1 and HTS-1 cells by using Transfast™ (Promega, Madison, WI) accordint to the manufacturer's protocol. Two days after the transfection, cells were used for chemotaxis or adhesion assay.

### [Production of recombinant caprine IP-10 and the antibody]

The nucleotide sequence encoding the mature region of caprine IP-10 (cIP-10) cDNA was PCR-amplified and cloned into an expression plasmid, pET-14b (Novagen, Madison, WI), which consisted of a histidine tag at the N-terminal side of cIP-10. This expression vector, pET-14b-cIP-10, was used to transform Escherichia coli BL21-SI (Invitrogen) cells, which were cultured overnight. These cells were harvested, resuspended in 50 mM NaH₂PO₄, 500mM NaCl, and 10 mM imidazole (pH 7.4), and disrupted on ice by sonication. After cell debris and insoluble proteins were removed by centrifugation, recombinant cIP-10 (rcIP-10) in the supernatant was examined using SDS-PAGE. Recombinant cIP-10 was purified using a nickel-chelating column (Hi-trap Chelating HP, Amersham Pharmacia Biotech Inc., Buckinghamshire, UK) on the chromatography system (AKTA, Amersham Pharmacis Biotech Inc.). The protein was eluted from the column with a linear gradient of imidazole (20 - 500 mM), which was then dialyzed against PBS to remove imidazole.

Recombinant cIP-10 (200 µg) proteins in PBS were mixed with Freund's complete adjuvant (Sigma) and injected subcutaneously into rabbits (n = 2). Recombinant cIP-10 proteins were injected two more times biweekly. A titer was checked 1 week after the last injection, and blood was collected on the following day. Anti-caprine IP-10 antibody was purified from antiserum using Hitrap Protein G column (Amersham Pharmacia Biotech Inc.).

### [SDS-PAGE and Western blot analysis]

After determining protein concentration using Protein assay kit II (Bio-Rad, Hercules, CA), concentrated uterine flushing media (3 to 8 µL, 10 µg of protein/lane, n = 3 each), or recombinant IP-10 (50 or 200 ng/lane) were subjected to Western blot analysis according to the method of Nagaoka, et al., (Biol. Reprod. (2003) 68; 1413-1421). Protein samples were boiled for 5 min in the SDS sample buffer, electrophoresed on 10% or 15% SDS-PAGE gels under reducing conditions. The gel was stained with Coomassie Blue. Proteins in the gel were transferred onto nitrocellulose membranes (Immobilon, Millipore, Bedford, MA) for Western blot analysis. The membranes were blocked with Block Ace (Dainippon Pharmceutical, Osaka) at room temperature for 1 hour, and then incubated with a rabbit polyclonal antibody to ovine IFN-τ (1:5000, a gift of Dr. Bazer), histidine tag (Sigma), or caprine IP-10 (1:1000) at 4°C for 12 hours. After incubation, membranes were washed three times in TBS-Tween 20, incubated with donkey anti-rabbit IgG-conjugated with horseradish peroxidases (Amersham Pharmacia Biotech Inc.) at room temperature for 1 hour, and washed three times in TBS-Tween 20. The bands were detected using ECL Western blot detection reagents (Amersham Pharmacia Biotech Inc.).

### [RT-PCR analysis]

Total RNAs were extracted from endometrial and conceptus tissues and cultured cells (n = 3 each) using Isogen (Nippon Gene, Tokyo, Japan). Total RNA samples were reverse-transcribed with SuperScriptII (Invitrogen) and oligo-dT primers (20 µL of reaction volume). Amounts of CXCR3, XCR1, and integrin receptor mRNAs were determined from PCR amplification using oligonucleotide primers (Table III) .

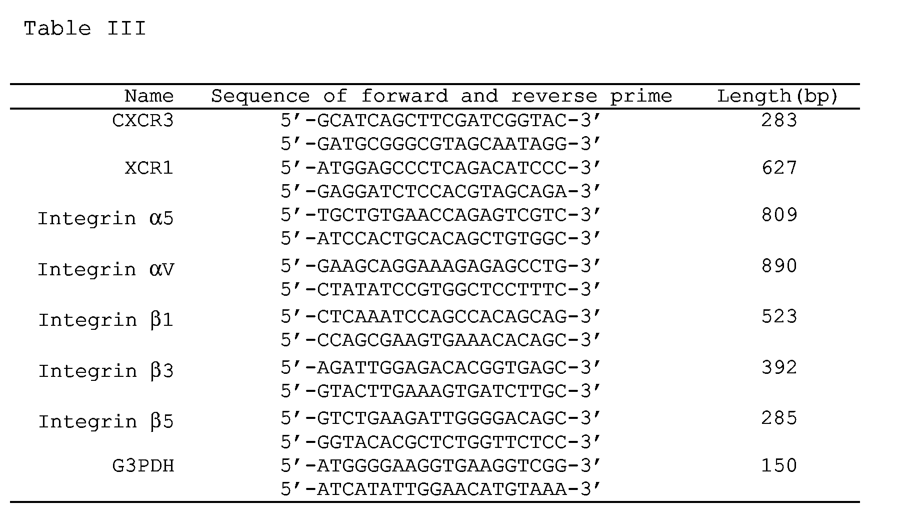

Each reaction consisting of primer pairs was run with RT template (1 µL) and AmpliTaq Gold (0.625 U; Roche Molecular System) in a final volume of 25 µL. All PCR reactions consisted of 40 cycles at 95°C for 1 min, 60°C for 1 min, and 72°C for 1 min, followed by the final extension at 72°C for 12 min. After agarose gel electrophoresis and visualization with ethidium bromide, PCR products were qualitatively detected by using an image analysis system (ATTO Corp., Tokyo, Japan) equipped with the Quantity One (v3.0 software; PDI, Inc., Huntington Station, NY).

### [Northern blot analysis]

Northern blot analysis was performed as a method of Nagaoka, et al., (Biol. Reprod. (2003) 68; 1413-1421). DIG-labeled cRNA probes were generated from IP-10 and CXCR3 cDNA plasmids by using T7 or SP6 RNA polymerase (Promega). Total RNAs were used for the determination of IP-10 mRNA whereas poly(A)⁺ RNA was used to examine CXCR3 mRNA. Poly(A)⁺ RNA was obtained from total RNA that had been isolated from PBMCs using Oligotex-dT30 (Takara). Total RNAs (20 µ g/lane, n = 3 each) or (A)⁺ RNA (2 µg/lane, n = 3 each) were separated by electrophoresis and transferred to a nylon membrane (Biodyne-B; Pall Corporation). The membrane was hybridized with cRNA probe at 63°C for 12 hours, and then signals were detected using DIG detection system.

### [Immunofluorescence analysis]

Frozen conceptus tissues sectioned (10 µm, n = 3 for each day examined) were mounted onto silan-coated slide and fixed in acetone. Nonspecific binding was blocked by the treatment with Block Ace at room temperature for 1 hour, and the slides were then incubated with a mouse monoclonal antibody to human CXCR3 (10 µg/ml, R&D Systems Inc., Minneapolis, MN) or normal mouse IgG (Sigma) at 4 °C for 12 hours. After the primary antibody incubation, slides were treated with fluoresceine isothiocyanate-conjugated goat anti-mouse IgG (15 µg/ml, Jackson ImmunoResearch Laboratories Inc., West Grove, PA) at room temperature for 1 hour. Nuclei were stained with propidium iodide (2 µg/ml, Sigma). The slides were scanning examined and digital images were captured on a confocal laser scanning microscope (FV300, OLYMPUS, Tokyo, Japan)

### [Protein biotinylation and detection]

Recombinant cIP-10 protein was biotinylated by EZ-link biotinylation reagent (Pierce, Rockford, IL). As a control, recombinant GST and recombinant caprine lymphotactin-GST protein were also labeled with biotin. The biotinylation was performed by incubating these proteins with 0.3 mg/mL EZ-link biotinylation reagent (1 mL) in PBS on ice for 2 hours. After the incubation, the resulting solutions were dialyzed to remove the free biotin and measured their protein concentrations. The biotinylated proteins (2 µg/lane) were applied on 15% SDS-PAGE gels under reducing condition and visualized by strepavidin-horseradish peroxidase (Amercham Pharmacia Biotech Inc.) in order to confirm the labeling efficiency.

Frozen conceptus tissue sections (10 µg, n = 3 each) were mounted onto silan-coated slides and fixed in acetone. The slides were blocked with Block Ace at room temperature for 1 hour and then incubated with biotinylated protein (100 µg/mL) at room temperature for 1 hour. After the incubation, the slides were incubated with streptavidin-horseradish peroxidase (1:5000, Amersham Pharmacia Biotech Inc.) at room temperature for 1 hour. The biotinylated protein bound to the tissues was visualized with a DAB solution (Sigma). Tissue sections were then counterstained with hematoxylin.

### [Chemotaxis assay]

Migration of KU-1, HTS-1, or primary trophoblast cells were assessed in a 96-well modified Boyden chamber (NeuroProbe, Cabin Johm, MD) using polyvinylpyrrolidone-free polycarbonate membrane (5 µm pore size, NeuroProbe). The polycarbonate membrane was coated with 10 µg/mL bovine plasma fibronectin (Wako Junyaku, Osaka, Japan) in advance. The assay was performed according to a method of Nagaoka, et al. (Biol. Reprod. (2003) 68: 1413-1421). Namely, DMEM-0.1% BSA (without phenol red or FCS) supplemented with indicated concentration of rcIP-10 was added to the bottom wells of the chemotaxis chamber, whereas cells (5×10⁶ cells/mL) in DMEM-0.1% BSA (without phenol red or FCS) were added to the top wells of the chamber. The chambers were incubated at 37°C and 5% CO₂ atmosphere for 2 hours, and then the membranes were removed, washed with PBS, fixed, and stained with Dif-Quick (Kokusai Shiyaku, Kobe, Japan). The number of cells that migrated to the lower surface was microscopically counted at six randomly chosen fields. For the blocking experiments, before addition to the bottom of the chamber, rcIP-10 was preincubated with anti-IP-10 antibody or control rabbit IgG (Sigma) at 37°C for 1 hour. Three independent experiments were done for each treatment.

### [Adhesion assay]

Twenty four-well plates were coated with type I collagen (Nitta gelatin) or fibronectin at a concentration of 10 µg/mL at room temperature for 2 hours, or plated with caprine epithelial cells. After washing with PBS three times, the plates were blocked with 1% BSA at room temperature for 30 min. HTS-1 or primary trophoblast cells were labeled with the intracellular fluorescent dye, 4 µM calsein-AM (Molecular Probes Inc., Eugene, OR) at 37°C for 30 min. After washing with PBS three times, the cells were incubated with the indicated rcIP-10 at 37°C for 1 hour, and were then added to each well. The plates were incubated at 37°C for 1 hour, and then washed with PBS three times to remove unbound cells. The remaining cells were treated with PBS containing 1% Triton X-100 and 10% ethanol. Fluorescence of cells was measured using fluorescence reader (excitation filter 485 nm and emission filter 535 nm) (ARVO™ SX 1420 Multilabel Counter, PerkinElmer Life Sciences Inc., Boston, MA). For the blocking experiments, rcIP-10 protein was preincubated with 30 µg/mL of anti-IP-10 antibody or control rabbit IgG (Sigma) at 37°C for 1 hour. To investigate the involvement of IP-10 on cell adhesion to fibronectin, the Gly-Arg-Gly-Asp-Ser-Pro-Lys (GRGDSPK, Sigma)synthetic peptide at a concentration of 50 mM, its control, Arg-Gly-Glu-Ser (RGES, Sigma), or 5 mM EDTA was preincubated with cells and rcIP-10 protein. Three independent experiment were performed for each substrate and treatment.

### [Statistical analysis]

Measurement of optical density (Western blot) and light intensity (RT-PCR) were subjected to least squares (LS) ANOVA, which employed the general linear models procedure of the statistical analysis system (version 6.0; SAS Institute, Cary, NY). The light intensity from G3PDH PCR products was used as covariates for RT-PCR analyses. In chemotaxis assays, the number of cells migrated or attached with a treatment was calculated as the number relative to the cells without any treatment, which was then analyzed statistically as aforementioned. The model used in the LS-ANOVA included treatment and replicate as sources of variation. The least square means (LSM) and standard error (S.E.) illustrated in Figures were derived from this analysis.

### [Preparation of rcIP-10 protein and the antibody]

Recombinant caprine IP-10 (rcIP-10) was expressed in Escherichia coli BL21-SI, which is transformed E. coli with pET-14b-cIP-10 plasmid. The rcIP-10 was purified using His tag systems, and confirmed by SDS-PAGE. Results of SDS-PAGE of cell lysates before (lane 1) and after (lane 2) purification using a nickel-chelating column are shown in Fig. 10A. Results of Western blot analysis of rcIP-10 (50 or 200 ng/lane) purified with either anti-His-tag antibody (lane 1) and anti-caprine IP-10 antibody (lane 2) are shown in Fig. 10B. Specific band at about 14kDa was detected.

To check the biological activities of rcIP-10, in vitro chemotaxis assay was carried out using bovine B-cell line, KU-1, transfected with the caprine CXCR3 cDNA. CXCR3 mRNA expression on the transfected cells was examined by Northern blot analysis. RNA was extracted from KU-1 cells that had been transformed with pcDNA3.1-caprine CXCR3 (CXCR3) or parental pcDNA3.1 (Mock) (Fig. 10C, left). Biological activity of rcIP-10 to CXCR3 transfected KU-1 cells expressing (circle) or not expressing (square) CXCR3 was tested by chemotaxis assay. Bars represent LSM ± S.E. The assay demonstrated that CXCR3 transfectants consistently responded to 1 to 20 ng/mL rcIP-10, but their responses declined at higher rcIP-10 concentrations, resulting in a characteristic bell-shaped dose response curve (Fig. 10C, right). For immunoneutralization experiments, chemotaxis activity was demonstrated (Fig. 10D) in KU-1 cells with the addition of 20 ng/mL rcIP-10, to which no further treatment was applied (-), or neutralized by the pretreatment with the anti-caprine IP-10 antibody (anti IP-10, 30 µg/mL), or with control rabbit IgG (control IgG). An asterisk indicates a significant difference (p < 0.05). The immunoneutralization experiments revealed that an anti-IP-10 antibody reduced the chemotactic activity of rcIP-10.

### [Expression of IP-10 within goat uterus during early pregnancy]

Presence of IFN-τ in the culture media (10 µg proteins/lane) derived from Days 14, 17, and 20 goat conceptuses was examined by Western blot analysis. The production of IFN-τ protein was the highest on Day 17 of pregnancy (Fig. 11A). Fig. 11B shows results of Northern blot of endometrial IP-10 mRNA. RNA (20 µ g/lane) from the conceptus (Day 17: Con) and endometrium (Day 14: D14, Day 17: D17, and Day 20: D20) of pregnant goat (left panel), and cyclic goats (Day 14, right panel) that had been stimulated with rcIFN-τ for 24 hours were electrophoresed on a 1.2% agarose gel. The Northern blot analysis revealed that IP-10 mRNA level in the caprine endometrium began to increase from day 17 of pregnancy, and that the conceptus factor IFN-τ stimulated IP-10 mRNA level by day 14 cyclic endometrium in vitro (Fig. 11B). Presence of IP-10 protein in the uterine flushing media (10 µg protein/lane) from cyclic and days 14 and 17 of pregnancy (n = 3 each) were detected using Western blot analysis Fig. 11C, left). Results of densitometric analysis of Western blots of IP-10 are shown in Fig. 11C, right. Bars represent LSM ± S.E., and an asterisk indicates a significant difference (p < 0.05) when compared with the value from Day cyclic uteri. It appeared that the uterine flushing media from Day 17 pregnant goats possessed more IP-10 than those of other days examined. Results of in situ hybridization analysis of IP-10 mRNA in the caprine uterus are shown in Fig. 11D. Panels a, b, and c represent DIG-labeled antisense caprine IP-10 cRNA on Day 14 cyclic, Day 14 pregnant, and Day 17 pregnant goats, respectively. Panel d is sense-IP-10 on Day 17 pregnant goats. Here, le; luminal epithelium, st; subepithelial stroma, tr; trophoblast, and scale bar; 50 µm. IP-10 mRNA was observed in the subepithelial stroma regions of pregnant endometrium, and the intensity was greater in Day 17 pregnant endometrium than that of in Day 14 cyclic or pregnant endometrium (Fig. 11D).

### [Expression and cellular localization of CXCR3 in the caprine conceptus]

Expression of CXCR3 mRNA in Days 14, 17, and 20 caprine endometia (D14, D17, and D20, n = 3) and Day 17 conceptus tissues (Con, n = 3) was examined using RT-PCR (Fig. 12A). Furthermore, the expression of CXCR3 mRNA in Days 17 and 20 conceptuses (2 µg poly(A)⁺ RNA/lane, n = 3 for each day) was confirmed by Northern blot analysis (Fig. 12B). Immunofluorescence analysis was performed using frozen conceptuses and anti-CXCR3 antibody. Results using anti-human CXCR3 monoclonal antibody (a), normal mouse IgG (negative control, d), and nuclei stained with propidium iodide were shown in the same field (b and e), and combined fluorescence image resulting from anti-CXCR3 and propidium iodide (c) are shown in Fig. 12C. Scale bar represents 100 µm. A patchy fluorescence associated with the trophoblast layer was found and the localization was clearly different from nuclei.

### [Binding of rcIP-10 to the caprine trophoblast cells]

Recombinant proteins, rcIP-10, GST, and caprine lymphotactin (Lymphotactin-GST) were biotinylated and the labeled proteins were detected with streptavidin-horseradish peroxidases (Fig. 13A). Expressions of IP-10 receptor, CXCR3, mRNA and lymphotactin receptor, XCR1, mRNA in the Day 17 caprine conceptus (Con), endometrium (Endo), or peripheral blood mononuclear cells (PBMCs) were confirmed by RT-PCR (Fig. 13B). Lymphotactin belongs to the C chemokine family, and its expression, similar to IP-10, was observed in the goat endometrium during early pregnancy. However, unlike CXCR3, the expression of lymphotactin receptor, XCR1, mRNA was not detected in the conceptus. For these reasons, lymphotactin/XCR was used as a negative control. Biotinylated proteins were incubated with Day 17 caprine conceptuses and visualized with steptavidin-horseradish peroxidases. The presence of CXCR3 was observed in the trophoblast cells incubated with only rcIP-10 (Fig. 13C). Scale bar represents 200 µm.

### [Stimulation of rcIP-10 on the migration of CXCR3-expressing conceptus cells]

Fig. 14A shows results of Northern blot analysis of RNAs extracted from HTS-1 cells (-), HTS-1 transfected with empty plasmid (Mock), and HTS-1 transfected with caprine CXCR3 cDNA (CXCR3). HTS-1 cells were subjected to chemotaxis assay. HTS-1 (square) transfected with the empty plasmid did not respond to rcIP-10. Contrarily, HTS-1 transfected with CXCR3-expressing plasmid consistently responded to 1 to 2o ng/mL rcIP-10. However, the responses declined as rcIP-10 concentrations increased, resulting in a characteristic bell-shaped dose response curve (Fig. 14A, right). Effect of rcIP-10 on migratory activity of HTS-1 cells that had been transfected (black bar) or not transfected (white bar) with the CXCR3-expressing plasmid was examined (Fig. 14B). These HTS-1 cells were treated with rcIP-10 (20 ng/mL), which had been pretreated with 30 µg/mL of the anti-cIP-10 antibody (Abs: IP-10) or 30 µg/mL of normal rabbit IgG (Abs: IgG). Ratio of migrated cells was calculated as the number of migrated cells treated with rcIP-10 divided by the number of migrated cells without the rcIP-10 treatment. An asterisk indicates a significant difference (p < 0.05). An anti-rcIP-10 antibody reduced the chemotactic activity of rcIP-10. Next, primary trophoblast cells expressing CXCR3 on Day 17 of pregnancy were subjected to the chemotaxis assay. The migration of trophoblast cells was stimulated by rcIP-10 protein, and the chemotactic activity was neutralized with the use of an anti-IP-10 antibody (Fig. 14C).

### [Effect of rcIP-10 on the adhesion of trophoblast cells to fibronectin and endometrial epithelial cells]

Results of the adhesion assay of caprine trophoblast cells (Day 17 of pregnancy) stimulated (+) or not stimulated (-) with rcIP-10 (20 ng/mL) to the plate coated with collage I, fibronectin, or non-substrate showed that the binding rate of rcIP-10-stimulated cells to the fibronectin-coated plates was considerably higher than that of others (Fig. 15A). Blocking experiment was performed by adhesion assay of trophoblast cells to fibronectin using the pretreatment of rcIP-10 with anti-cIP-10 antibody (IP-10) or normal rabbit IgG (IgG) (Fig. 15B). Fifty mM of Arg-Gly-Asp (RGD) peptide (which inhibits cell adhesion occurring through RGD sites of fibronectin), 5 mM of Arg-Gly-Glu (RGE) peptide (inactive control), or 5 mM of EDTA (inhibitor of integrin signaling), were applied to the trophoblast cells, which were then subjected to chemotaxis assay (Fig. 15B). The RGD peptide decreased the adhesion of rcIP-10-stimulated cells to fibronectin, but did not the RGE peptide. EDTA also decreased the adhesion of trophoblast cells to fibronectin.

In HTS-1 cells (black bar) transfected with CXCR3 cDNA, the adhesion of cells of fibronectin was stimulated by rcIP-10 protein and inhibited by anti-IP-10 antibody (Fig. 15C). The addition of RGD peptide or EDTA inhibited the adhesion of cells transfected with CXCR3 to fibronectin (Fig. 15C). The RGD peptide and EDTA treatments inhibited migration activities of cells (white bar) transfected with empty plasmids.

HTS-1 cells (black bar) transfected with CXCR3 plasmid or HTS-1 cells (white bar) transfected with empty plasmid were subjected to the adhesion assay with endometrial epithelial cells (Fig. 15D). The adhesion of CXCR3-expressing HTS-1 to the endometrial epithelial cells was increased by rcIP-10 protein and inhibited by the use of antibody to the IP-10 in the neutralizing experiment. EDTA-treated trophoblast cells lost the adhesion activity to epithelial cells. RGD peptide inhibited the adhesion activity. Bars represent LSM ± S.E., and an asterisk indicates a significant difference (p < 0.05).

### [Expression of integrin subunits in the caprine trophoblast cells stimulated by rcIP-10]

The expression of integrin subunits was examined in HTS-1 cells stimulated by rcIP-10. Transcripts for integrin subunits, α5, αV, β1, β3, and β5 were detected by using RT-PCR. The expression of integrin α5, αV, and β3 subunits mRNA in trophoblast cells were stimulated by rcIP-10, and the stimulation was diminished by the neutralization with the anti-IP-10 antibody (Fig. 16). The expression of integrin β1 and β5 subunits mRNA was not influenced by rcIP-10. Bars represent LSM ± S.E.

The results above show that expressions of IP-10 and IFN-γ mRNA time-varyingly and cell-specifically occur during early pregnancy in the caprine uterus as well as in the ovine uterus, and conceptus IFN-τ has an ability to regulate endometrial IP-10 expression. These findings suggest that IP-10 regulated by IFN-τ plays an important role in the recruitment and/or redistribution of immune cells during early pregnancy.

The present invention provides a way for investigating relationship between IP-10 and IFN-τ and IP-10 functions in uterus.

### INDUSTRIAL APPLICABILITY

The present invention provides a technology for utilizing IP-10, which controls implantation of conceptus or its process, and the present invention provides pharmaceutical drugs, veterinary drugs, therapeutic methods, activity-measuring methods, assay methods, and reagents used therefore, which control or utilize the maternal system in the pregnant recognition process. According to the present invention, pregnancy can be securely promoted or prevented in animals including human and domestic animals, and useful materials or medicine for such purposes can be readily developed.

While specific details of the present invention have been described in terms of preferred embodiments and examples, it will be apparent to those of skill in the field of the art that variations may be applied to those disclosed in the foregoing. In light of the disclosure, various modifications and rearrangements which can be made to those set forth herein are deemed to be within the spirit and scope of the appended claims.

### <Free text sequence>

SEQ ID NO: 3, Description of Artificial Sequence:
Oligonucleotide to act as a primer for PCR

SEQ ID NO: 4, Description of Artificial Sequence:
Oligonucleotide to act as a primer for PCR

SEQ ID NO: 5, Description of Artificial Sequence:
Oligonucleotide to act as a primer for PCR

SEQ ID NO: 6, Description of Artificial Sequence:
Oligonucleotide to act as a primer for PCR

SEQ ID NO: 7, Description of Artificial Sequence:
Oligonucleotide to act as a primer for PCR

SEQ ID NO: 8, Description of Artificial Sequence:
Oligonucleotide to act as a primer for PCR

SEQ ID NO: 9, Description of Artificial Sequence:
Oligonucleotide to act as a primer for PCR

SEQ ID NO: 10, Description of Artificial Sequence:
Oligonucleotide to act as a primer for PCR

SEQ ID NO: 11, Description of Artificial Sequence:
Oligonucleotide to act as a primer for PCR

SEQ ID NO: 12, Description of Artificial Sequence:
Oligonucleotide to act as a primer for PCR

SEQ ID NO: 13, Description of Artificial Sequence:
Oligonucleotide to act as a primer for PCR

SEQ ID NO: 14, Description of Artificial Sequence:
Oligonucleotide to act as a primer for PCR

SEQ ID NO: 15, Description of Artificial Sequence:
Oligonucleotide to act as a primer for PCR

SEQ ID NO: 16, Description of Artificial Sequence:
Oligonucleotide to act as a primer for PCR

SEQ ID NO: 17, Description of Artificial Sequence:
Oligonucleotide to act as a primer for PCR

SEQ ID NO: 18, Description of Artificial Sequence:
Oligonucleotide to act as a primer for PCR

SEQ ID NO: 19, Description of Artificial Sequence:
Oligonucleotide to act as a primer for PCR

SEQ ID NO: 20, Description of Artificial Sequence:
Oligonucleotide to act as a primer for PCR

SEQ ID NO: 21, Description of Artificial Sequence:
Oligonucleotide to act as a primer for PCR

SEQ ID NO: 22, Description of Artificial Sequence:
Oligonucleotide to act as a primer for PCR

SEQ ID NO: 23, Description of Artificial Sequence:
Oligonucleotide to act as a primer for PCR

SEQ ID NO: 24, Description of Artificial Sequence:
Oligonucleotide to act as a primer for PCR

SEQ ID NO: 25, Description of Artificial Sequence:
Oligonucleotide to act as a primer for PCR

SEQ ID NO: 26, Description of Artificial Sequence:
Oligonucleotide to act as a primer for PCR

## Claims

1. A pharmaceutical drug and/or veterinary drug comprising an effective amount of one or more members selected from the group consisting of an IP-10 protein, and IP-10 analogues and IP-10 derivatives having at least a deletion, addition, and/or substitution of one or more amino acid residues in IP-10 and having a biological activity essentially equal or equivalent to that of intact IP-10,
the pharmaceutical drug and/or veterinary drug being an agent selected from the group consisting of (a) an agent for activating conceptus migration, (b) an agent for promoting conceptus implantation on the uterine wall, (c) an agent for treating sterility, (d) an agent for promoting pregnancy, (e) an agent for controlling interaction between conceptus and maternal system, (f) an agent for activating immunocyte migration, and (g) an agent for controlling immune function in the uterus.

2. The pharmaceutical drug and/or veterinary drug according to claim 1, wherein the IP-10 is derived from mammal including human, bovine, buffalo, equine, donkey, ovine, goat, camel, swine, deer, reindeer, yak, canine, cat, and ape.

3. A method for obtaining a biological activity selected from the group consisting of (a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
which comprises treating a sample with a material selected from the group consisting of IP-10, and IP-10 analogues and IP-10 derivatives having at least a deletion, addition, and/or substitution of one or more amino acid residues in IP-10 and having a biological activity essentially equal or equivalent to that of intact IP-10.

4. A reagent comprising a material selected from the group consisting of IP-10, and IP-10 analogues and IP-10 derivatives having at least a deletion, addition, and/or substitution of one or more amino acid residues in IP-10 and having a biological activity essentially equal or equivalent to that of intact IP-10 and being useful for the method according to claim 3.

5. An assay for measuring an IP-10 activity to determine a biological activity selected from the group consisting of (a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

6. A reagent that is useful for the assay according to claim 5.

7. A pharmaceutical drug and/or veterinary drug comprising a nucleic acid having a nucleotide sequence encoding a member selected from the group consisting of IP-10, and IP-10 analogues and IP-10 derivatives having at least a deletion, addition, and/or substitution of one or more amino acid residues in IP-10 and having a biological activity essentially equal or equivalent to that of intact IP-10,
the pharmaceutical drug and/or veterinary drug being an agent selected from the group consisting of (a) an agent for activating conceptus migration, (b) an agent for promoting conceptus implantation on the uterine wall, (c) an agent for treating sterility, (d) an agent for promoting pregnancy, (e) an agent for controlling interaction between conceptus and maternal system, (f) an agent for activating immunocyte migration, and (g) an agent for controlling immune function in the uterus.

8. A pharmaceutical drug and/or veterinary drug comprising a nucleic acid selected from the group consisting of
(i) a nucleotide sequence comprising at least one open reading frame portion present in SEQ ID NO: 1 and,
(ii) a nucleotide sequence capable of hybridizing with at least one sequence described in the above (i) under a stringent condition, and
(iii) a nucleotide sequence encoding a peptide containing an amino acid sequence at least 80% homologous to a polypeptide shown in FIG. 2 or shown by SEQ ID NO: 2, wherein said peptide has a biological activity substantially equal to that of IP-10 (for example, ovine IP-10), including a biological activity, or an antigenic equivalent thereof, selected from the group consisting of (a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the pharmaceutical drug and/or veterinary drug being an agent selected from the group consisting of (a) an agent for activating conceptus migration, (b) an agent for promoting conceptus implantation on the uterine wall, (c) an agent for treating sterility, (d) an agent for promoting pregnancy, (e) an agent for controlling interaction between conceptus and maternal system, (f) an agent for activating immunocyte migration, and (g) an agent for controlling immune function in the uterus.

9. A pharmaceutical drug comprising a compound, or a salt thereof, for promoting or inhibiting a biological activity selected from the group consisting of (a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
said biological activity being owned by
(A) a material, or its salt, selected from the group consisting of IP-10 and IP-10 analogues or IP-10 derivatives having at least a deletion, addition, and/or substitution of one or more amino acid residues in IP-10 and having biological activities essentially equal or equivalent to those of intact IP-10, or
(B) a nucleic acid selected from the group consisting of
(i) a nucleotide sequence comprising at least an open reading frame portion present in SEQ ID NO: 1 and,
(ii) a nucleotide sequence capable of hybridizing with at least one sequence described in the above (i) under a stringent condition, and
(iii) a nucleotide sequence encoding a peptide containing an amino acid sequence at least 80% homologous to a polypeptide in Fig. 2 or shown by SEQ ID NO: 2, wherein said peptide has a biological activity substantially equal to that of IP-10 (for example, ovine IP-10), including a biological activity, or an antigenic equivalent thereof, selected from (a) activating conceptus migration, (b) promoting conceptus implantation on the uterine wall, (c) treating sterility, (d) promoting pregnancy, (e) controlling interaction between conceptus and maternal system, (f) activating immunocyte migration, and (g) controlling immune function in the uterus.

10. A method or kit for screening a compound promoting or inhibiting a biological activity owned by a member selected from the group consisting of IP-10, IP-10 analogues and IP-10 derivatives having at least a deletion, addition, and/or substitution of one or more amino acid residues in IP-10 and having a biological activity essentially equal or equivalent to that of intact IP-10, or salts thereof, and IP-10 nucleic acids encoding the same, wherein the biological activity is selected from the group consisting of (a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the method or the kit using or comprising (A) a compound, or its salt, selected from the group consisting of IP-10, the IP-10 analogues and the IP-10 derivatives, or (B) a material selected from the group consisting of nucleic acids encoding the compound in (A), vectors containing the nucleic acid, and host cells transformed with the nucleic acid or the vector.

11. The method or kit according to claim 10, wherein the method or kit is useful for screening a compound promoting the production of IP-10 and preventing development and/or progress of sterility.

12. A compound for controlling the production of IP-10, which is obtained or identified by screening with the method or kit according to claim 10 or 11.

13. A reagent for detecting the presence of a mutated portion capable of altering the activity or expression of IP-10 wherein said mutated portion is present in IP-10, a gene encoding IP-10, or the corresponding RNA, and genetically diagnosing a disease associated with IP-10.

14. The diagnostic reagent according to claim 13, which comprises at least a material selected from the group consisting of a restriction enzyme capable of specifically recognizing a mutation in IP-10 gene, mRNA, or hnRNA, and an isoschizomer thereof; and an oligonucleotide primer useful in amplification of a gene including a mutation in IP-10 gene, mRNA, or hnRNA.

15. A method for genetic diagnosis of a disease associated with an IP-10 gene which comprises the steps of;
(a) preparing a nucleic acid sample,
(b) subjecting the nucleic acid sample in the step (a) to gene amplification to give amplified nucleic acid fragments containing one or more mutations in the IP-10 gene, and
(c) determining the presence of mutation in the nucleic acid fragment prepared in the step (c).

16. A method for determining or diagnosing a biological activity in a specimen which comprises quantitating an IP-10 polynucleotide present in the specimen and using as an indicator the IP-10 polynucleotide amount to determine or diagnose a biological activity level in the specimen
wherein said biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

17. A method for determining or diagnosing a biological activity in a specimen which comprises quantitating an IP-10 protein present in the specimen and using as an indicator the IP-10 protein amount to determine or diagnose a biological activity level in the specimen
wherein said biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

18. A composition for determining or diagnosing a biological activity in a specimen which comprises at least a member selected from an oligonucleotide or polynucleotide which hybridizes with IP-10 polynucleotide under a stringent condition,
wherein the biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

19. A nucleic acid array for determining or diagnosing a biological activity level in a specimen which comprises (i) an oligonucleotide or polynucleotide which hybridizes with IP-10 polynucleotide under a stringent condition or (ii) IP-10 polynucleotide,
wherein the biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

20. A primer set used for PCR-amplification of IP-10 polynucleotide in a specimen and determining or diagnosing the degree of a biological activity of a specimen,
wherein the biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

21. A diagnosis kit for determining or diagnosing the degree of a biological activity of a specimen comprising at least one antibody capable of recognizing IP-10,
wherein the biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

22. A diagnosis kit for determining or diagnosing the degree of a biological activity of a specimen comprising at least elements consisting of
(i) an immobilized antibody capable of recognizing IP-10, and
(ii) an antibody capable of recognizing an IP-10 epitope other than that being recognized by the antibody (i),
wherein the biological activity is selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus.

23. A method for measuring the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the method comprising at least the steps of:
(i) contacting a biological specimen with a support immobilized with an antibody capable of recognizing IP-10;
(ii) washing the support contacted with the biological specimen;
(iii) contacting the support contacted with the biological specimen with a labeled antibody, wherein the labeled antibody is capable of recognizing an epitope on IP-10 other than that being recognized by the immobilized antibody;
(iv) measuring a label on the support or a free label;
(v) using the amount of the label measured in the step (iv) as an indicator of the amount of IP-10 and comparing it with a result of a normal biological specimen; and
(vi) using the amount of IP-10 being significantly different from the result of the normal biological specimen as an indicator of the degree of abnormality or its risk associated with the biological activity.

24. A method for measuring the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the method comprising at least the steps of:
(i) preparing RNA from a biological specimen;
(ii) separating the RNA prepared in the step (i) by electrophoresis;
(iii) hybridizing the RNA separated in the step (ii) with a labeled nucleotide probe which hybridizes IP-10 polynucleotide under a stringent condition;
(iv) using the amount of the label hybridized in the step (iii) as an indicator of expression of IP-10 polynucleotide and comparing it with a result of a normal biological specimen; and
(v) using the amount of expression of IP-10 polynucleotide being significantly different from the result of the normal biological specimen as an indicator of the degree of abnormality or its risk associated with the biological activity.

25. A method for measuring the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the method comprising at least the steps of:
(i) preparing RNA from a biological specimen;
(ii) generating a first cDNA strand by using the RNA prepared in the step (i) as a template with a dT primer;
(iii) amplifying IP-10 polynucleotide by PCR using the cDNA generated in the step (ii) as a template with a primer set for amplifying the IP-10 polynucleotide;
(iv) separating the PCR product in the step (iii) by electrophoresis;
(v) hybridizing the PCR product separated in the step (iv) with a labeled nucleotide probe which hybridizes IP-10 polynucleotide under a stringent condition;
(vi) using the amount of the label hybridized in the step (v) as an indicator of expression of IP-10 polynucleotide and comparing it with a result of a normal biological specimen; and
(vii) using the amount of expression of IP-10 polynucleotide being significantly different from the result of the normal biological specimen as an indicator of the degree of abnormality or its risk associated with the biological activity.

26. A method for measuring the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the method comprising at least the steps of:
(i) treating a biological specimen for tissue fixation;
(ii) sectioning the fixed tissue prepared in the step (i);
(iii) staining the sectioned tissue immunohistologically with an antibody capable of recognizing IP-10;
(iv) comparing the degree of the immunohistological stained biological specimen with that of a normal biological specimen; and
(v) using the amount of IP-10 protein being significantly different from the result of the normal biological specimen as an indicator of the degree of abnormality or its risk associated with the biological activity.

27. A method for measuring the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the method comprising at least one step selected from the group consisting of:
(i) amplifying IP-10 polynucleotide in the biological specimen by PCR using a primer set for amplifying the IP-10 polynucleotide;
(ii) analyzing a nucleic acid fraction separated from the biological specimen by using (I) an oligonucleotide or polynucleotide which hybridizes with IP-10 polynucleotide under a stringent condition or (II) a nucleic acid array having IP-10 polynucleotide; and
(iii) hybridizing a nucleic acid fraction separated from the biological specimen with an oligonucleotide or polynucleotide which hybridizes with IP-10 polynucleotide under a stringent condition; and
the step of measuring the amount of IP-10 polynucleotide in the biological specimen and then comparing the amount of the IP-10 polynucleotide in the specimen with that in a normal biological specimen.

28. The method according to any one of claims 23 to 27 for measuring abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
by quantitatively measuring IP-10 protein or expression of IP-10 polynucleotide.

29. A reagent used in the method according to any one of claims 23 to 27 for measuring abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
wherein the reagent contains an antibody selected from the group consisting of (i) an antibody capable of recognizing IP-10, (ii) an antibody capable of binding an epitope on IP-10 which is different from that recognized by the antibody (i), (iii) an immobilized antibody (i) or antibody (ii), and (iv) a labeled antibody (i) or antibody (ii).

30. A method for measuring or diagnosing the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
by measuring an amount of IP-10 protein or expression of IP-10 polynucleotide in a sample by using an antibody capable of recognizing IP-10.

31. A reagent for measuring or diagnosing the degree of abnormality or its risk associated with a biological activity selected from the group consisting of:
(a) activation of conceptus migration, (b) promotion of conceptus implantation on the uterine wall, (c) treatment of sterility, (d) promotion of pregnancy, (e) control of interaction between conceptus and maternal system, (f) activation of immunocyte migration, and (g) control of immune function in the uterus,
the reagent containing an antibody capable of recognizing IP-10 for measuring an amount of IP-10 protein or expression of IP-10 polynucleotide in a sample.
